# FASCICULE DE BREVET EUROPEEN

(11) **EP 0 498 721 B1**
(45) Date de publication et mention de la délivrance du brevet: **22.12.1999**
(21) Numéro de dépôt: 92400294.2
(22) Date de dépôt: 05.02.1992
(51) Int. Cl.: C07D 215/233, A61K 31/47, C07D 215/36, C07D 401/06, C07D 237/28, C07D 471/04, C07D 401/10

(54) **Dérivés N-substitués de la quinoléine, leur procédé de préparation, leur application à titre de médicaments et les compositions pharmaceutiques les renfermant**
N-substituierte Chinolinderivate, Verfahren zur Herstellung, ihre Verwendung als Arzneimittel und diese enthaltende pharmazeutische Zusammensetzungen
N-substituted quinoline derivatives, process for their preparation, their use as pharmaceuticals and pharmaceutical compositions containing them

(30) Priorité: 07.02.1991 FR 9101372; 20.08.1991 FR 9110433; 20.11.1991 FR 9114282
(43) Date de publication de la demande: 12.08.1992
(73) Titulaire: HOECHST MARION ROUSSEL, 92800 Puteaux (FR)
(72) Inventeur: Clemence, François, F-75009 Paris (FR); Fortin, Michel, F-75018 Paris (FR); Haesslein, Jean-Luc, F-77181 Courtry (FR)
(74) Mandataire: Vieillefosse, Jean-Claude

(56) Documents cités:
- EP-A- 0 104 959
- EP-A- 0 343 574
- FR-A- 2 242 983
- FR-A- 2 377 400
- FR-A- 2 452 484
- GB-A- 1 419 788
- GB-A- 1 472 767
- CHEMICAL ABSTRACTS, vol. 111, 1989, page 758, abrégé no. 194610c, Columbus, Ohio, US; & JP-A-01 61 461 (OTSUKA PHARMACEUTICAL CO., LTD) 08-03-1989
- JOURNAL OF MEDICINAL CHEMISTRY, vol. 15, no. 3, mars 1972, pages 233-235; B.R. BAKER et al.: "Irreversible enzyme inhibitors. 190. Inhibition of some dehydrogenases by 1-substituted-1,4-dihydro-4-quinolone-3-carboxylic acids"
- JOURNAL OF MEDICINAL CHEMISTRY, vol. 15, no. 3, mars 1972, pages 230-233; B.R. BAKER et al.: "Irreversible enzyme inhibitors. 189. Inhibition of some dehydrogenases by derivatives of 4-hydroxyquinoline-2 and -3-carboxylic acids"
- BULLETIN OF THE CHEMICAL SOCIETY OF JAPAN, vol. 46, no. 2, février 1973, pages 530-534, The Chemical Society of Japan, Tokyo, JP; Y. YOKOYAMA et al.: "The reaction of beta-amino alpha,beta-unsaturated esters with amines"
- CHEMICAL ABSTRACTS, vol. 85, 1976, page 466, abrégé no. 45850y, Columbus, Ohio, US; S.I. YAKIMOVICH et al.: "Tautomerism in a series of nitrogen derivatives of ethyl alpha-ethoxalylcarboxylates", & ZH. ORG. KHIM. 1976, 12(5), 949-56

## Description

La présente invention concerne de nouveaux dérivés N-substitués de la quinoléine, leur procédé de préparation, les nouveaux intermédiaires obtenus, leur application à titre de médicaments et les compositions pharmaceutiques les renfermant.
EP-A-0343574 décrit des quinolones pour le traitement de congestion cardiaque, d'angine de poitrine, on d'oedèmes.
J. Med. Chem 1972, vol 15, No 3 pp 230-235 décrit des composés inhibiteurs d'enzymes (déshydrogénases).
Chem. Abstracts, vol 111, 1989, No 194610 c décrit la préparation de 4-oxoquinolines comme vasodilatateur.
FR-A-2377400 décrit des dérivés d'acide 3-quinoléine carboxylique comme analgésiques
GB-A-1419788 décrit des 2-trifluorométhyl-4-quinolinols comme herbicides.
GB-A-1472767 décrit un procédé de préparation de dérivés de cinnoline.
EP-A-0412848 décrit des dérivés de quinoline comme antihypertenseurs.
EP-A-0456442 décrit des dérivés de quinoline comme antagonistes de l'angiotensine.
EP-A-0453210 décrit des dérivés de pyridine comme antihypertenseurs.
EP-A-0407192 décrit des dérivés de 4-acyloxyquinoline comme insecticides et acaricides.
EP-A-0326328 décrit des dérivés de quinoline, quinazoline et cinnoline comme fongicides.
Synthesis, vol. 2, 1984 pp 150-152 décrit un procédé d'obtention de 4-oxo-1,4-dihydroquinoline-2-carboxylates d'alkyle.
J.Chem. Soc. , Vol 18, 1971, pp 3088-3097 décrit la méthylation de methoxy-et alkyl-cinnolines et 4(1H)-cinnolones.
Chemishe Berichte, vol 104(8), pp 2483-2486, 1971 décrit un procédé de préparation de quinolone.

La présente invention a pour objet les produits de formule (I) : 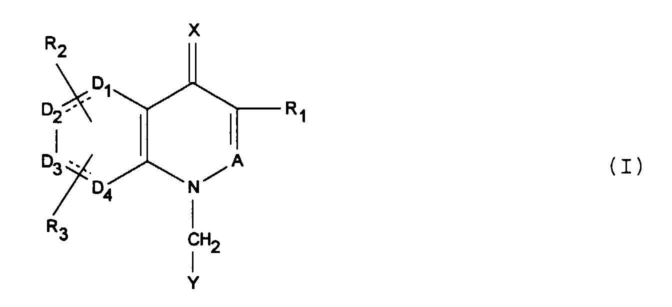 dans laquelle : 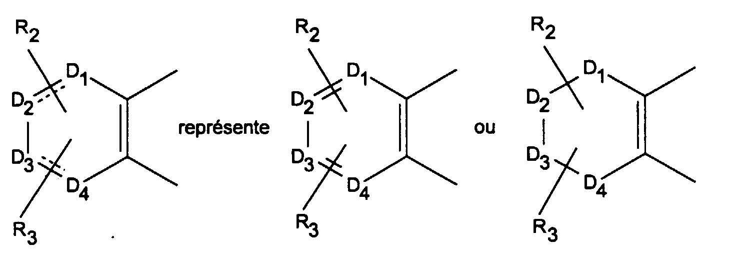
avec D₁, D₂ représentant tous deux un radical méthine ou un radical méthylène, éventuellement substitués par un radical choisi parmi R₂ et R₃,
D₃ et D₄, identiques ou différents, représentant un atome d'azote, un radical méthine ou un radical méthylène, éventuellement substitués par un radical choisi parmi R₂ et R₃,
l'un au moins de D₃ et D₄ représentent un radical méthine ou méthylène, éventuellement substitué par un radical choisi parmi R₂ et R₃,
R₁ représente un radical méthyle, éthyle, n-propyle, n-propényle, n-butyle,
R₂ et R₃, identiques ou différents, sont choisis dans le groupe formé par :
   - l'atome d'hydrogène,
   - les radicaux mercapto et alkylthio,
   - les radicaux alkyle linéaires ou ramifiés renfermant au plus 6 atomes de carbone et éventuellement substitués par un ou plusieurs substituants identiques ou différents choisis parmi les atomes d'halogène, le radical hydroxyle, le radical amino, mono et dialkylamino, pyrrolidinyle, morpholinyle, pipéridinyle,
   - le radical carboxy libre, salifié ou estérifié par un radical alkyle linéaire ou ramifié renfermant au plus 4 atomes de carbone,
   - le radical pyrrolidinyl-carbonyle, morpholinyl-carbonyle, carbamoyle, dialkylcarbamoyle, pipéridynyl-carbonyle,
      A représente un atome d'azote ou un radical CH,
      X représente un atome d'oxygène ou un atome de soufre,
      Y représente un radical phényle ou biphényle éventuellement substitué par un ou plusieurs radicaux choisis parmi les radicaux cyano, carboxy libre, salifié et estérifié, tétrazole et isoxazole,
à l'exclusion des produits de formule (I) dans laquelle 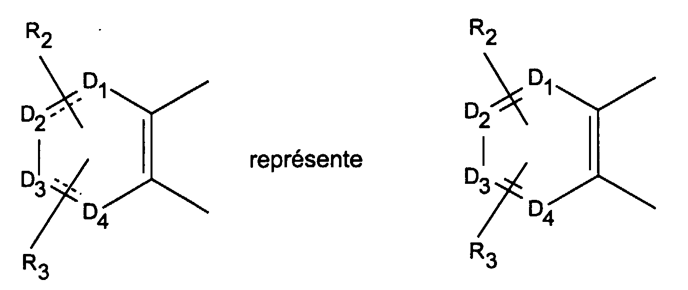
avec D₁, D₂, D₃ et D₄ représentant chacun un radical méthine X représente un atome d'oxygène et A représente un radical CH, R₁, R₂ et R₃, identiques représentent tous trois un radical méthyle, et -Y représente un radical phényle,
lesdits produits de formule (I) étant sous toutes les formes isomères possibles racémiques, énantiomères et diastéréoisomères, ainsi que les sels d'addition avec les acides minéraux et organiques ou avec les bases minérales et organiques, desdits produits de formule (I).
La présente invention a également pour objet les produits suivants :
. Acide 4'-((3-butyl-1,4-dihydro-7-formyl-4-oxo-1-quinoléinyl)méthyl)-(1,1'-biphényl)-2-carboxylique.
. Acide 3-butyl-1-(2'-carboxy-(1,1'-biphényl)-4-yl)méthyl)-1,4-dihydro-4-oxo-7-quinoléinepropanoique.
. Acide 4'-((3-butyl-6-(2-carboxyéthenyl)-1,4-dihydro-4-oxo-1-quinoléinyl) méthyl)-(1,1'-biphényl)-2-carboxylique.
. Acide 4'-((3-butyl-1,4-dihydro-6-formyl-4-oxo-1-quinoléinyl)méthyl)-(1,1'-biphényl)-2-carboxylique.
. Acide 4'-(3-butyl-1,4-dihydro-7-(3-hydroxy-3-oxo-1-propényl)-4-oxo-1-quinoléinyl)méthyl)-(1,1'-biphényl)-2-carboxylique.
. Acide 3-butyl-1-((2'-carboxy-(1,1'-biphényl)-4-yl)méthyl)-1,4-dihydro-4-oxo-7-quinoléineacétique.
. Acide 4'-((3-butyl-1,4-dihydro-4-oxo-7-(2-(1-pyrrolidinyl)-2-oxoéthyl)-1-quinoléinyl)méthyl)-(1,1'-biphényl)-2-carboxylique.
. Acide 1,4-dihydro-4-oxo-1((2'-(1H-tétrazol-5-yl)(1,1'-biphényl)-4-yl)méthyl-quinoléine-3,8-dicarboxylique.
. 3-butyl-1-((2'-(1H-tétrazol-5-yl)-1,1'-biphényl)-4-yl)méthyl)-6,7,8-trifluoro-4(1H)-quinoléinone.
. 3-butyl-6,8-difluoro-7-(1-pyrrolidinyl)-1-((2'-(1H-tétrazol-5-yl)-(1,1'-biphényl)-4-yl)méthyl)-4-(1H)-quinoléinone
ces produits étant sous toutes les formes isomères possibles racémiques, énantiomères et diastéréoisomères, ainsi que les sels d'addition avec les acides minéraux et organiques ou avec les bases minérales et organiques de ces produits.

Dans les produits de la présente invention notamment de formule (I), et dans ce qui suit :
- le terme radical alkyle linéaire ou ramifié désigne de préférence les radicaux méthyle, éthyle, n-propyle, isopropyle, n-butyle, isobutyle, sec-butyle et tert-butyle mais peut également représenter un radical pentyle ou hexyle et particulièrement isopentyle et isohexyle,
- le terme atome d'halogène désigne de préférence l'atome de chlore, mais peut aussi représenter un atome de fluor, de brome ou d'iode ;
- le terme radical alkylthio linéaire ou ramifié désigne les radicaux dans lesquels le radical alkyle peut représenter, par exemple, les valeurs indiquées ci-dessus pour le radical alkyle ; le radical alkylthio représente de préférence les radicaux méthylthio ou éthylthio, mais peut aussi représenter un radical propylthio, isopropylthio, n-butylthio, sec-butylthio, tert-butylthio, isopentylthio ou isohexylthio.

Les radicaux amino, que peuvent représenter l'un ou plusieurs des éventuels substituants des radicaux définis dans les produits de formule (I) et dans ce qui suit, désignent des radicaux dans lesquels à l'atome d'azote sont liés deux radicaux, identiques ou différents, choisis parmi l'atome d'hydrogène ; les radicaux alkyle tels que définis ci-dessus pour donner de préférence les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, pyrrolidinyle, pipéridino, morpholino.
Le ou les radicaux carboxy des produits de formule (I) peuvent être salifiés ou estérifiés par les groupements divers connus de l'homme de métier, parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle,

Les sels d'addition avec les acides minéraux ou organiques des produits de la présente invention et notamment de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Le ou les radicaux carboxy des produits de la présente invention et notamment de formule (I), peuvent être salifiés par des bases minérales telles que, par exemple, un équivalent de sodium, de potassium, de lithium, le calcium, le magnésium ou l'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Parmi les produits, objet de l'invention, peuvent être cités tout particulièrement, les produits répondant aux définitions suivantes :
. l'acide 4'-[(3-butyl 1,4-dihydro-4-oxo-1-quinoléinyl) méthyl] -(1,1'-biphényl)-2-carboxylique,
. l'acide 4'-[(3-butyl 1,4-dihydro-4-thioxo-1-quinoléinyl) méthyl] (1,1'-biphényl) 2-carboxylique,
. l'acide 3-butyl 1-[(2'-carboxy (1,1'-biphényl) 4-yl) méthyl] 1,4-dihydro-4-oxo-7-quinoléinecarboxylique,
. l'acide 4'-[(3-butyl 1,4-dihydro 8-bromo 4-oxo 1-quinoléinyl) méthyl] (1,1'-biphényl) 2-carboxylique,
. l'acide 4'-[(3-butyl 1,4-dihydro 7-(hydroxyméthyl) 4-oxo 1-quinoléinyl) méthyl] (1,1'-biphényl) 2-carboxylique,
. l'acide 4'-[(3-butyl 1,4-dihydro 4-oxo 7-[(1-pyrrolidinyl) carbonyl] 1-quinoléinyl) méthyl] (1,1'-biphényl) 2-carboxylique,
. l'acide 4'-[(3-butyl 1,4,5,6,7,8-hexahydro 4-oxo 1-[(1-quinoléinyl) méthyl] (1,1'-biphényl) 2-carboxylique,
. l'acide 4'-[(3-butyl 1,4,5,6,7,8-hexahydro 6-méthyl 4-oxo 1-quinoléinyl) méthyl] (1,1'-biphényl) 2-carboxylique,
. l'acide 3-butyl 1,4-dihydro 1-[[2'-(1H-tétrazol-5-yl) (1,1'-biphényl) 4-yl] méthyl] 8-quinoléinecarboxylique,
. l'acide 1,4-dihydro 3-méthyl 4-oxo 1-[[2'-(1H-tétrazol-5-yl) (1,1'-biphényl) 4-yl] méthyl] 8-quinoléinecarboxylique,
. l'acide 1,4-dihydro 3-éthyl 4-oxo 1-[[2'-(1H-tétrazol-5-yl) (1,1'-biphényl) 4-yl] méthyl] 8-quinoléinecarboxylique.

L'invention a également pour objet un procédé de préparation des produits de la présente invention et notamment des produits de formule (I) telle que définie ci-dessus, caractérisé en ce que :
**soit** l'on fait réagir un produit de formule (II) : 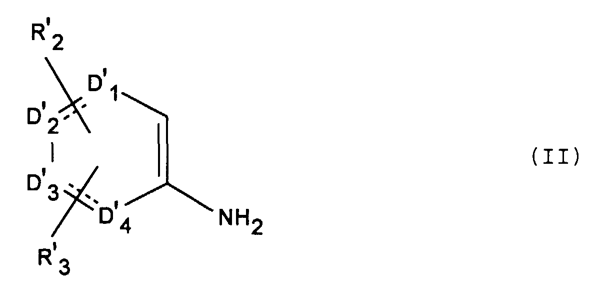 dans laquelle D'₁, D'₂, D'₃, D'₄, R'₂ et R'₃ ont les significations indiquées ci-dessus, respectivement pour D₁, D₂, D₃, D₄, R₂ et R₃ dans lesquelles les éventuelles fonctions réactives sont éventuellement protégées par des groupements protecteurs, avec un produit de formule (III) : 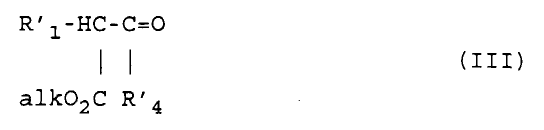 dans laquelle R'₁ et R'₄ ont les significations indiquées ci-dessus, respectivement pour R₁ et R₄ dans lesquelles les éventuelles fonctions réactives sont éventuellement protégées par des groupements protecteurs et alk représente un radical alkyle renfermant au plus 6 atomes de carbone, pour obtenir des produits de formule (IV) : 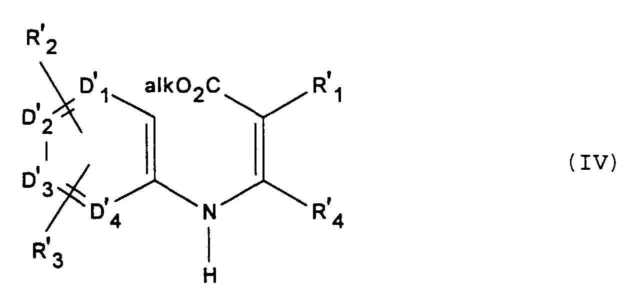 dans laquelle D'₁, D'₂, D'₃, D'₄, R'₂, R'₃, R'₄ et alk ont les significations indiquées ci-dessus, que l'on cyclise pour obtenir des produits de formule (V) : 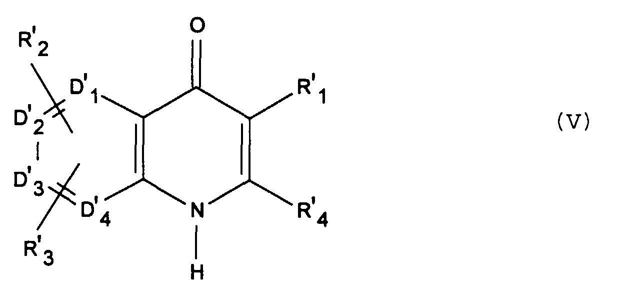 dans laquelle D'₁, D'₂, D'₃, D'₄, R'₁, R'₂, R'₃ et R'₄ ont les significations indiquées ci-dessus,
**soit** l'on fait réagir un produit de formule (VI) : 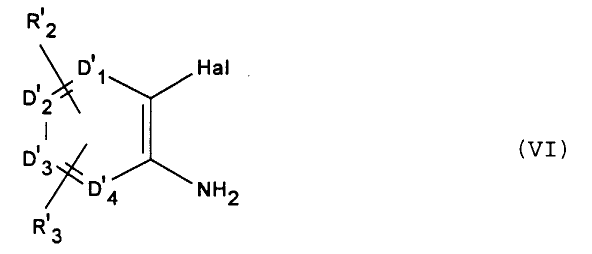 dans laquelle D'₁, D'₂, D'₃, D'₄, R'₂ et R'₃ ont les significations indiquées ci-dessus et Hal représente un atome d'halogène, avec un produit de formule (VII) :

   R'₁-C≡-CH (VII)

   dans laquelle R'₁ a la signification indiquée ci-dessus, pour obtenir des produits de formule (VIII) : 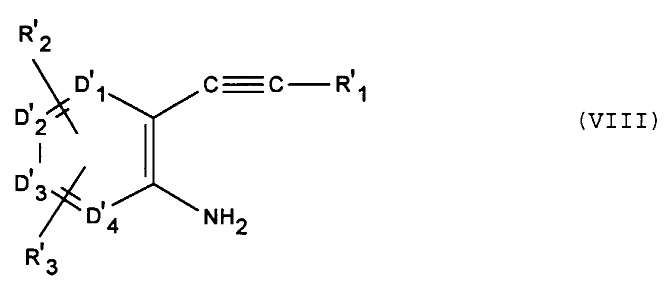 dans laquelle D'₁, D'₂, D'₃, D'₄, R'₁, R'₂ et R'₃ ont les significations indiquées ci-dessus, que l'on soumet à une réaction de cyclisation pour obtenir un produit de formule (IX) : 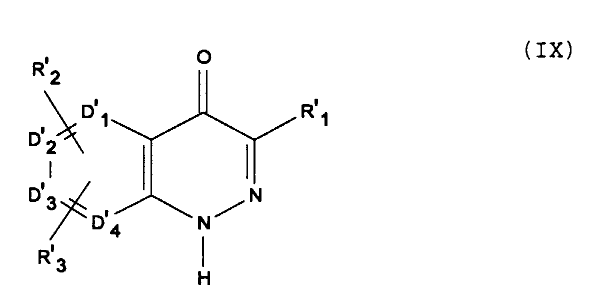 dans laquelle D'₁, D'₂, D'₃, D'₄, R'₁, R'₂ et R'₃ ont les significations indiquées ci-dessus, produits de formules (V) et (IX) que, si désiré, l'on soumet à une réaction d'hydrogé nation pour obtenir respectivement les produits de formule (V_{B}) : 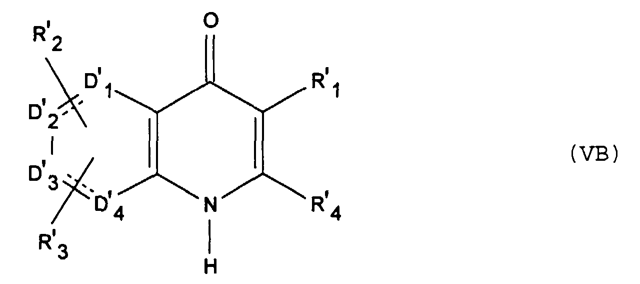 et les produits de formule (IX_{B}) : 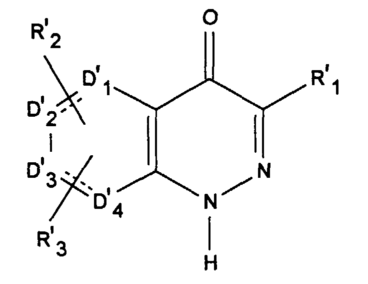 produits de formules (V_{B}) et (IX) dans lesquels D'₁, D'₂, D'₃, D'₄, R'₁, R'₂, R'₃ et R'₄ ont les significations indiquées ci-dessus, et produits de formules (V), (V_{B}), (IX) et (IX_{B}) que si désiré, l'on soumet à une réaction de transformation de la fonction oxo en une fonction thioxo et que l'on fait réagir avec un composé de formule (X) :

   Hal-CH₂-Y' (X)

   dans laquelle Hal représente un atome d'halogène et Y' a la signification indiquée ci-dessus, pour Y dans lesquelles les éventuelles fonctions réactives sont éventuellement protégées par des groupements protecteurs, pour obtenir un produit de formule (I') : 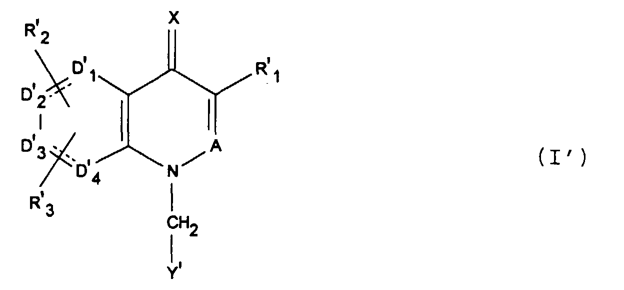 dans laquelle : 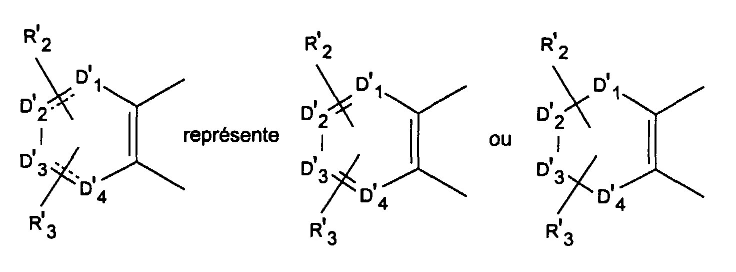 D'₁, D'₂, D'₃, D'₄, R'₁, R'₂, R'₃, R'₄, R'₅ et Y' ont la signification indiquée ci-dessus, A représente le radical C-R'₄ ou un atome d'azote, et X a la signification indiquée ci-dessus, produits de formule (I') dans laquelle 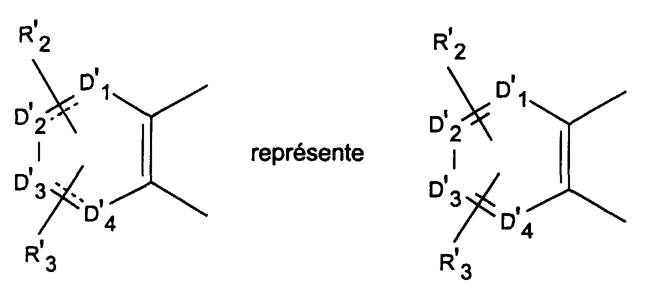 que l'on peut, si désiré, soumettre à une réaction d'hydrogénation pour obtenir des produits de formule (I') dans laquelle 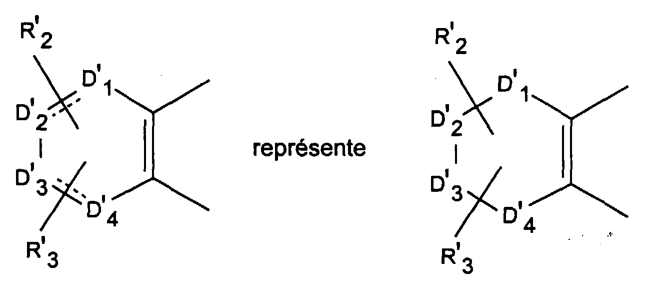 produit de formule (I') que l'on soumet, si désiré et si nécessaire, à l'une ou plusieurs des réactions suivantes, dans un ordre quelconque :
   - une réaction de transformation de fonction oxo C=O en fonction thioxo C=S,
   - une réaction d'élimination des groupements protecteurs que peuvent porter les fonctions réactives protégées,
   - une réaction de salification par un acide minéral ou organique ou par une base minérale ou organique pour obtenir le sel correspondant,
   - une réaction d'estérification de fonction acide,
   - une réaction de saponification de fonction ester en fonction acide,
   - une réaction de transformation de fonction alkyloxy en fonction hydroxyle,
   - une réaction de transformation de la fonction cyano en fonction acide,
   - une réaction de réduction de la fonction carboxy en fonction alcool,
   - une réaction de dédoublement des formes racémiques,
   les produits ainsi obtenus étant sous toutes les formes isomères possibles racémiques, énantiomères et diastéréoisomères.

Dans des conditions préférentielles de mise en oeuvre de l'invention, le procédé ci-dessus de préparation des produits de la présente invention et notamment de formule (I) peut être réalisé de la manière suivante :
le produit de formule (IV) peut être obtenu par réaction du dérivé carbonylé de formule (III) sur la fonction amine libre du produit de formule (II) par exemple en présence d'un solvant tel que par exemple du toluène ou un alcool tel que l'éthanol ou encore l'acide acétique au reflux ; la réaction peut également être réalisée en absence de solvant en portant le mélange au point de fusion des composés, en présence ou non, d'un agent dessicant tel que par exemple, de la Siliporite®.

La réaction de cyclisation du produit de formule (IV) ainsi formé en produit de formule (V) peut être réalisée par exemple par chauffage du composé à son point de fusion ou encore dans un solvant tel que par exemple l'oxyde de biphényle ou un mélange d'oxyde de phényle et de biphényle à une température d'environ 100 à 250°C.

Le produit de formule (VIII) peut être obtenu par réaction du produit de formule (VII) avec le dérivé halogèné de formule (VI) par exemple en présence d'un sel de cuivre tel que par exemple du iodure de cuivre et d'un catalyseur tel que par exemple un catalyseur au palladium.

La réaction de cyclisation du produit de formule (VIII) ainsi formé en produit de formule (IX) peut être réalisée par exemple par action d'acide nitreux obtenu in situ par exemple en présence de nitrite de sodium ou de potassium dans un milieu acide tel que par exemple dans de l'acide chlorhydrique.

La réaction d'hydrogénation des produits de formule (V) en produits de formule (V_{B}) ou des produits de formule (IX) en produits de formule (IX_{B}) peut être réalisée dans les conditions usuelles connues de l'homme de métier et par exemple dans un solvant tel que le méthanol, l'éthanol, l'acétate d'éthyle, l'acide acétique et par hydrogénation en présence par exemple d'oxyde de platine, de palladium sur charbon, de rhodium sur charbon.

Les produits de formules (V), (V_{B}), (IX) et (IX_{B}) peuvent être soumis à une réaction de transformation de la fonction oxo en fonction thioxo qui peut être réalisée selon les méthodes usuelles connues de l'homme de métier telles que par exemple à l'aide du réactif de Lawesson ou encore de pentasulfure de phosphore au reflux dans un solvant tel que par exemple le toluène ou un alcool tel que par exemple l'éthanol.

L'addition du composé de formule (X) sur la fonction amine libre du produit de formules (V), (V_{B}), (IX) ou (IX_{B}) pour obtenir des produits de formule (I') peut étre réalisée par exemple en présence d'une base faible telle que par exemple du carbonate de sodium ou de potassium ou encore en présence d'hydrure de sodium au reflux dans un solvant tel que par exemple l'acétone, l'éther ou encore le tétrahydrofuranne.

La fonction oxo des produits de formules (V), (V_{B}), (IX) et (IX_{B}), peut être également protégée et la réaction d'addition s'opère alors sur la fonction amine libre définie ci-dessus, par simple chauffage avec le produit de formule (X).

L'addition du composé de formule (X) sur la fonction amine libre du produit de formule (V) donne des produits de formule (I') dans laquelle A représente le radical =C-R'₄ tel que défini ci-dessus et l'addition du produit de formule (X) sur la fonction amine libre du produit de formule (IX) donne des produits de formule (I') dans laquelle A représente un atome d'azote.

Le produit de formule (X) peut également réagir avec la fonction oxo non protégée des produits de formules (V), (V_{B}), (IX) et (IX_{B}) pour donner des produits de formule (A) :

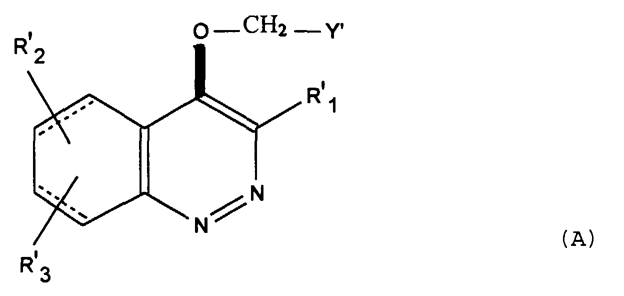

Dans ce cas, on peut obtenir un mélange de produits de formules (I') et (A) par réaction des produits de formules (V), (V_{B}), (IX) et (IX_{B}) avec le composé de formule (X) dans les conditions définies ci-dessus.

La réaction d'hydrogénation de produits de formule (I') pour obtenir d'autres produits de formule (I') peut être réalisée par exemple dans les mêmes conditions que celles indiquées ci-dessus pour l'hydrogénation de produits de formules (V) et (IX) respectivement en produits de formules (V_{B}) et (IX_{B}).

Selon les valeurs de R'₁, R'₂, R'₃, A, Y' et X, les produits de formule (I') ainsi obtenus constituent ou non des produits de la présente invention.

Les produits de formule (I') ainsi obtenus, en particulier pour donner des produits de la présente invention peuvent être soumis, si désiré et si nécessaire, à l'une ou plusieurs des réactions indiquées ci-dessus.

Parmi celles-ci, la réaction de transformation de la fonction oxo en fonction thioxo peut être réalisée ainsi qu'il est indiqué ci-dessus.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, trialkylsilyle, dihydropyranne, méthoxyméthyle ou tétrahydropyrannyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acide des produits décrits ci-dessus peuvent être, si désiré, amidifiées par une amine primaire ou secondaire par exemple dans du chlorure de méthylène en présence de chlorhydrate de 1-éthyl-3-(diméthylaminopropyl) carbodiimide à la température ambiante,
- les fonctions acide peuvent être protégées par exemple sous forme d'esters formés avec les esters facilement clivables tels que les esters benzyliques ou ter butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique ou par une base minérale ou organique, en particulier sur les éventuelles fonctions carboxy, ces réactions pouvant être réalisées selon les méthodes usuelles connues de l'homme de métier.

Les produits décrits ci-dessus peuvent, si désiré, faire l'objet, sur les éventuelles fonctions carboxy, de réactions d'estérification qui peuvent être réalisées selon les méthodes usuelles connues de l'homme de métier.

Les éventuelles fonctions ester des produits décrits ci-dessus peuvent être, si désiré, saponifiées en fonction acide, ces réactions de saponification pouvant être réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions alkyloxy telles que notamment méthoxy des produits décrits ci-dessus peuvent être, si désiré, transformées en fonction hydroxyle dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

Les éventuelles fonctions cyano des produits décrits ci-dessus peuvent être, si désiré, transformées en fonction acide dans les conditions usuelles connues de l'homme de métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions carboxy ou carboxy estérifiées des produits décrits ci-dessus peuvent, si désiré, être réduites en fonction alcool par les méthodes connues de l'homme de métier et notamment par de l'hydrure de lithium et d'aluminium dans un solvant tel que par exemple le tétrahydrofuranne ou encore le dioxanne ou l'éther éthylique.

Les éventuelles formes optiquement actives des produits de formule (I) peuvent être préparées par dédoublement des racémiques selon les méthodes usuelles.

L'invention a aussi pour objet un procédé de préparation des produits de la présente invention et notamment de formule (I) caractérisé en ce que l'on prend les produits de formules (V), (V_{B}), (IX) et (IX_{B}) comme produits de départ et en ce que l'on opère comme indiqué précédemment.

L'invention a aussi pour objet un procédé de préparation des produits de formules (V), (V_{B}), (IX) et (IX_{B}) caractérisé en ce que l'on prend les produits de formule (II) comme produits de départ et en ce que l'on opère comme indiqué précédemment.

L'invention a de même pour objet un procédé de préparation de produits de formule (I_{EB}), correspondant aux produits de la présente invention et notamment de formule (I), telle que définie ci-dessus, dans laquelle A représente le radical CH caractérisé en ce que :
**soit** l'on fait réagir un produit de formule (II_{E}) : 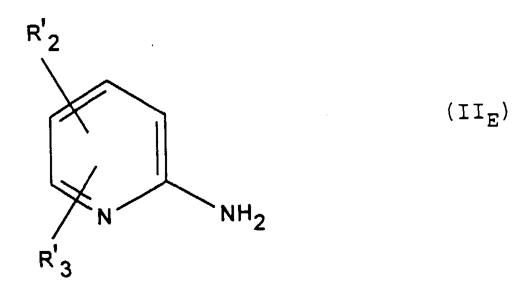 dans laquelle R'₂ et R'₃ ont les significations indiquées ci-dessus, respectivement pour R₂ et R₃ dans lesquelles les éventuelles fonctions réactives sont éventuellement protégées par des groupements protecteurs, avec un produit de formule (XI) : 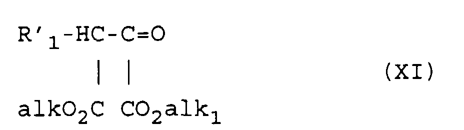 dans laquelle R'₁ a la signification indiquée ci-dessus, pour R₁ dans laquelle les éventuelles fonctions réactives sont éventuellement protégées par des groupements protecteurs et alk et alk₁, identiques ou différents, représentent un radical alkyle renfermant au plus 6 atomes de carbone, pour obtenir des produits de formule (XII) : 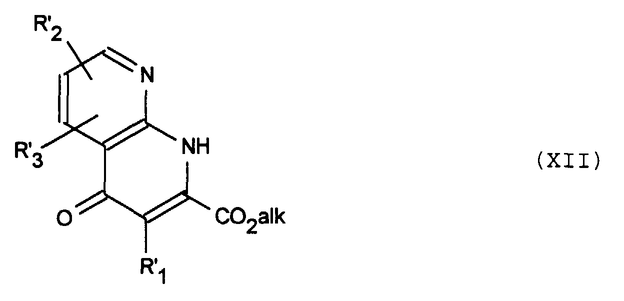 dans laquelle R'₁, R'₂, R'₃ et alk ont les significations indiquées ci-dessus,
**soit** l'on fait réagir un produit de formule (II_{F}) : 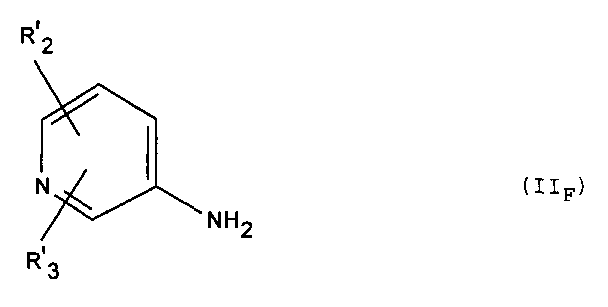 dans laquelle R'₂ et R'₃ ont les significations indiquées ci-dessus, avec le produit de formule (XI) tel que défini ci-dessus, pour obtenir des produits de formule (XIII) : 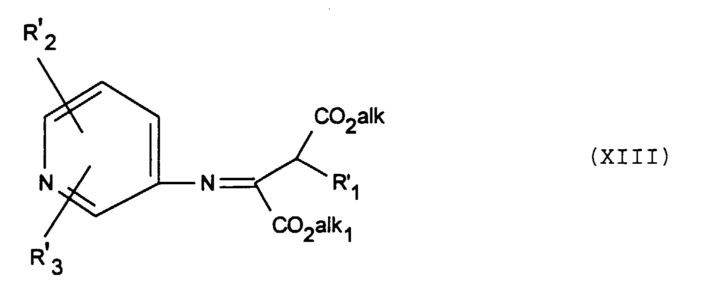 dans laquelle R'₁, R'₂, R'₃, alk et alk₁ ont les significations indiquées ci-dessus, produits de formules (XII) et (XIII) que l'on transforme en produit de formule (XIV) : 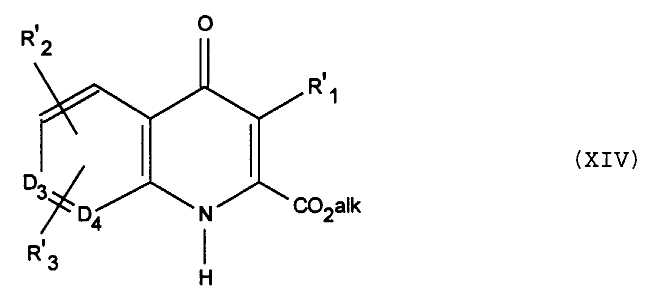 dans laquelle R'₁, R'₂, R'₃ et alk₁ ont les significations indiquées ci-dessus et D₃ et D₄ ont les significations indiquées ci-dessus, produits de formule (XIV) que, si désiré, l'on soumet à une réaction d'hydrogénation pour obtenir les produits de formule (XV) : 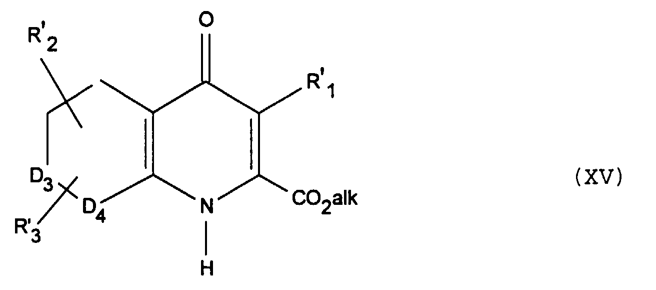 dans laquelle D₃, D₄, R'₁, R'₂, R'₃ et alk₁ ont les significations indiquées ci-dessus, produits de formules (XIV) et (XV) que l'on soumet à une réaction de saponification puis de décarboxylation, pour obtenir les produits correspondants de formule (XIV') : 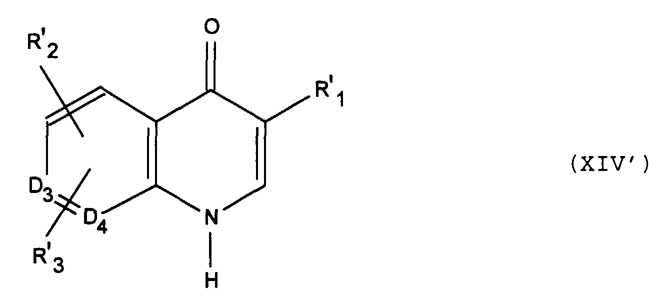 et de formule (XV') : 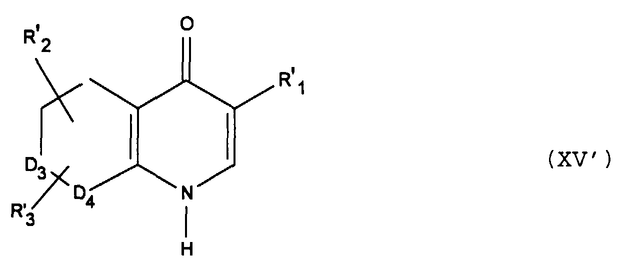 produits de formules (XIV') et (XV') que si désiré, l'on soumet à une réaction de transformation de la fonction oxo en une fonction thioxo et que l'on fait réagir avec un composé de formule (X) :

   Hal-CH₂-Y' (X)

   dans laquelle Y'a la signification indiquée ci-dessus pour Y dans laquelle les éventuelles fonctions réactives sont éventuellement protégées par des groupements protecteurs, pour obtenir un produit de formule (I'_{EB}) : 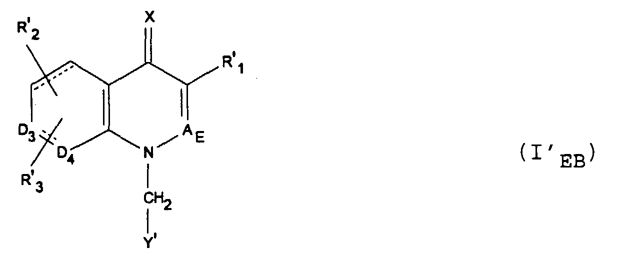 dans laquelle : 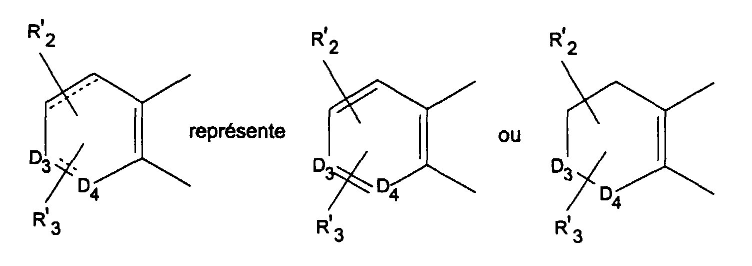 D₃, D₄, R'₁, R'₂, R'₃ et Y' ont les significations indiquées ci-dessus, A_{E} représente le radical CH et X a la signification indiquée ci-dessus, produits de formule (I'_{EB}) dans laquelle 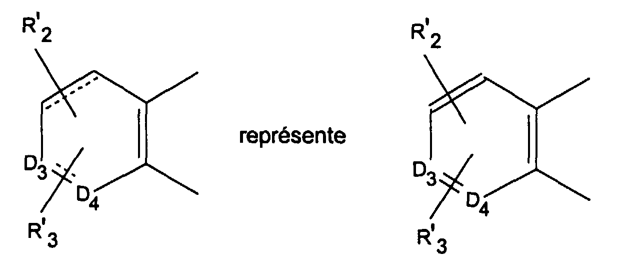 que l'on peut, si désiré, soumettre à une réaction d'hydrogénation pour obtenir des produits de formule (I'_{EB}) dans laquelle 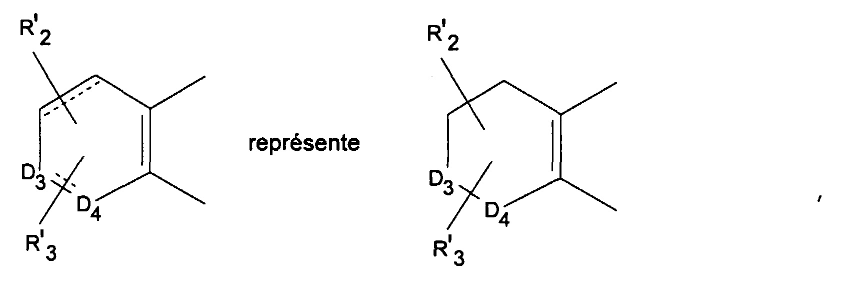 produit de formule (I'_{EB}) que l'on soumet, si désiré et si nécessaire, à l'une ou plusieurs des réactions suivantes, dans un ordre quelconque :
   - une réaction de transformation de fonction oxo C=O en fonction thioxo C=S,
   - une réaction d'élimination des groupements protecteurs que peuvent porter les fonctions réactives protégées,
   - une réaction de salification par un acide minéral ou organique ou par une base minérale ou organique pour obtenir le sel correspondant,
   - une réaction d'estérification de fonction acide,
   - une réaction de saponification de fonction ester en fonction acide,
   - une réaction de transformation de fonction alkyloxy en fonction hydroxyle,
   - une réaction de transformation de la fonction cyano en fonction acide,
   - une réaction de réduction de la fonction carboxy en fonction alcool,
   - une réaction de dédoublement des formes racémiques,
   les produits ainsi obtenus étant sous toutes les formes isomères possibles racémiques, énantiomères et diastéréoisomères.

Dans des conditions préférentielles de mise en oeuvre de l'invention, le procédé ci-dessus de préparation des produits de formule (I_{EB}) telle que définie ci-dessus, peut-être réalisé de la manière suivante :
le produit de formule (XII), à partir des produits de formules (II_{E}) et (XI) et le produit de formule (XIII) à partir des produits de formules (II_{F}) et (XI) peuvent être respectivement obtenus dans les conditions décrites ci-dessus d'obtention du produit de formule (IV) à partir des produits de formules (II) et (III).

Les produits de formule (XIV) sont obtenus par chauffage, par exemple dans du Dowtherm ou de la paraffine à des températures comprises entre 250 et 350°C, à partir des produits de formules (XII) et (XIII), les produits de formule (XII) donnant les produits de formule (XIV) dans lesquels D₄ représente un atome d'azote et D₃ représente un radical méthine éventuellement substitué par un radical choisi parmi R'₂ et R'₃ et les produits de formule (XIII) donnant les produits de formule (XIV) dans lesquels D₃ représente un atome d'azote et D₄ représente un radical méthine tel que défini ci-dessus.

La réaction d'hydrogénation des produits de formule (XIV) en produits de formule (XV) peut être réalisée dans les conditions décrites ci-dessus d'hydrogénation des produits de formule (V) en produits de formule (V_{B}) ou encore du produit de formule (IX) en produit de formule (IX_{B}). La réaction de décarboxylation des produits de formule (XV) en produits de formule (XV') peut être réalisée dans les conditions usuelles connues de l'homme de métier et notamment par saponification de l'ester, en présence d'une base telle que par exemple l'hydroxyde de sodium ou de potassium, suivi d'une acidification puis d'un chauffage à une température d'environ 150-250°C, dans un solvant tel que le Dowtherm.

L'addition du composé de formule (X) sur les produits de formules (XV) et (XV') pour obtenir les produits de formule (I'_{EB}) telle que définie ci-dessus peut être réalisée dans les conditions d'addition du composé de formule (X) sur les produits de formules (V), (V_{B}), (IX) ou (IX_{B}) pour obtenir les produits de formule (I').

Les réactions auxquelles, si désiré ou si nécessaire, l'on soumet les produits de formule (I'_{EB}) pour obtenir des produits de formule (I_{EB}) peuvent être réalisées dans les conditions décrites pour les réactions correspondantes auxquelles peuvent être soumis les produits de formule (I') pour obtenir des produits de formule (I).

Les composés de la présente invention et notamment de formule (I), tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Certains produits de la présente invention possèdent également des propriétés antagonistes pour le récepteur à l'endothéline et sont ainsi notamment antagonistes de l'effet vasoconstricteur de l'endothéline.

Les composés de la présente invention possèdent également la propriété d'améliorer les fonctions cognitives.

Ces propriétés justifient leur application en thérapeutique et l'invention a également pour objet à titre de médicaments, les produits de la présente invention tels que définis ci-dessus, ces produits étant sous toutes les formes isomères possibles racémiques ou optiquement actives, ainsi que les sels d'addition avec les acides minéraux ou organiques pharmaceutiquement acceptables desdits produits de la présente invention.

L'invention a particulièrement pour objet à titre de médicaments les produits tels que définis ci-dessus.

L'invention a plus particulièrement pour objet, à titre de médicaments, les produits décrits ci-aprés dans les exemples et notamment les produits de formule (I) suivants :
. l'acide 4'-[(3-butyl 1,4-dihydro-4-oxo-1-quinoléinyl) méthyl] -(1,1'-biphényl)-2-carboxylique,
. l'acide 4'-[(3-butyl 1,4-dihydro-4-thioxo-1-quinoléinyl) méthyl] (1,1'-biphényl) 2-carboxylique,
. l'acide 3-butyl 1-[(2'-carboxy (1,1'-biphényl) 4-yl) méthyl] 1,4-dihydro-4-oxo-7-quinoléinecarboxylique,
. l'acide 4'-[(3-butyl 1,4-dihydro 8-bromo 4-oxo 1-quinoléinyl) méthyl] (1,1'-biphényl) 2-carboxylique,
. l'acide 4'-[(3-butyl 1,4-dihydro 7-(hydroxyméthyl) 4-oxo 1-quinoléinyl) méthyl] (1,1'-biphényl) 2-carboxylique,
. l'acide 4'-[(3-butyl 1,4-dihydro 4-oxo 7-[(1-pyrrolidinyl) carbonyl] 1-quinoléinyl) méthyl] (1,1'-biphényl) 2-carboxylique,
. l'acide 4'-[(3-butyl 1,4,5,6,7,8-hexahydro 4-oxo 1-[(1-quinoléinyl) méthyl] (1,1'-biphényl) 2-carboxylique,
. l'acide 4'-[(3-butyl 1,4,5,6,7,8-hexahydro 6-méthyl 4-oxo 1-quinoléinyl) méthyl] (1,1'-biphényl) 2-carboxylique,
. l'acide 3-butyl 1,4-dihydro 1-[[2'-(1H-tétrazol-5-yl) (1,1'-biphényl) 4-yl] méthyl] 8-quinoléinecarboxylique,
. l'acide 1,4-dihydro 3-méthyl 4-oxo 1-[[2'-(1H-tétrazol-5-yl) (1,1'-biphényl) 4-yl] méthyl] 8-quinoléinecarboxylique,
. l'acide 1,4-dihydro 3-éthyl 4-oxo 1-[[2'-(1H-tétrazol-5-yl) (1,1'-biphényl) 4-yl] méthyl] 8-quinoléinecarboxylique,
ainsi que leurs sels d'addition avec les acides minéraux ou organiques pharmaceutiquement acceptables.

Les médicaments, objet de l'invention, peuvent être utilisés dans le traitement des affections cardiovasculaires présentant une altération de la vasomotricité : infarctus du myocarde, insuffisance cardiaque, insuffisance rénale, angine de poitrine, spasme vasculaire cérébral, maladie de Raynaud, hypertension artérielle et toutes les affections consécutives à une ischémie. Ces médicaments, objet de l'invention, pourraient également être utilisés pour le traitement du glaucome, de l'athérosclérose, de l'asthme et de différents types de spasmes viscéraux, ainsi qu'à titre de substances protectrices neuronales ou encore dans la prévention des resténoses post-angioplastie.

Ils peuvent également être utilisés dans le traitement de certains désordres gastro-intestinaux, gynécologiques et en particulier pour un effet relaxant au niveau de l'utérus.

Les médicaments, objet de l'invention peuvent également être utilisés dans le traitement des troubles de la mémoire et des fonctions cognitives, de l'anxiété, de la dépression, de la démence sénile et de la maladie d'Alzheimer.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (II), (II_{E}), (II_{F}), (III), (VI), (VII), (X) et (XI), peuvent être disponibles dans le commerce ou peuvent être préparés selon les méthodes usuelles connues de l'homme de métier.

Parmi les composés de formule (II) qui peuvent être trouvés dans le commerce, on peut citer par exemple certains composés de formule (II) telle que l'un de R'₂ ou R'₃ représente un radical amino comme par exemple le méta-aminobenzoate de méthyle que l'on peut trouver, par exemple, sous forme de produit commercialisé par exemple par LANCASTER.

Comme autres composés de formule (II) qui peuvent être trouvés dans le commerce, on peut citer par exemple certains composés de formule (II) telle que l'un de R₂' ou R₃' représente un radical nitro comme par exemple l'orthonitroaniline que l'on peut trouver, par exemple, sous forme de produit commercialisé par UCB.

Comme composé de départ de formule (II) on peut citer, par exemple, 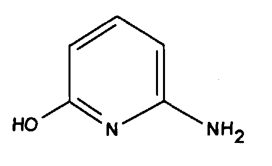 commercialisé par Columbia, 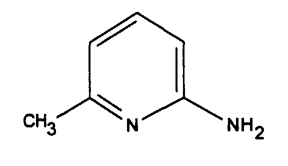 commercialisé par Aldrich.

Comme composés de départ de formules (II_{E}) et (II_{F}) on peut citer, par exemple, 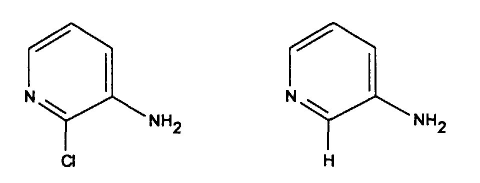 ces deux composés étant commercialisés par Aldrich.

Les composés de formule (III) peuvent constituer des esters dérivés de l'acide formylacétique.

Parmi les exemples de préparation de tels composés de formule (III) décrits dans la littérature, on peut citer notamment les références suivantes :
J. Hel. Chem. 20, p. 623-628 (1983)
Liebigs Ann. Chem. 697, p. 62-68 (1966).

Les composés de formules (VI) peuvent constituer notamment des dérivés de l'ortho-halo aniline.

Parmi les exemples de préparation de tels composés de formules (VI) décrits dans la littérature, on peut citer notamment les références suivantes :
Ann. Chim. 1962, 52, p. 727.

Les composés de formule (VII) peuvent constituer notamment des dérivés de l'acétylène.

Parmi les exemples de préparation de tels composés de formule (VII) décrits dans la littérature, on peut citer notamment les références suivantes :
J. Am. Chem. Soc. 1937, 59, p. 1490.

Des exemples de préparation de composés de formule (X) sont décrits dans la littérature et des exemples en sont donnés notamment dans les brevets US 4,880,804 ou EP 0 253 310.

Un procédé de préparation de certains produits de formule (X) telle que définie ci-dessus peut consister à soumettre le iodobenzoate de méthyle à l'action du iodotoluène, la réaction se réalisant par exemple en présence de cuivre en poudre à une température d'environ 100°C à 300°C, pour obtenir un produit de formule (X_{c}) : 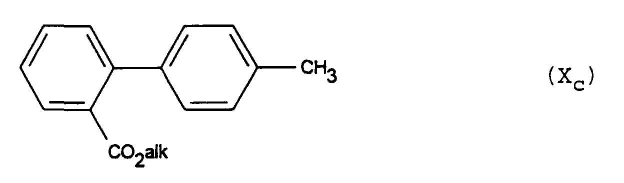 dont le radical carboxy estérifié peut, si désiré, être libéré du radical alkyle par les méthodes classiques connues de l'homme de métier ou indiquées ci-dessus, par exemple d'hydrolyse acide ou alcaline, que l'on peut soumettre à une réaction de bromation sur le radical méthyle par les méthodes classiques connues de l'homme de métier par exemple par action du n-bromosuccinimide dans le tétrachlorure de carbone.

Comme composé de départ de formule (XI), on peut citer, par exemple, 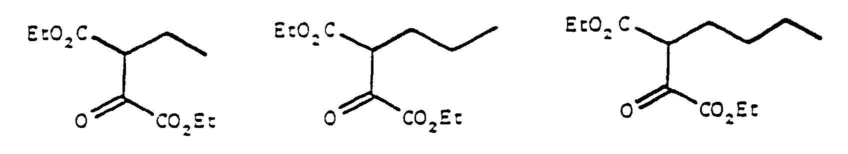 préparés par exemple selon la réaction 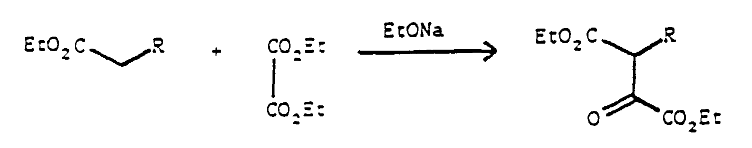 (J. of Het. Chem. 20 p. 623, 1983) et 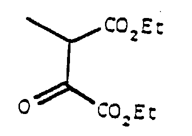 (diéthyloxalpropionate) commercialisé par Aldrich.

La présente invention a ainsi pour objet l'utilisation des produits de l'invention :
pour la préparation de médicaments, destinés :
**soit** au traitement de l'hypertension artérielle, des insuffisances cardiaques, des insuffisances rénales et dans la prévention des resténoses post-angioplastie,
**soit** au traitement de certains désordres gastro-intestinaux ou gynécologiques,
**soit** au traitement des troubles de la mémoire et des fonctions cognitives, de l'anxiété, de la dépression, de la démence sénile et de la maladie d'Alzheimer.

La présente invention a particulièrement pour objet l'utilisation telle que définie ci-dessus, caractérisée en ce que les produits sont utilisés dans la préparation de médicaments destinés au traitement de l'hypertension artérielle, des insuffisances cardiaques, des insuffisances rénales, dans la prévention des resténoses post-angioplastie, ainsi que dans le traitement de diverses formes de spasmes vasculaires.

La présente invention a tout particulièrement pour objet l'utilisation telle que définie ci-dessus, caractérisée en ce que les produits sont utilisés dans la préparation de médicaments destinés au traitement des troubles de la mémoire et des fonctions cognitives, de l'anxiété, de la dépression, de la démence sénile et de la maladie d'Alzheimer.

Les exemples suivants illustrent l'invention sans toutefois la limiter.

### Préparation de l'exemple 1 : 3-butyl 1H quinoléinone

Le produit utilisé au départ de l'exemple 1 a été préparé de la façon suivante.

### Stade A : 2-butyl 3-oxo butanedioate de diéthyle

A une suspension d'éthylate de sodium (préparé par agitation pendant 1 heure à 40°C de 2,3 g de sodium et 150 cm³ d'éthanol, puis évaporation du solvant), dans 100 cm³ d'éther, on ajoute 13,55 cm³ de diéthyl oxalate. On chauffe 15 minutes au reflux, refroidit légèrement et ajoute 50 cm³ de caproate d'éthyle, on agite 3 heures au reflux et 16 heures à 30-35°C. On ajoute 50 cm³ d'eau sépare la phase aqueuse, par décantation, la lave avec de l'éther par deux fois et acidifie avec de l'acide chlorhydrique 2N. On extrait 3 fois à l'éther, lave les phases organiques à l'eau puis avec une solution saturée de chlorure de sodium, sèche et évapore à sec. Le produit obtenu, 9,5 g est utilisé tel quel pour le stade suivant.

### Stade B : 2-butyl 3-(phénylamino) 2-butènedioate de diéthyle

On agite 3 jours à 75°C, un mélange de 1 g d'aniline avec 2,65 g du produit obtenu au stade A ci-dessus et 150 mg de siliporite ® NK10, on refroidit et chromatographie le milieu réactionnel sur silice (éluant : hexane-acétate d'éthyle (9-1)) on obtient 1,55 g de produit attendu.

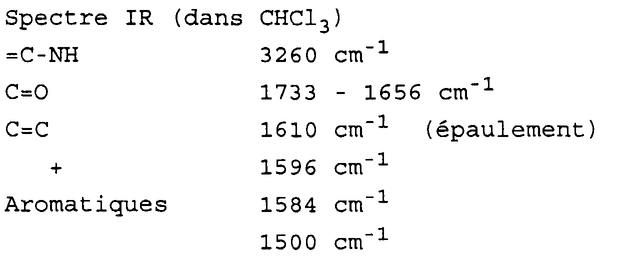

### Stade C : 3-butyl 4-hydroxy 2-quinoléine carboxylate d'éthyle et 3-butyl 1,4-dihydro 4-oxo 2-quinoléine carboxylate d'éthyle

On chauffe pendant 30 minutes à l'aide d'un bain à 250°C un mélange de 2,5 g du produit obtenu au stade B ci-dessus, avec 30 cm³ de diphényléther. On laisse revenir à température ambiante, essore, lave avec du pentane et recueille 1,82 g de produit recherché.

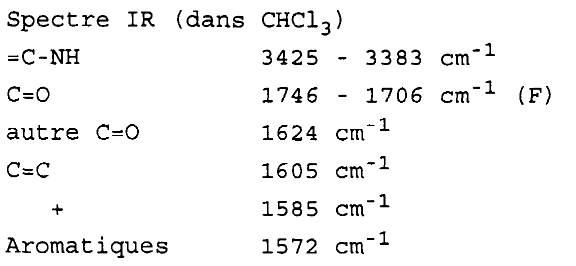

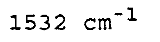

### Stade D : Acide 3-butyl 1,4-dihydro 4-oxo 2-quinoléine carboxylique

On chauffe 1 heure à 60°C, 1,8 g de l'ester obtenu au stade C ci-dessus, avec 25 cm³ de soude en solution N. On refroidit et acidifie avec de l'acide chlorhydrique N, on filtre, lave à l'eau et sèche à 60°C sous pression réduite, on obtient 1,58 g du produit recherché.

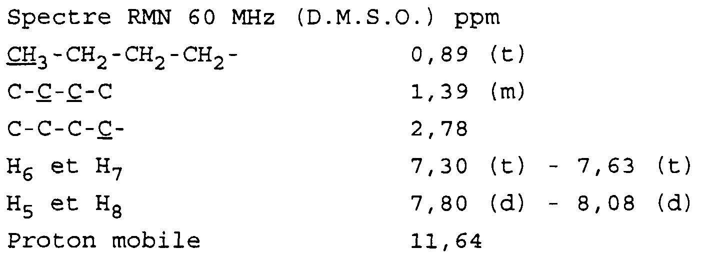

### Stade E : 3-butyl 4(1H)-quinoléinone

On chauffe 30 minutes à 250°C, un mélange de 1,55 g du produit obtenu au stade D ci-dessus et 10 cm³ de diphényl éther. On refroidit, filtre, lave avec du pentane et sèche sous pression réduite. On obtient 1,17 g de produit que l'on dissout dans 80 cm³ d'éthanol et traite 15 minutes au reflux en présence de charbon actif, on filtre sur hyflosupercel, évapore l'éthanol jusqu'à sec et reprend avec du pentane, filtre, lave avec du pentane et sèche à 50°C sous pression réduite. On obtient 971 mg de produit recherché.

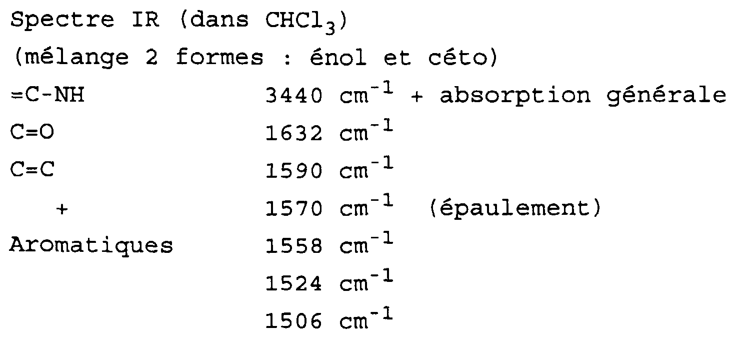

### EXEMPLE 1 : 4-(3-butyl 1,4-dihydro 4-oxo quinoléinyl méthyl) benzonitrile

A une suspension de 2,3 g du produit obtenu au stade E de la préparation de l'exemple 1 dans 50 cm³ d'acétone, on ajoute 3,3 g de carbonate de potassium activé. On agite 10 minutes puis on ajoute 4,7 g de 4-bromobenzonitrile, on chauffe pendant 4 heures au reflux. On évapore l'acétone et reprend le résidu avec 100 cm³ d'eau. On extrait avec de l'acétate d'éthyle, lave avec une solution saturée de chlorure d'ammonium, sèche et évapore à sec. On obtient 4,1 g du produit attendu que l'on chromatographie sur silice (éluant : chlorure de méthylène-méthanol (98-2)). On recueille 3,3 g du produit recherché. F = 168°C.

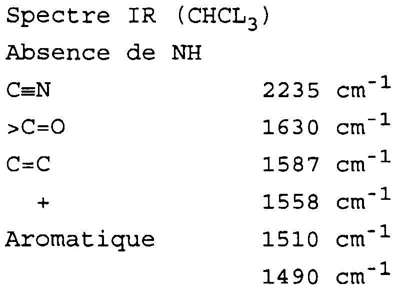

### EXEMPLE 2 : Acide 4-[(3-butyl 1,4-dihydro 4-oxo 1-quinoléinyl) méthyl] benzoïque

On chauffe au reflux, sous agitation, pendant 4 heures un mélange de 0,5 g du produit obtenu à l'exemple 1 avec 8 cm³ d'éthanol et 1,6 cm³ de soude en solution 5N. On refroidit à 0°C et acidifie avec de l'acide chlorhydrique concentré. On agite 1 nuit à 0°C, essore, lave à l'eau, sèche, évapore à sec. On obtient 0,48 g de produit que l'on recristallise dans 20 cm³ d'éthanol. On recueille 275 mg du produit recherché. F = 224°C.

| Analyse pour C₂₁H₂₁NO₃ = 335,41 | | | |
|---|---|---|---|
| calculés : | C% 75,20 | H% 6,31 | N% 4,17 |
| trouvés : | 75,3 | 6,3 | 4,1 |

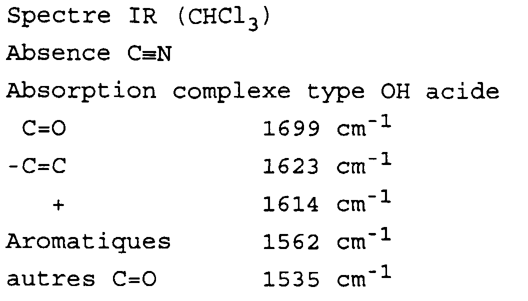

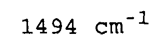

### EXEMPLE 3 : 4'-[(3-butyl 1,4-dihydro 4-oxo 1-quinoléinyl) méthyl] (1,1'-biphényl) 2-carboxylate de méthyle

On opère comme à l'exemple 1, à partir de 4,02 g du composé obtenu au stade E de la préparation de l'exemple 1, en utilisant 5,52 g de carbonate de potassium et 6,2 g de bromométhyl-(1,1'-biphényl) 2-carboxylate de méthyle (préparation selon EP 0253310). Après chromatographie sur silice (éluant : chlorure de méthylène-méthanol (98-2)). On obtient 8 g du produit recherché.

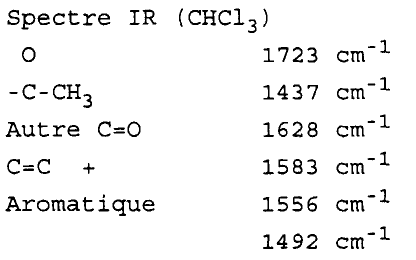

### EXEMPLE 4 : Acide 4'-[(3-butyl 1,4-dihydro 4-oxo 1-quinoléinyl) méthyl] (1,1'-biphényl) 2-carboxylique

On opère comme à l'exemple 2 à partir de 0,48 g du produit obtenu à l'exemple 3.

Après recristallisation dans l'éthanol on recueille 0,240 g de produit recherché. F = 214°C.

| Analyse pour : C₂₇H₂₅NO₃ = 411,51 | | | |
|---|---|---|---|
| calculés : | C% 78,8 | H% 6,12 | N% 3,403 |
| trouvés : | 78,7 | 6,1 | 3,4 |

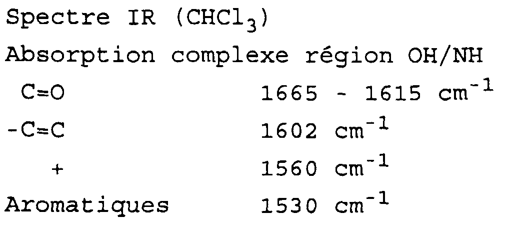

### EXEMPLE 5 : 4'-[(3-butyl 1,4-dihydro 4-thioxo 1-quinoléinyl) méthyl] (1,1'-biphényl) 2-carboxylate de méthyle

A une solution de 400 mg du produit obtenu à l'exemple 3, dans 20 cm³ de toluène, on ajoute 210 mg de réactif de Lawesson et chauffe 1 heure 30 au reflux. On verse dans 100 cm³ d'eau et extrait avec de l'acétate d'éthyle, lave à l'eau, sèche, filtre et évapore à sec, on obtient 0,6 g du produit attendu que l'on chromatographie sur silice (éluant : chlorure de méthylène - acétone (98-2)) pour recueillir 0,42 g du produit recherché F = 158°C.

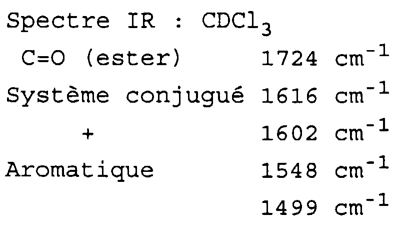

### EXEMPLE 6 : Acide 4'-[(3-butyl 1,4-dihydro 4-thioxo 1-quinoléinyl) méthyl] (1,1'-biphényl) 2-carboxylique

On opère comme à l'exemple 2 à partir de 400 mg de produit obtenu à l'exemple 5 en utilisant 15 cm³ de soude concentrée, on recueille 0,35 g de produit brut F > 260°C. Après recristallisation dans l'éthanol, on obtient 270 mg du produit recherché. F > 260°C.

| Analyse pour C₂₇H₂₅NO₂S = 427,57 | | | | |
|---|---|---|---|---|
| calculés : | C% 75,85 | H% 5,89 | N% 3,27 | S% 7,5 |
| trouvés : | 75,7 | 5,8 | 3,2 | 7,5 |

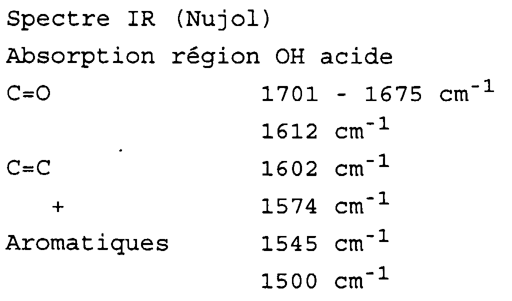

### Préparation de l'exemple 7 : 3-butyl 5-(méthylthio) 4-(1H) quinoléinone

### Stade A : 2-butyl 3-[[(3-méthylthio) phényl] amino] 2-butène dioate de diéthyle

A une solution de 4,3 g de produit obtenu au stade A de la préparation de l'exemple 1 avec 2 cm³ de 3-méthyl mercapto aniline, dans 100 cm³ de toluène, on ajoute 50 mg d'acide paratoluène sulfonique. On agite 4 heures au reflux en éliminant l'eau formée. On évapore à sec et chromatographie le résidu sur silice (éluant : chlorure de méthylène à 30 % d'hexane), on obtient 5,1 g du produit recherché. F = 55°C.

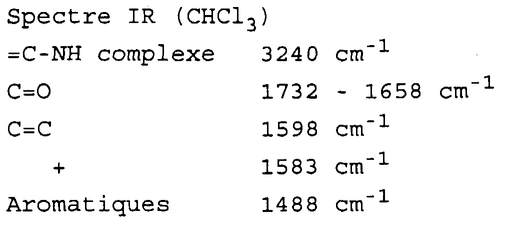

### Stade B : 3-butyl 1,4-dihydro 5-(méthylthio) 4-oxo 2-quinoléine carboxylate d'éthyle

On chauffe 45 minutes à 250°C, 1 g du produit obtenu au stade A. On refroidit et chromatographie sur silice le produit brut réactionnel (éluant : chlorure de méthylène) on obtient 700 mg du produit recherché. F = 80°C.

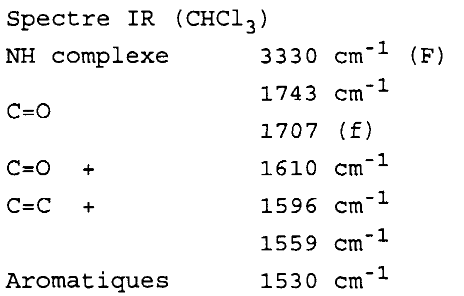

### Stade C : Chlorhydrate de l'acide 3-butyl 1,4-dihydro 5-(méthylthio) 4-oxo 2-quinoléine carboxylique

On agite 2 heures au reflux 0,63 g du produit obtenu au stade B ci-dessus dans 10 cm³ d'une solution N de soude. On verse dans l'eau glacée, acidifie avec de l'acide chlorhydrique concentré, essore, lave à l'eau, sèche et empâte dans 100 cm³ d'acétate d'éthyle. On obtient 495 mg du produit recherché. F = 240°C.

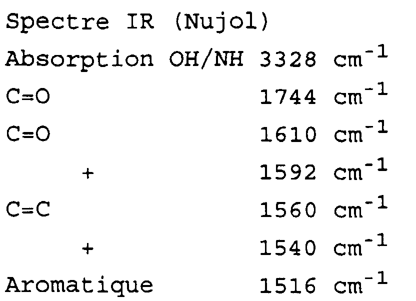

### Stade D : 3-butyl 5-(méthylthio) 4-(1H) quinoléinone

On chauffe 5 minutes à 260°C, 390 mg du produit obtenu au stade C. On obtient 300 mg de produit recherché. F = 144°C.

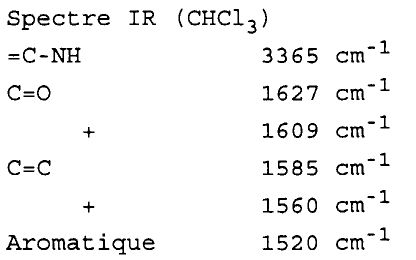

### EXEMPLE 7 : 4'-[[3-butyl 1,4-dihydro 5-(méthylthio) 4-oxo 1-quinoléinyl] méthyl] (1,1'-biphényl) 2-carboxylate de méthyle

On opère comme à l'exemple 1, à partir de 1,1 g du produit obtenu au stade D de la préparation de l'exemple 7, en utilisant 1,63 g de 4-bromométhyl (1,1'-biphényl) 2-carboxylate de méthyle (préparé selon EP 0253310) et 1,2 g de carbonate de potassium. Après chromatographie sur silice (éluant : acétate d'éthyle-hexane (5-5)). On obtient 270 mg du produit attendu.

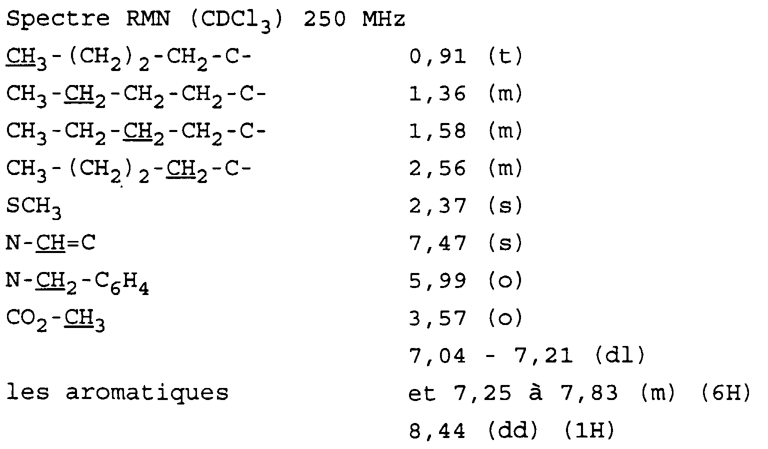

### EXEMPLE 8 : Acide 4'-[(3-butyl 1,4-dihydro 5-(méthylthio) 4-oxo 1-quinoléinyl) méthyl] (1,1'-biphényl) 2-carboxylique

On agite pendant 2 heures au reflux un mélange de 270 mg du produit obtenu à l'exemple 7 avec 5 cm³ d'une solution de soude 5N dans 5 cm³ d'éthanol. On coule dans l'eau et acidifie avec de l'hydrogénophosphate de sodium, extrait avec de l'acétate d'éthyle, lave à l'eau, sèche et évapore à sec On chromatographie le résidu sur silice (éluant : chlorure de méthylène-méthanol (9-1)). On empâte le produit obtenu dans un mélange de 5 cm³ d'acétate d'étyle et 5 cm³ d'éther isopropylique. On recueille ainsi 90 mg du composé recherché. F = 155°C.

| Analyse pour C₂₈H₂₇NO₃S = 457,60 | | | | |
|---|---|---|---|---|
| calculés : | C% 73,5 | H% 5,95 | N% 3,06 | S% 7,00 |
| trouvés : | 73,5 | 5,8 | 3,2 | 6,8 |

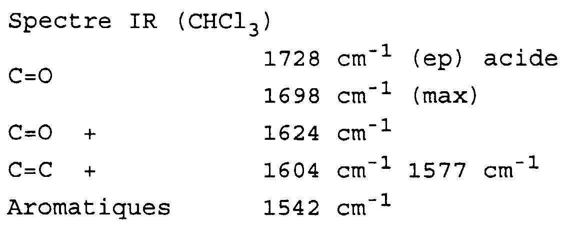

### Préparation de l'exemple 9 : 3-butyl 1,4-dihydro 4-oxo 7-quinoléine carboxylate de méthyle

### Stade A : 2-butyl 3-[[3-(méthoxycarbonyl) phényl] amino] 2-butènedioate de diéthyle

On prépare une solution de 3,02 g de méta amino benzoate de méthyle et de 4,9 g de 2-butyl 3-oxo butanedioate de diéthyle obtenu au stade A de la préparation de l'exemple 1 dans 100 cm³ de toluène à laquelle on ajoute 20 mg d'acide paratoluène sulfonique. On agite au reflux pendant environ 4 heures. On évapore à sec et chromatographie le résidu sur silice (éluant : acétate d'éthyle - hexane (2-8)). On obtient 6,5 g de produit attendu.

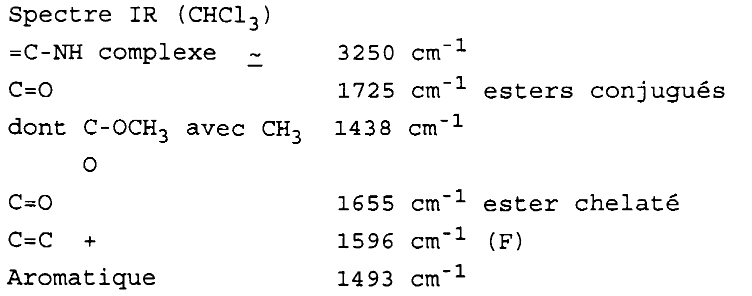

### Stade B : 3-butyl 1,4-dihydro 4-oxo 2,7-quinoléine dicarboxylate de 2-éthyle 7-méthyle

On opère comme au stade B de la préparation de l'exemple 7 à partir de 6 g du produit obtenu au stade A ci-dessus. On obtient après chromatographie sur silice (éluant : acétate d'éthyle-hexane (4-6)) 1,8 g du produit attendu. F = 210°C.

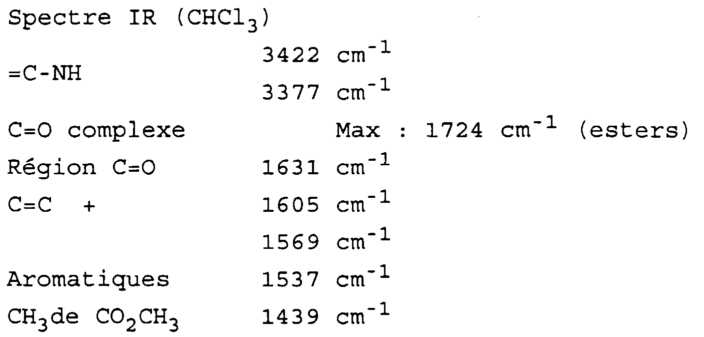

### Stade C : Acide 3-butyl 1,4-dihydro 4-oxo 2,7-quinoléine dicarboxylique

On opère comme au stade C de la préparation de l'exemple 7 à partir de 1,8 g du produit obtenu ci-dessus au stade B. On obtient 1,27 g du produit attendu. F > 260°C.

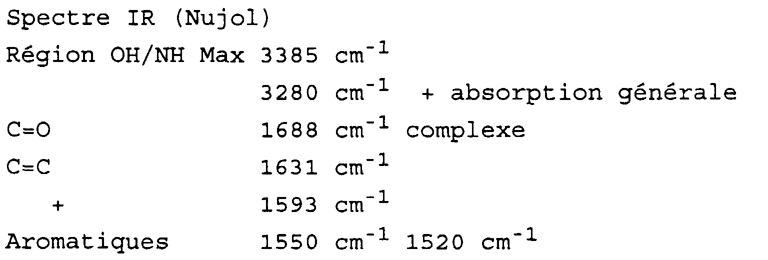

### Stade D : Acide 3-butyl 1,4-dihydro 4-oxo 7-quinoléine carboxylique

On opère comme au stade D de la préparation de l'exemple 7 à partir de 1,23 g du produit obtenu au stade C ci-dessus. On obtient 1 g du produit attendu PF > 260°C.

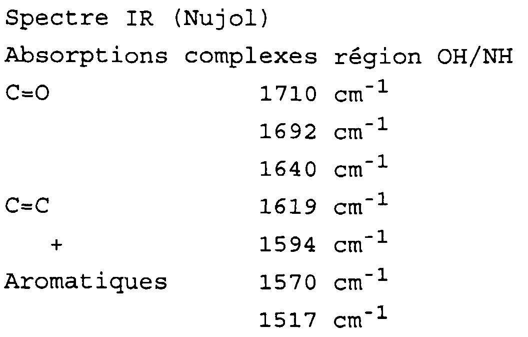

### Stade E : 3-butyl 1,4-dihydro 4-oxo 7-quinoléine carboxylate de méthyle

A une solution dans le chlorure de méthylène du produit obtenu ci-dessus au stade D, on ajoute du diazométhane jusqu'à coloration jaune persistante. L'excès de diazométhane est détruit à l'acide acétique. On évapore à sec le milieu et on recueille le produit attendu.

### EXEMPLE 9 : 3-butyl 1-[[2'-(méthoxycarbonyl) (1,1'-biphényl) 4-yl] méthyl] 1,4-dihydro 4-oxo 7-quinoléine carboxylate de méthyle

On opère comme à l'exemple 1 à partir de 220 mg du produit obtenu au stade E de la préparation de l'exemple ci-dessus et en utilisant 285 mg de bromométhyl (1-1'-biphényl) 2-carboxylate de méthyle (préparation selon EP 0253310).

Après chromatographie sur silice (éluant : chlorure de méthylène - acétonitrile 87-13), on obtient 340 mg du produit recherché.

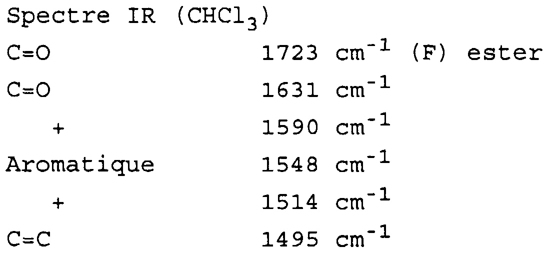

### EXEMPLE 10 : Acide 3-butyl 1-[[2'-carboxy (1,1'-biphényl) 4-yl] méthyl] 1,4-dihydro 4-oxo 7-quinoléine carboxylique

On opère comme à l'exemple 2, à partir de 290 mg du produit obtenu à l'exemple 9, en utilisant 5 cm³ d'une solution 5N de soude. On obtient 210 mg du produit recherché. F > 260°C.

| Analyse pour C₂₈H₂₅NO₅ = 455,515 | | | |
|---|---|---|---|
| calculés : | C% 73,83 | H% 5,53 | N% 3,07 |
| trouvés : | 73,7 | 5,5 | 3,0 |

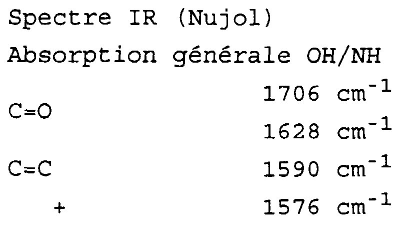

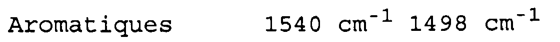

### Préparation de l'exemple 11 : 3-butyl 7-hydroxy méthyl 4-(1H)-quinoléinone

A une solution de 240 mg du produit obtenu au stade E de la préparation de l'exemple 9, dans 50 cm³ de tétrahydrofuranne, on ajoute 35 mg d'hydrure d'aluminium-lithium, on agite 30 minutes à température ambiante. On ajoute du tétrahydrofuranne à 10 % d'eau puis 50 cm³ d'eau. On extrait avec de l'acétate d'éthyle, lave à l'eau, sèche et évapore à sec. Après chromatographie sur silice (éluant : chlorure de méthylène-méthanol (9-1)), on obtient 170 mg du produit recherché. F = 200°C.

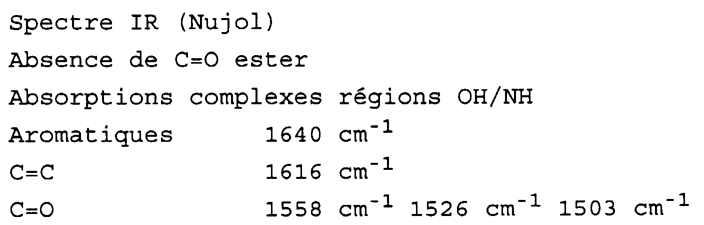

### EXEMPLE 11 : 4'-[[3-butyl 1,4-dihydro 7-(hydroxyméthyl) 4-oxo 1-quinoléinyl] méthyl] (1,1'-biphényl) 2-carboxylate de méthyle

On opère comme à l'exemple 1 à partir de 160 mg du produit obtenu selon la préparation décrite ci-dessus, en utilisant 260 mg de bromométhyl (1,1'-biphényl) 2-carboxylate de méthyle (préparation selon EP 0253310). Après chromatographie sur silice (éluant : chlorure de méthylène-méthanol (95-5)). On obtient 280 mg du produit recherché. F = 140°C.

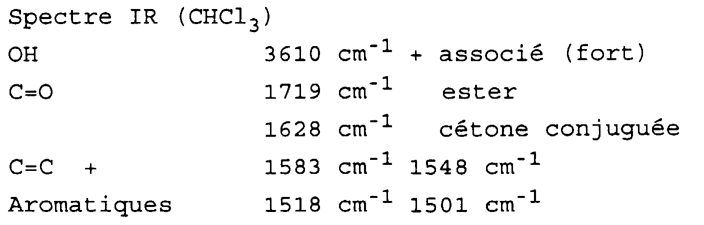

### EXEMPLE 12 : Acide 4'-[[3-butyl 1,4-dihydro 7-(hydroxyméthyl) 4-oxo 1-quinoléinyl] méthyl] (1,1'-biphényl) 2-carboxylique

On opère comme à l'exemple 2, à partir de 280 mg du produit obtenu à l'exemple 11 en utilisant 5 cm³ d'une solution normale de soude. Après recristallisation dans l'éthanol à 50 % d'eau, on obtient 200 mg du produit recherché. F = 212°C.

| Analyse pour C₂₈H₂₇NO₄ = 441,5 | | | |
|---|---|---|---|
| calculés : | C% 76,17 | H% 6,16 | N% 3,17 |
| trouvés : | 76,4 | 6,0 | 3,1 |

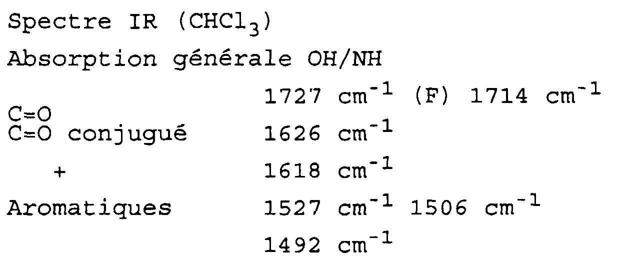

### Préparation de l'exemple 13 : 3-butyl 4-hydroxy cinnoline

### Stade A : 2-(1-hexynyl) benzène amine

A une solution de 4,4 g de 2-iodoaniline dans 100 cm³ de triéthylamine, on ajoute 32 mg d'iodure de cuivre et 140 mg de chlorure de bis(triphénylphosphine) palladium puis 2,3 cm³ de 1-hexyne. On agite 15 heures à température ambiante. On évapore à sec, reprend le résidu avec de l'éther, filtre l'insoluble, le lave à l'éther et évapore à sec le filtrat. Le résidu est chromatographié sur silice (éluant : hexane-acétate d'éthyle (9-1)). On obtient 3,27 g de produit recherché.

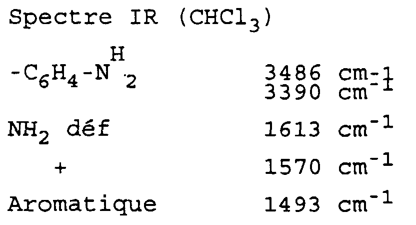

### Stade B : 3-butyl 4-hydroxy cinnoline

A une suspension préparée à 0°C, de 3,2 g du produit obtenu au stade A dans 100 cm³ d'acide chlorhydrique concentré, on ajoute à 0°C, une solution de 2 g de nitrite de sodium dans 60 cm³ d'eau. On agite pendant 1 heure 30 minutes à 0°C puis 1 heure à 100°C. On verse le milieu réactionnel refroidi, dans 100 cm³ d'eau glacée. On essore et lave à l'eau, le produit brut humide est repris par 100 cm³ d'eau alcalinisé avec de l'ammoniaque concentré, on essore et lave à l'eau, on obtient après séchage à 70°C, 1,47 g du produit recherché. F = 180°C.

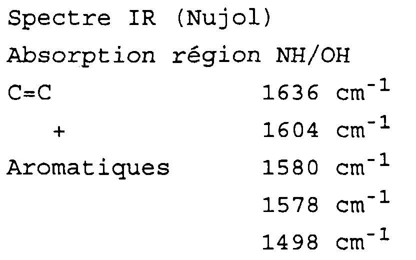

### EXEMPLE 13 : 4-[[3-butyl 1,4-dihydro 4-oxo 1-cinnoléinyl] méthyl] benzonitrile

On opère comme à l'exemple 1, à partir de 406 mg du produit obtenu au stade B de la préparation de l'exemple 13, en utilisant 430 mg de 4-bromométhyl benzonitrile et 830 mg de carbonate de potassium. Après chromatographie sur silice (éluant : chlorure de méthylène-méthanol (98-2)), on obtient 460 mg du produit recherché. F = 180°C.

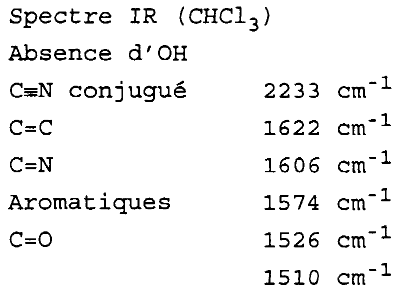

### EXEMPLE 14 : Acide 4-[[3-butyl 1,4-dihydro 4-oxo 1-cinnolinyl] méthyl] benzoïque

On opère comme à l'exemple 2 à partir de 400 mg du produit obtenu à l'exemple 13 en utilisant 4 cm³ d'une solution de soude 5N. On obtient 400 mg de produit attendu. F = 220°C. Après recristallisation dans l'acétonitrile (50 cm³) on recueille 270 mg de produit purifié. F = 220°C.

| Analyse pour C₂₀H₂₀N₂O₃ = 336,393 | | | |
|---|---|---|---|
| calculés : | C% 71,41 | H% 5,99 | N% 8,33 |
| trouvés : | 71,3 | 6,0 | 8,3 |

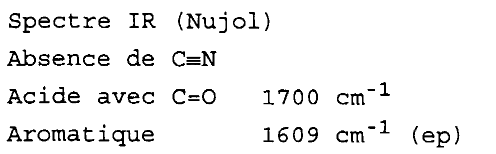

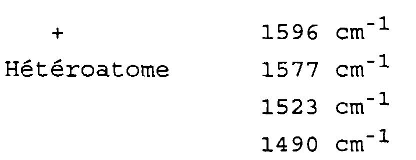

### EXEMPLE 15 : 4'-[[3-butyl 1,4-dihydro 4-oxo 7-[(1-pyrrolidinyl) carbonyl] 1-quinoléinyl] méthyl] (1,1'-biphényl) 2-carboxylate de méthyle

### Stade A : 3-butyl 1,4-dihydro 4-oxo 7-[(1-pyrrolidinyl) carbonyl quinoléine

On prépare une suspension de 1,52 g du produit obtenu au stade D de la préparation de l'exemple 9 dans 100 cm³ de tétrahydrofuranne à laquelle on ajoute 0,65 cm³ de pyrrolidine, le milieu réactionnel est refroidi à 0°C et on y ajoute successivement 1 cm³ de triéthylamine 100 cm³ de chlorure de méthylène, 1 g d'hydroxybenzotriazole et 1,82 g de 1-(3-diméthylaminopropyl) 3-éthyl carbodiimide hydrochloride.

On agite pendant 3 heures à la température ambiante. On ajoute ensuite de l'eau glacée, acidifie à pH = 5-6, avec de l'acide chlorhydrique 0,1 N. On lave la phase organique à l'eau, la sèche et évapore à sec. On chromatographie le résidu sur silice (éluant : chlorure de méthylène-méthanol (9-1)). On obtient 1,8 g du produit attendu.

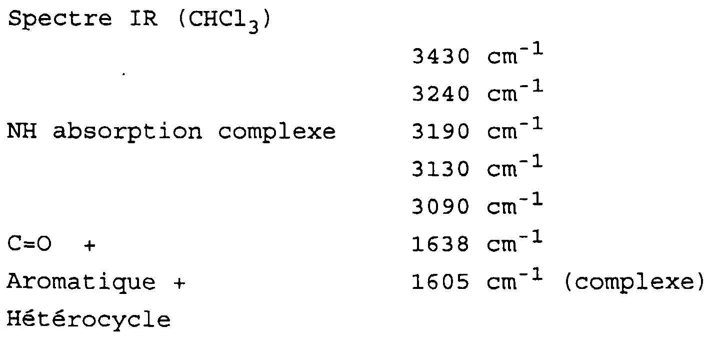

### Stade B : 4'-[[3-butyl 1,4-dihydro 4-oxo 7-[(1-pyrrolidinyl) carbonyl] 1-quinoléinyl] méthyl] (1,1'-biphényl) 2-carboxylate de méthyle

On opère comme à l'exemple 3 à partir de 500 mg du produit obtenu au stade A ci-dessus. On obtient 0,675 g du produit attendu. F = 191°C après empâtage éther isopropylique.

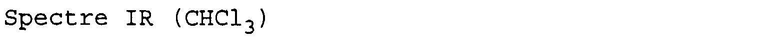

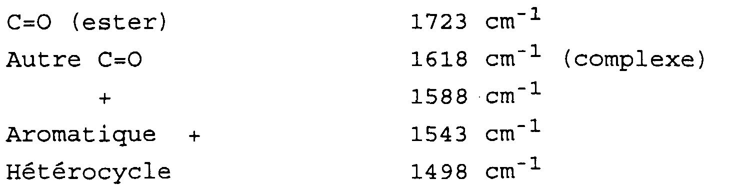

### EXEMPLE 16 : Acide 4'-[[3-butyl 1,4-dihydro 4-oxo 7-[(1-pyrrolidinyl) carbonyl] 1-quinoléinyl] méthyl] (1,1'-biphényl) 2-carboxylique

On opère comme à l'exemple 2 à partir de 1,1 g du produit obtenu à l'exemple 15. On obtient, après recristallisation dans l'éthanol, 0,67 g du produit attendu. PF > 260°C.

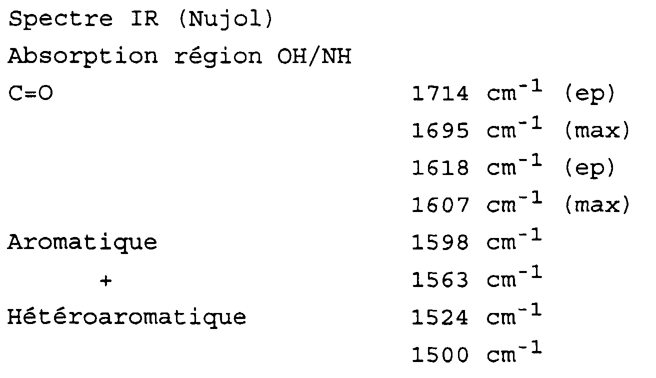

| Microanalyse pour C₃₂H₃₂N₂O₄ = 508,62 | | | |
|---|---|---|---|
| calculés : | C% 75,57 | H% 6,34 | N% 5,51 |
| trouvés : | 75,4 | 6,2 | 5,3 |

### Préparation du produit 17 : 4-[[3-butyl 8-bromo 4-quinoléinyl] oxy] benzyle

### Stade A : 2-butyl 3-[[2-bromophényl] amino] 2-butènedioate de diéthyle

On agite pendant 4 heures au reflux une solution de 5 g de bromoaniline avec 7,9 g de 2-butyl 3-oxo butanedioate de diéthyle dans 100 cm³ de toluène et 100 mg d'acide paratoluène sulfonique. On évapore à sec et chromatographie le résidu sur silice (éluant : chlorure de méthylène-hexane (6-4)). On obtient 7,2 g du produit recherché.

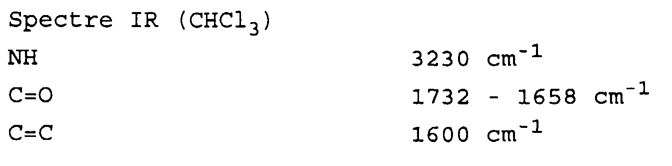

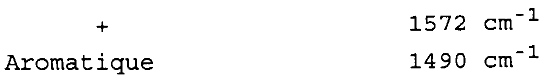

### Stade B : 3-butyl 1,4-dihydro 8-bromo 4-oxo 2-quinoléine carboxylate d'éthyle

On opère comme au stade B de la préparation de l'exemple 7 à partir de 7 g du produit obtenu au stade A. Après chromatographie sur silice (éluant : chlorure de méthylène), on obtient 6,3 g du produit attendu (F = 88°C).

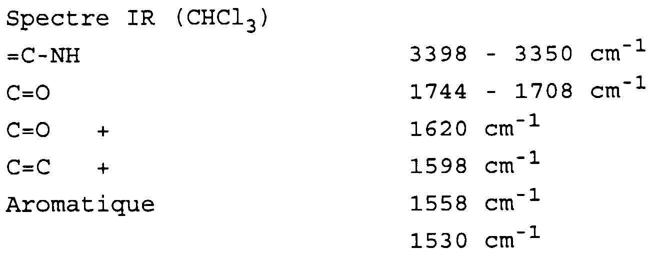

### Stade C : Acide 3-butyl 1,4-dihydro 8-bromo 4-oxo 2-quinoléine carboxylique

On opère comme au stade C de la préparation de l'exemple 7 à partir de 6,3 g du produit obtenu au stade B ci-dessus. On obtient 6 g du produit recherché (F = 220°C).

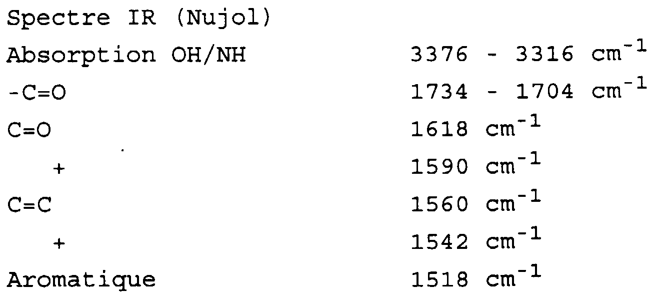

### Stade D : 3-butyl 8-bromo 4-(1H) quinoléinone

On opère comme au stade D de la préparation de l'exemple 7 à partir de 6 g du produit obtenu au stade C ci-dessus. On obtient 3,4 g du produit recherché (F = 172°C).

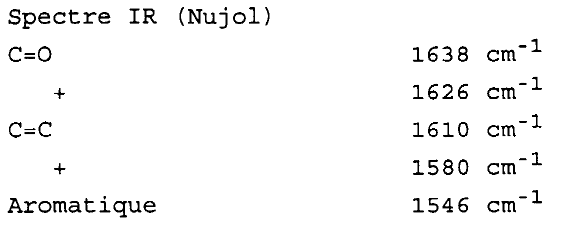

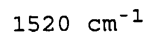

### Stade E : 4-[(3-butyl 8-bromo 4-quinolinyl) oxy] benzyle.

On agite 5 heures au reflux, 2 g du produit obtenu au stade D ci-dessus, dans 100 cm³ d'acétone avec 1 cm³ de bromure de benzyle et 2 g de carbonate de potassium. Après chromatographie sur silice (éluant : chlorure de méthylène) on obtient 2,12 g du produit recherché.

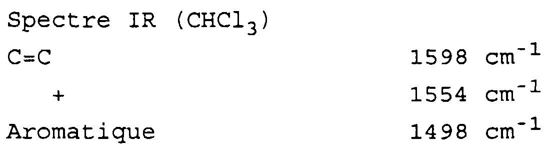

### Préparation de l'exemple 19 : 4-[[(2-butyl 4-quinoléinyl) oxy] méthyl] benzyle

### Stade A : 3-oxo heptanoate d'éthyle

A une suspension de 27,3 g d'hydrure de sodium (à 50 % dans l'huile, lavé 3 fois avec de l'hexane) dans 250 cm³ d'éther, on ajoute 70,8 g de carbonate d'éthyle en solution dans 50 cm³ d'éther. On agite 10 minutes et on ajoute 30 g d'hexanone. On chauffe 2 heures au reflux. On ajoute une solution de 35 cm³ d'éther contenant 12 cm³ d'éthanol. On refroidit la solution à 0°C et ajoute une solution de 36 cm³ d'acide acétique dans 300 cm³ d'eau, on ajoute 12 cm³ d'une solution saturée de bicarbonate de sodium, on extrait avec de l'éther, lave à l'eau, sèche, filtre et évapore à sec sous pression réduite. On obtient 100 g d'huile que l'on distille sous une pression de 3 mbar à 70°C. On obtient 32,5 g de produit recherché.

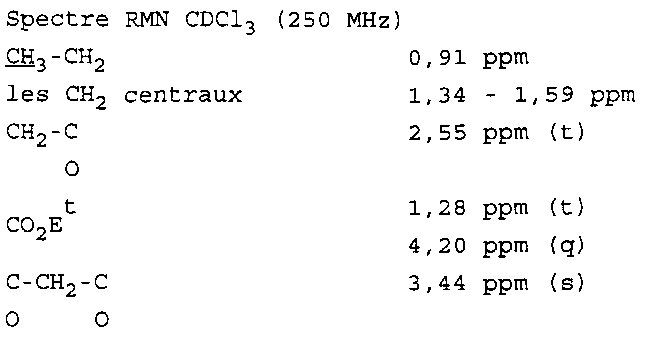

### Stade B : 3-(phénylamino) 2-heptènoate d'éthyle

On agite pendant 48 heures un mélange de 17,9 g d'aniline avec 45 g de 3-oxo heptanoate d'éthyle obtenu comme au stade A, en présence de 5 g de siliporite activée. Le mélange réactionnel est chromatographié sur silice (éluant : hexaneacétate d'éthyle (95-5)) . On obtient 28,7 g du produit recherché.

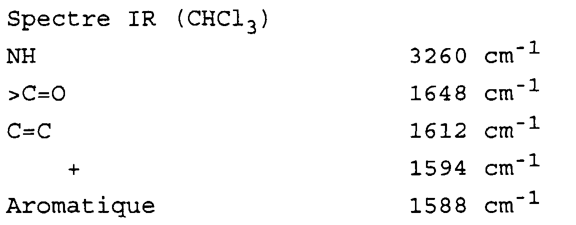

### Stade C : 2-butyl 4(1H)-quinoléinone

On chauffe pendant 45 minutes à 250°C, une solution de 28,7 g du composé obtenu au stade B ci-dessus, dans 60 cm³ de diphényl éther. On laisse refroidir, empâte dans 400 cm³ de pentane, essore et lave avec du pentane. On obtient 16,95 g de produit recherché. F = 140°C.

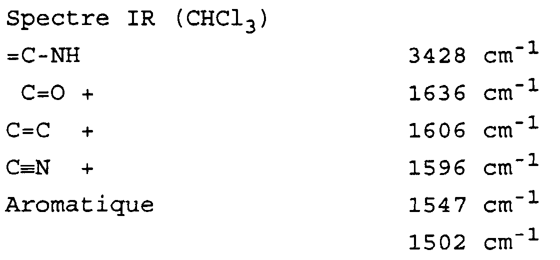

### Stade D : 4-[[(2-butyl 4-quinoléinyl) oxy] méthyl] benzyle

On agite 5 heures au reflux 3 g du produit obtenu au stade C, 2,8 cm³ de bromure de benzyle et 4,1 g de carbonate de potassium dans 70 cm³ d'acétone. On coule dans 100 cm³ d'eau, extrait à l'acétate d'éthyle. Après chromatographie sur silice (éluant : chlorure de méthylène - méthanol (95-5)) on obtient 3,2 g du produit recherché.

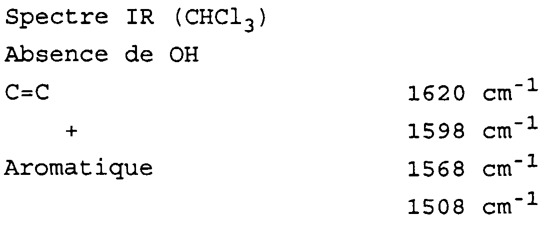

### EXEMPLE 19 : 4'-[(2-butyl 1,4-dihydro 4-oxo 1-quinoléinyl) methyl] (1,1'-biphényl) 2-carboxylate de méthyle

On chauffe 15 heures à 130°C, un mélange de 1 g de 4-[(2-butyl 4-quinoléinyl) oxy] benzyle obtenu au stade D de la préparation de l'exemple 19 et 1,4 g de bromométhyl (1,1'-biphényl) 2-carboxylate de méthyle. Après chromatographie sur silice, on obtient 1,3 g du composé recherché.

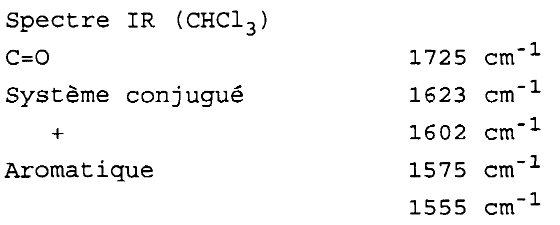

### EXEMPLE 20 : Acide 4'-[(2-butyl 1,4-dihydro 4-oxo 1-quinoléinyl) méthyl] (1,1'-biphényl) 2-carboxylique

On opère comme à l'exemple 2, à partir du produit obtenu à l'exemple 19, en utilisant 3 cm³ de soude N et 2 cm³ d'éthanol. Après recristallisation dans le diméthylformamide, on obtient 155 mg du produit recherché (F > 260°C).

| Analyse pour C₂₇H₂₅NO₃ = 411,48 | | | |
|---|---|---|---|
| calculés : | C% 78,80 | H% 6,12 | N% 3,4 |
| trouvés : | 78,6 | 6,0 | 3,3 |

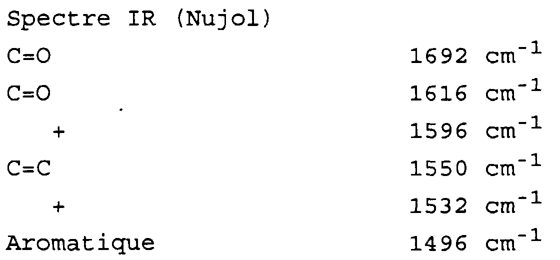

### Préparation de l'exemple 21 : 3-butyl 1,4,5,6,7,8-hexahydro 4-oxo 1-quinoléine.

On introduit 1 g du produit obtenu au stade E de la préparation de l'exemple 1 dans 60 cm³ de méthanol. On ajoute environ 10 mg d'oxyde de platine et met à hydrogéner sous pression d'environ 200 mbar. Après environ 3 heures d'agitation à la température ambiante, on ajoute 10 mg d'oxyde de platine et agite 16 heures sous pression de 200 mbar d'hydrogène. On filtre la solution et évapore. Après chromatographie (éluant : chlorure de méthylène-méthanol 98-2), on obtient 0,6 g de produit attendu. F = 223°C.

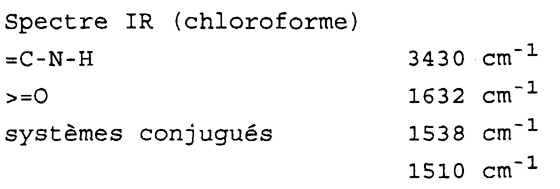

### Exemple 21 A : 4'-[(3-butyl 1,4,5,6,7,8-hexahydro 4-oxo 1-quinoléinyl) méthyl] (1,1'-biphényl 2-carboxylate de méthyle ainsi que B : 4'-[((3-butyl 1,4,5,6,7,8-hexahydro 4-quinoléinyl) oxy] méthyl] (1,1'-biphényl) 2-carboxylate de méthyle.

On introduit 0,4 g du produit obtenu à la préparation de l'exemple 21 dans 10 cm³ d'acétone anhydre. On ajoute 0,52 g de carbonate de potassium et 0,6 g de bromométhyl (1,1'-biphényl) 2-carboxylate de méthyle. On chauffe une nuit à reflux puis verse le milieu réactionnel dans 100 cm³ d'eau et extrait la phase aqueuse avec 3 fois 50 cm³ d'acétate d'éthyle. On sèche et évapore à sec. Après chromatographie (éluant : chlorure de méthylène-méthanol 98-2), on obtient 0,45 g du produit attendu A ainsi que 0,15 g du produit B.

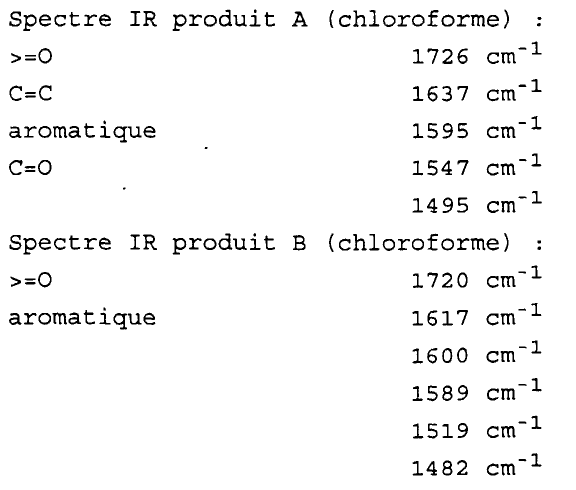

### Exemple 22 : Acide 4'-[(3-butyl 1,4,5,6,7,8-hexahydro 4-oxo 1-quinoléinyl) méthyl] (1,1'-biphényl) 2-carboxylique.

On introduit 0,43 g du produit A obtenu à l'exemple 21 dans 10 cm³ d'une solution normale de soude. On chauffe 1 heure à 60°C, agite environ 1 heure et ajoute 1 cm³ d'éthanol. On agite une nuit à environ 40°C, refroidit à température ambiante et ajoute lentement de l'acide acétique glacial jusqu'à cristallisation de l'acide. On agite environ 1 heure, essore, lave à l'eau puis à l'éther. On recristallise dans 80 cm³ d'éthanol et obtient 0,310 g de produit attendu. F = 260°C.

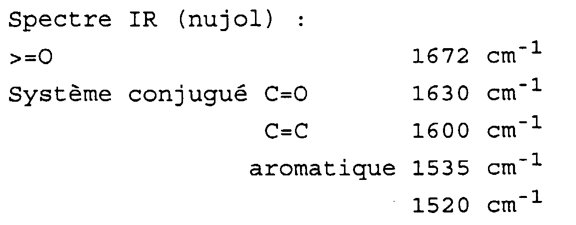

A partir du produit B également obtenu avec l'exemple 21 et en procédant dans les mêmes conditions que pour obtenir l'exemple 22 à partir du produit A de l'exemple 21, on obtient l'acide 4'-[((3-butyl 1,4,5,6,7,8-hexahydro 4-oxo 1-quinoléinyl) oxy) méthyl] (1,1'-biphényl) 2-carboxylique.

### Préparation de l'exemple 23

### Stade A : 3-butyl 4-hydroxy 2,6-quinoléine dicarboxylate de 2-éthyl 6-méthyle.

### a) 2-butyl 3-[(4-(méthoxycarbonyl) phényl) amino] 2-butènedioate de diéthyle.

On introduit 27,8 g de 4-aminobenzoate de méthyle et 47 g de 2-butyl 3-oxo butanedioate de diéthyle et ajoute 2 g de siliporite activée. On agite environ 30 heures à 60°C. Après chromatographie (éluant : hexane-acétate d'éthyle 95-5), on obtient 70 g de produit attendu.

### b) 3-butyl 4-hydroxy 2,6-quinoléine dicarboxylate de 2-éthyl 6-méthyle.

Les 70 g de produit obtenu en a) ci-dessus sont mélangés à 70 cm³ de DOWTHERM. On chauffe à environ 250°C environ 30 minutes, refroidit, empâte dans l'éther et essore. On obtient 45 g de produit attendu. F = 160°C.

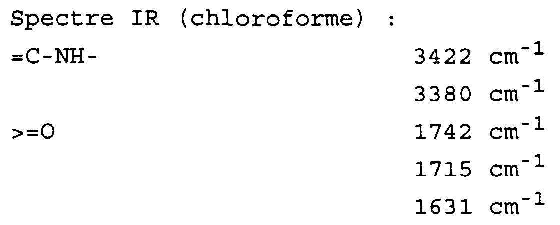

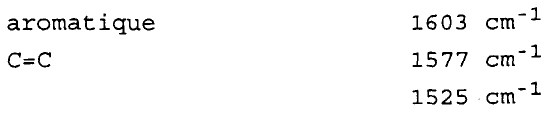

### Stade B : Acide 3-butyl 4-hydroxy 6-quinoléine carboxylique.

### a) Acide 3-butyl 1,4-dihydro 4-hydroxy 2,6-quinoléine dicarboxylique.

On introduit 40 g du produit obtenu au stade A ci-dessus dans 150 cm³ de soude concentrée et ajoute 15 cm³ d'éthanol. On chauffe environ 4 heures à environ 80°C, ajoute 100 cm³ d'un mélange de glace et eau, acidifie avec de l'acide chlorhydrique concentré, essore, lave à l'eau et sèche. On obtient 24 g de produit attendu. F > 260°C.

### b) Acide 3-butyl 4-hydroxy 6-quinoléine carboxylique.

On introduit 24 g du produit obtenu en a) ci-dessus dans 350 cm³ de DOWTHERM, chauffe à environ 250°C pendant 5 heures, refroidit à température ambiante, empâte dans l'éther, essore, lave à l'eau et sèche. On obtient 17,8 g de produit attendu. F > 260°C.

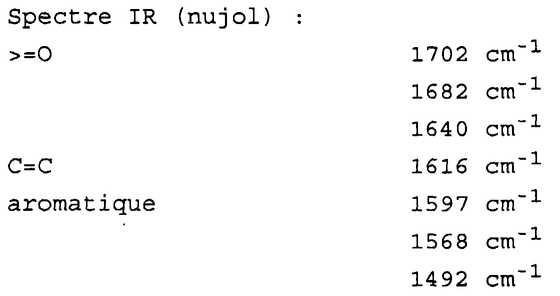

### Stade C : 3-butyl 6-hydroxyméthyl 4-(1H)-quinoléine.

On introduit 3 g du produit obtenu au stade B ci-dessus dans 800 cm³ de tétrahydrofuranne. On ajoute 1,8 g de tétrahydrure de lithium et d'aluminium et agite environ 5 heures à température ambiante. On ajoute environ 5 cm³ d'une solution tétrahydrofuranne-eau 80-20, ajoute lentement une solution saturée de sel de tartrate double, filtre, lave le précipité avec du tétrahydrofuranne et sèche. On obtient 2,5 g du produit attendu. F > 260°C.

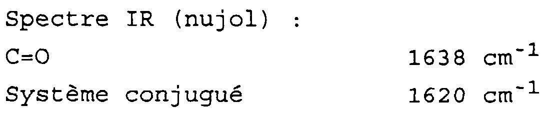

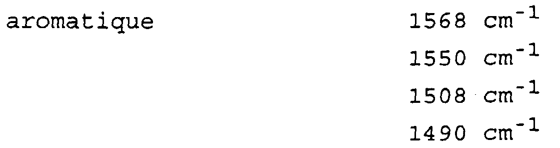

### Stade D : 3-butyl 1,4,5,6,7,8-hexahydro 6-méthyl 4-(1H)-quinoléine (1) (ainsi que 3-butyl 1,4,5,6,7,8-hexahydro 6-hydroxyméthyl 4-(1H)-quinoléine (2)).

On maintient 0,5 g du produit obtenu au stade C ci-dessus dans 100 cm³ de méthanol, ajoute 10 mg d'oxyde de platine et met à hydrogéner sous environ 300 mbar environ 24 heures. On filtre, lave au méthanol et sèche. Après chromatographie (éluant : chlorure de méthylène-méthanol 95-5), on obtient 0,47 g de produit attendu (1). F ≈ 260°.

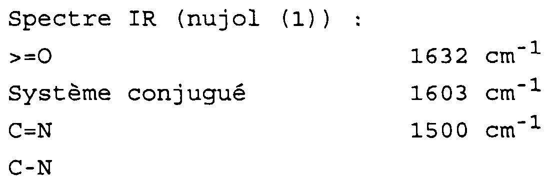

### Exemple 23 (A) : 4'-[(3-butyl 1,4,5,6,7,8-hexahydro 6-méthyl 4-oxo 1-quinoléinyl) méthyl] (1,1'-biphényl) 2-carboxylate de méthyle ainsi que (B) 4'-[((butyl 1,4,5,6,7,8-hexahydro 6-méthyl 4-quinoléinyl) oxy) méthyl] (1,1'-biphényl) 2-carboxylate de méthyle.

On opère comme à l'exemple 21 à partir de 0,45 g du produit (A) obtenu au stade D ci-dessus de la préparation de l'exemple 23 dans 20 cm³ d'acétone anhydre, ajoute 0,55 g de carbonate de potassium et 0,61 g de bromométhyl (1,1'-biphényl) 2-carboxylate de méthyle. On chauffe environ 3 heures au reflux, verse le milieu réactionnel dans 100 cm³ d'eau, extrait la phase aqueuse par 3 fois 50 cm³ d'acétate d'éthyle, sèche la phase organique et évapore. Après chromatographie (éluant : chlorure de méthylène-méthanol 95-5), on obtient 0,58 g de produit attendu (A). On obtient également 0,1 g de produit (B).

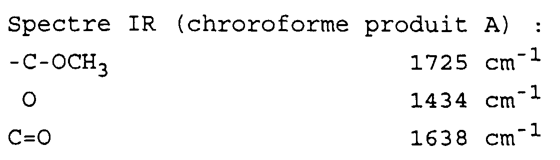

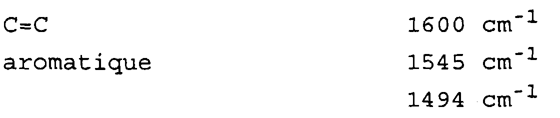

### Exemple 24 : Acide 4'-[(3-butyl 1,4,5,6,7,8-hexahydro 6-méthyl 4-oxo 1-quinoléinyl) méthyl] (1,1'-biphényl) 2-carboxylique.

On opère comme à l'exemple 22 à partir de 0,4 g du produit de l'exemple 23 (soit A) dans 10 cm³ de soude 2N. On agite une nuit à environ 60°C, refroidit à température ambiante, acidifie avec de l'acide acétique glacial, décante, empâte dans une solution d'acide chlorhydrique 2N, essore, sèche, recristallise dans 50 cm³ d'un mélange éthanol-eau (49-1) et obtient 0,220 g de produit attendu. F = 255°C.

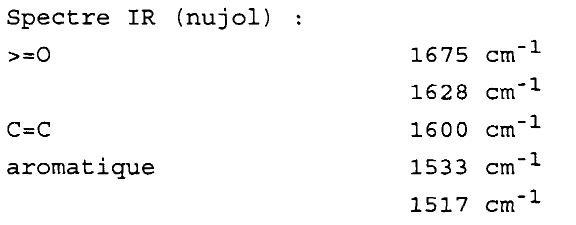

- A partir du produit (B) également obtenu avec l'exemple 23, et en procédant dans les mêmes conditions que pour obtenir l'exemple 24 à partir du produit (A) de l'exemple 23, on peut obtenir l'acide 4'-[[(3-butyl 1,4,5,6,7,8-hexahydro 6-méthyl 4-quinoléinyl) oxy] méthyl] (1,1'-biphényl) 2-carboxylique.
- A partir du produit (2) également obtenu au stade D de la préparation de l'exemple 23 et en procédant de la même manière que pour obtenir l'exemple 23 à partir du produit (1) obtenu à ce stade D sus-dit, on peut obtenir les produits (A1) 4'-[(3-butyl 1,4,5,6,7,8-hexahydro 6-hydroxyméthyl 4-oxo 1-quinoléinyl) méthyl] (1,1'-biphényl) 2-carboxylate de méthyle ainsi que (B1) 4'-[[(3-butyl 1,4,5,6,7,8-hexahydro 6-hydroxyméthyl 4-quinoléinyl) oxy] méthyl] (1,1'-biphényl) 2-carboxylate de méthyle.
- A partir des produits (A1) et (B1) définis ci-dessus et en procédant de la même manière que pour obtenir l'exemple 24, on peut obtenir respectivement à partir de (A1) l'acide 4'-[(3-butyl 1,4,5,6,7,8-hexahydro 6-hydroxyméthyl 4-oxo 1-quinoléinyl) méthyl] (1,1'-biphényl) 2-carboxylique et à partir de (B1) l'acide 4'-[[(3-butyl 1,4,5,6,7,8-hexahydro 6-hydroxyméthyl 4-quinoléinyl) oxy] méthyl] (1,1'-biphényl) 2-carboxylique.

Les produits des exemples 25 à 52 qui suivent illustrent également l'invention sans toutefois la limiter et répondent à la formule (I_{B}) telle que définie ci-dessus dans laquelle X représente un atome d'oxygène, R₁ représente un radical n-butyle, Y représente le radical 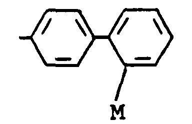 et R₂, R₃, R₄ ainsi que M ont les significations indiquées dans le tableau ci-après.

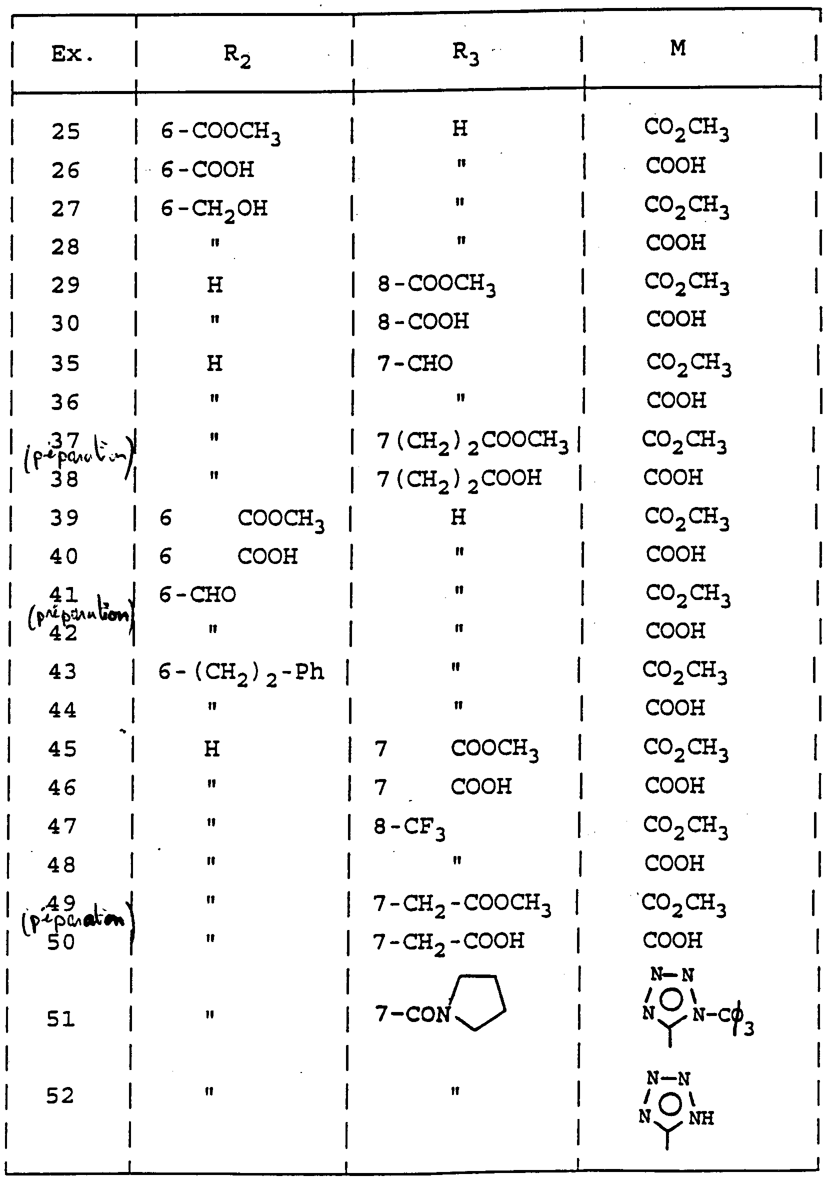

Les produits des exemples 25 à 52 ont été préparés ainsi qu'il est indiqué pour les produits des exemples décrits ci-dessus.

Les produits des exemples 26, 28, 30, 32, 34, 36, 38, 40, 42, 44, 46, 48, 50 et 52 sont caractérisés dans le tableau ci-après par leurs points de fusion et leurs résultats de microanalyse chimique.

| | | Microanalyse | | | | | |
|---|---|---|---|---|---|---|---|
| Ex. | F° | Calculé | | | Trouvé | | |
| | | C% | H% | N% | C% | H% | N% |
| 26 | > 260°C | 73,83 | 5,53 | 3,07 | 73,1 | 5,4 | 3,0 |
| 28 | 258°C | 76,17 | 6,16 | 3,14 | 76,1 | 6,2 | 3,1 |
| 30 | 219°C | 73,83 | 5,53 | 3,07 | 73,7 | 5,2 | 2,9 |
| 34 | 150°C | 75,57 | 6,3 | 5,5 | 75,7 | 6,2 | 5,4 |
| 36 | 225°C | 76,51 | 5,73 | 3,18 | 76,7 | 5,7 | 3,0 |
| 38 | 210°C | 74,52 | 6,04 | 2,90 | 74,4 | 5,9 | 2,9 |
| 40 | > 260°C | 74,83 | 5,65 | 2,9 | 74,6 | 5,6 | 2,7 |
| 42 | > 260°C | 76,5 | 5,73 | 3,18 | 76,1 | 5,8 | 3 |
| 44 | 250°C | 81,5 | 6,45 | 2,72 | 81,2 | 6,4 | 2,6 |
| 46 | > 260°C | 74,83 | 5,65 | 2,91 | 74,6 | 5,9 | 3,1 |
| 48 | 196°C | 70,14 | 5,04 | | 70,2 | 5,1 | |
| 50 | 245°C | 74,18 | 5,80 | 2,98 | 74,0 | 5,9 | 2,9 |
| 52 | dec.190- | 72,16 | 6,05 | 15,78 | 71,9 | 6,1 | 15,1 |
| | 195°C | | | | | | |

Les produits décrits dans le tableau ci-dessous qui constituent les exemples 53 à 128 répondent à la formule : 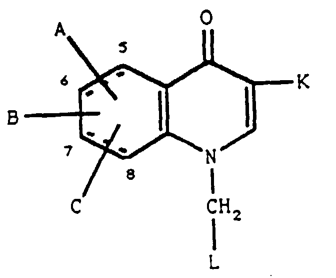 dans laquelle A, B et C ont les significations et l'emplacement sur le cycle (soit les positions 5, 6, 7 ou 8) indiqués dans le tableau ci-dessous, L est représenté par les nombres 3, 4, 5 ou 6 qui ont les significations suivantes :
- les nombres 1 à 6 représentent un radical biphényle de formule : 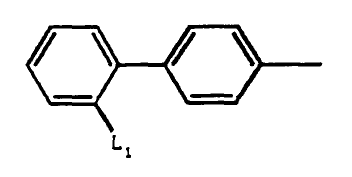 tels que
   . le nombre 3 correspond à L1 représentant -CO₂CH₃
   . le nombre 4 correspond à L1 représentant -CO₂H
   . le nombre 5 correspond à L1 représentant un radical tétrazolyle salifié par le radical triphényl méthyle
   . le nombre 6 correspond à L1 représentant un radical tétrazolyle 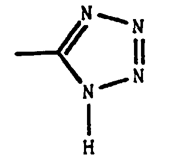
K a la signification indiquée dans le tableau ci-dessous et 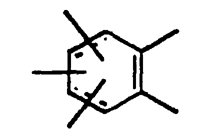 représente
**soit** 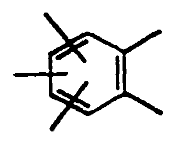 indiqué par la lettre Q dans la colonne intitulée "N",
**soit** 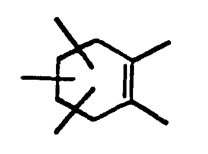 indiqué par les lettre TQ dans la colonne intitulée "N".

Ces produits ont été obtenus selon les mêmes procédés que ceux indiqués ci-dessus. F°C désigne le point de fusion des produits.

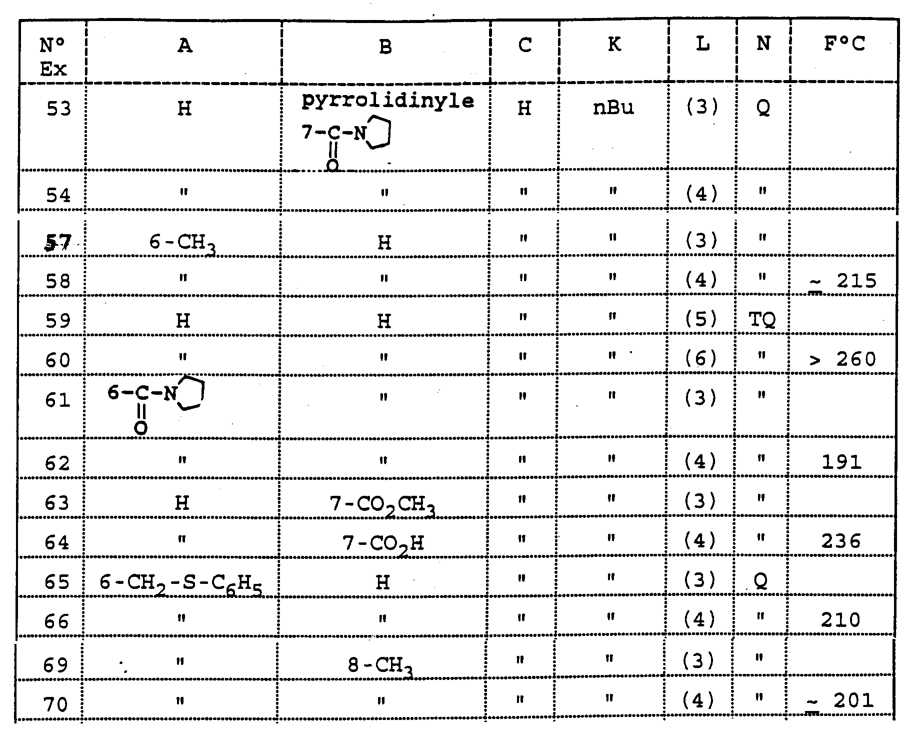

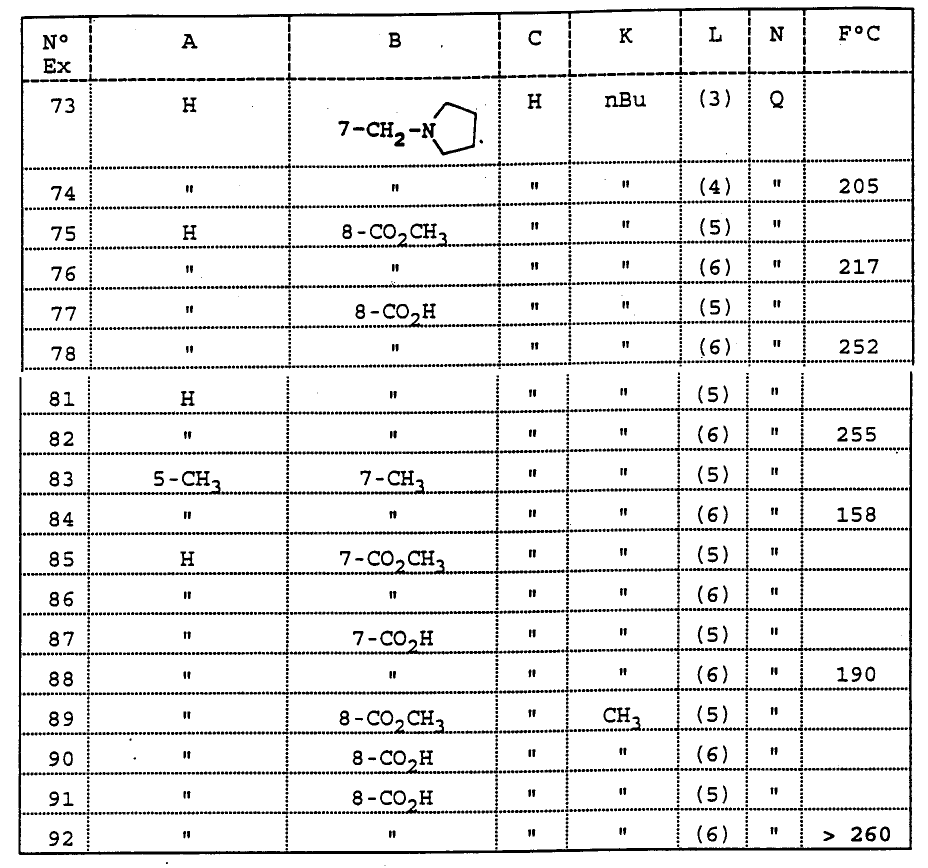

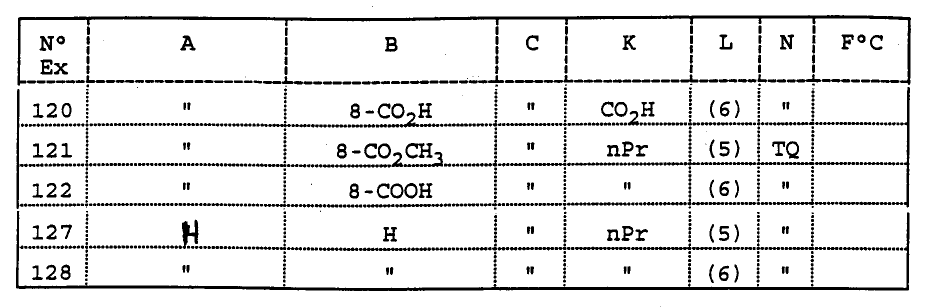

Parmi les produits de formule (I) telle que définie ci-dessus obtenus dans le cadre de la présente invention, peuvent être cités tout particulièrement les produits décrits ci-dessous qui peuvent être obtenus comme exemples 129 à 146 :

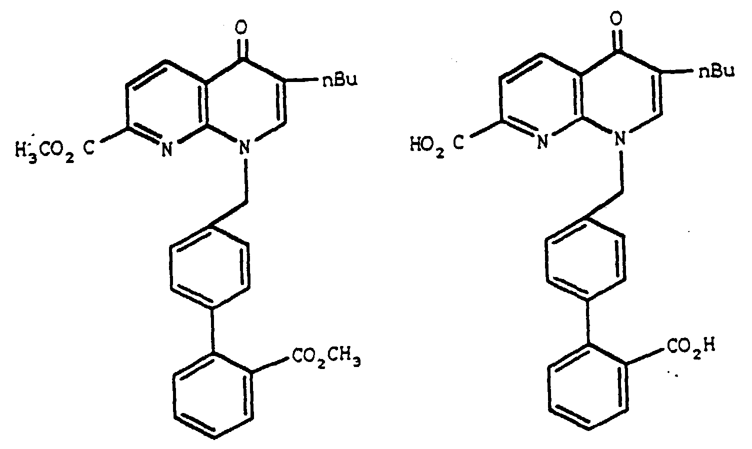

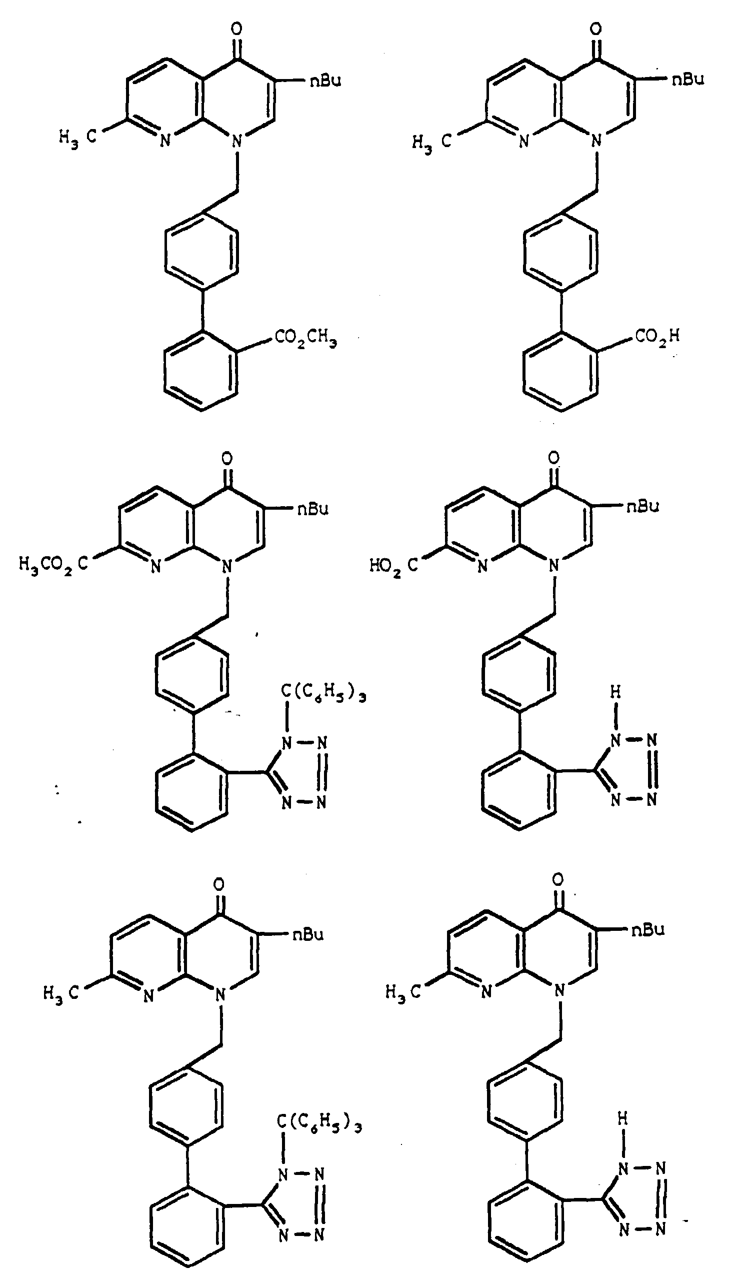

Les produits analogues des produits des exemples 131 à 138 ci-dessus, dans lesquels l'atome d'azote en position 8 se situe en position 7 de la quinolone tandis que le substituant en position 7 se situe en position 8, peuvent être obtenus suivant le procédé décrit ci-dessus et constituent les exemples 139 à 146.

### EXEMPLE 147 : de composition pharmaceutique

On a préparé des comprimés répondant à la formule suivante :

| | |
|---|---|
| produit de l'exemple 10 | 10 mg |
| Excipient pour un comprimé terminé à | 100 mg |
| (détail de l'excipient : lactose, talc, amidon, stéarate de magnésium) | |

### RESULTATS PHARMACOLOGIQUES

### 1) Test sur le récepteur de l'angiotensine II

On utilise une préparation membranaire fraîche obtenue à partir de foie de rat. Le tissu est broyé au polytron dans un tampon Tris 50 mM pH 7,4 le broyage est suivi de 3 centrifugations à 30 000 g 15 minutes avec reprises intermédiaires des culots dans le tampon Tris pH 7,4.

Les derniers culots sont remis en suspension dans un tampon d'incubation pH = 7,4 (Tris 20 mM, NaCl 135 mM, KCl 10 mM, glucose 5 mM, MgCl₂ 10 mM de fluorure de phényl méthyl sulfonyle 0,3 mM, bacitracine 0,1 mM (sérum albumine bovine 0,2 %).

On répartit des fractions aliquotes de 2 ml dans des tubes à hémolyse et ajoute de la ¹²⁵I angiotensine II (25 000 DPM/tube) et le produit à étudier. (Le produit est d'abord testé à 3 x 10⁻⁵M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition du produit de l'exemple 94 du brevet européen 0253310 à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

| Résultats : | |
|---|---|
| Produit de l'exemple | CI₅₀ en nanomoles |
| 10 | 92 |
| 4 | 180 |
| 6 | 400 |
| 16 | 79 |
| 22 | 90 |
| 30 | 54 |
| 52 | 8,7 |

### 2) Mise en évidence de l'activité antagoniste de l'angiotensine II sur la veine porte isolée

La veine porte est prélevée, sur des rats mâles Wistar (350 g environ) (IFFA Crédo France) après dislocation cervicale, et mise rapidement dans une solution physiologique (voir ci-dessous) à température ambiante. Un anneau de 1 mm environ est monté dans un bain à organe isolé contenant 20 ml de la solution physiologique suivante (composition en mM : NaCl 118,3 - KCl 4,7 - MgS0₄ 1,2 - KH₂PO₄ 1,2 - NaHCO₃ 25 - glucose 11,1 - CaCl2 2,5) le milieu est maintenu à 37°C et oxygéné par un mélange O₂ 95 %, CO₂ 5 %. La tension initiale imposée est de 1 g, les anneaux sont laissés au repos pendant 60 à 90 minutes. Afin d'éviter les contractions spontanées, du vérapamil est ajouté au bain d'incubation (1.10⁻⁶M).

A la fin de la période de repos l'angiotensine II (hypertensine Ciba) 3.10⁻⁸M est ajoutée dans le bain d'incubation et laissée au contact de la préparation pendant 1 minute. Cette opération est répétée chaque 30 minutes, le tissu étant lavé 3 ou 4 fois entre deux stimulations à l'angiotensine. Le composé à étudier est introduit dans le bain 15 minutes avant une nouvelle stimulation par l'angiotensine. Des concentrations croissantes de la molécule étant appliquées, une CI₅₀ (concentration qui produit une inhibition de 50 % de la réponse à l'angiotensine) peut être calculée, celle-ci est exprimée en nanomoles.

| Résultats : | |
|---|---|
| Produit de l'exemple | CI₅₀ en nanomoles |
| 10 | 31 |
| 4 | 96,5 |
| 6 | 66 |
| 12 | 49 |
| 16 | 31,7 |

### 3) Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal pour l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des composés vis-à-vis de l'angiotensine II (Hypertensine Ciba) est abordée de la façon suivante.
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules à étudier sont injectées 5 minutes avant l'angiotensine II.

Les effets presseurs de l'angiotensine II en présence de l'antagoniste sont exprimés en pourcentage des effets presseurs de l'angiotensine II administrée seule. La dose inhibitrice à 50 % de l'effet étudié est ainsi déterminée.

Chaque animal est considéré comme son propre témoin.

| Résultats : | |
|---|---|
| Produit de l'exemple | Dose inhibitrice à 50 % (mg/kg) |
| 10 | 1,27 |
| 4 | 7,55 |
| 6 | 6,65 |
| 12 | 3,76 |
| 16 | 1,2 |
| 50 | 0,61 |
| 52 | 0,23 |
| 22 | 1 |
| 30 | 0,21 |

### 4) Test d'évitement passif

Des souris mâles (CD₁ Charles River) d'un poids de 25-30 g sont placées dans la partie lumineuse d'un box à deux compartiments communicants par une ouverture (F. Barzaghi et G. Giuliani, Brit. J. Pharmacol. en cours de publication).

A l'intant où la souris passe du compartiment lumineux au compartiment obscur, l'ouverture se ferme et elle est immédiatement punie par une décharge électrique aux pattes. L'animal soumis à cette procédure apprend à mémoriser la punition. En fait, si on le remet dans le compartiment lumineux, il évitera ainsi de franchir l'ouverture et de rentrer dans le compartiment obscur.

Pour induire une amnésie, les animaux sont traités avec de la SCOPOLAMINE (0,7 mg/kg I.P.) 15 minutes avant l'apprentissage, les produits sont administrés par voie orale aux doses de 0,1 ; 0,25 ; 1 ; 2,5 ; 10 ; 25 ; 100 ; 250 ; 1000 µg/kg.

10 à 50 animaux ont été utilisés par dose.

L'effet antiamnésique des produits est évalué 24 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence avec une courbe doseréponse en forme de cloche.

Les résultats sont exprimés en pourcentages d'augmentation du temps de latence, par rapport aux témoins correspondants.

Les résultats sont les suivants :

| EX. | POURCENTAGE D'AUGMENTATION DU TEMPS DE LATENCE PAR RAPPORT AUX TEMOINS | | | | | | | | |
|---|---|---|---|---|---|---|---|---|---|
| | Dose : µg/kg, s. c. | | | | | | | | |
| | 0,1 | 0,25 | 1 | 2,5 | 10 | 25 | 100 | 250 | 1000 |
| 22 | | | | | 8 (20) | 26 (20) | 66 (20) | 128 (20) | 127 (20) |
| 30 | | | 23 (20) | | 37 (20) | 65 (30) | 91 (30) | 144 (20) | 126 (20) |
| 38 | 1 (10) | | 57 (10) | | 118 (30) | 132 (20) | 131 (20) | 101 (10) | 158 (10) |
| 56 | | | | | 24 (20) | 49 (20) | 98 (20) | 105 (20) | 122 (20) |
| 50 | 20 (20) | 31 (20) | 84 (30) | 93 (20) | 190 (20) | 225 (20) | 149 (20) | | |
| 58 | 3 (20) | | 29 (20) | | 112 (30) | 110 (20) | 129 (20) | | |

## Revendications (Revendications pour l'(les) Etat(s) contractant(s) suivant(s): AT, BE, CH, LI, DE, DK, FR, GB, IT, LU, MC, NL, PT, SE)

1. Les produits de formule (I) : 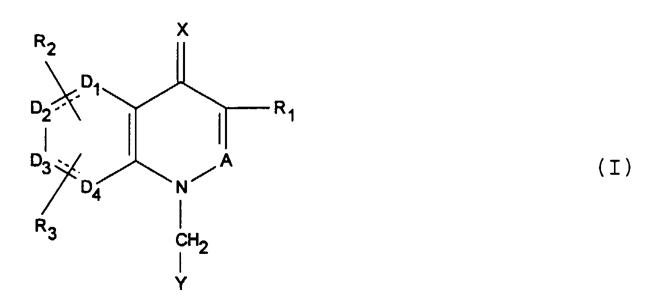 dans laquelle : 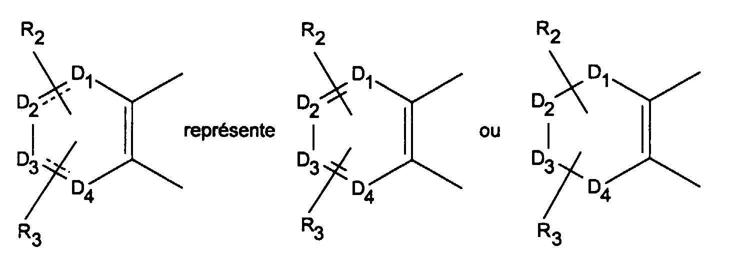
avec D₁, D₂ représentant tous deux un radical méthine ou un radical méthylène, éventuellement substitués par un radical choisi parmi R₂ et R₃,
D₃ et D₄, identiques ou différents, représentant un atome d'azote, un radical méthine ou un radical méthylène, éventuellement substitués par un radical choisi parmi R₂ et R₃,
l'un au moins de D₃ et D₄ représentent un radical méthine ou méthylène, éventuellement substitué par un radical choisi parmi R₂ et R₃,
R₁ représente un radical méthyle, éthyle, n-propyle, n-propényle, n-butyle,
R₂ et R₃, identiques ou différents, sont choisis dans le groupe formé par :
- l'atome d'hydrogène,
- les radicaux mercapto et alkylthio,
- les radicaux alkyle linéaires ou ramifiés renfermant au plus 6 atomes de carbone et éventuellement substitués par un ou plusieurs substituants identiques ou différents choisis parmi les atomes d'halogène, le radical hydroxyle, le radical amino, mono et dialkylamino, pyrrolidinyle, morpholinyle, pipéridinyle,
- le radical carboxy libre, salifié ou estérifié par un radical alkyle linéaire ou ramifié renfermant au plus 4 atomes de carbone,
- le radical pyrrolidinyl-carbonyle, morpholinyl-carbonyle, carbamoyle, dialkylcarbamoyle, pipéridynyl-carbonyle,
A représente un atome d'azote ou un radical CH,
X représente un atome d'oxygène ou un atome de soufre,
Y représente un radical phényle ou biphényle éventuellement substitué par un ou plusieurs radicaux choisis parmi les radicaux cyano, carboxy libre, salifié et estérifié, tétrazole et isoxazole,
à l'exclusion des produits de formule (I) dans laquelle 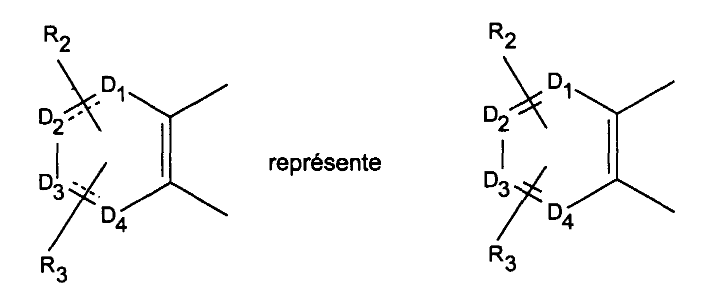
avec D₁, D₂, D₃ et D₄ représentant chacun un radical méthine X représente un atome d'oxygène et A représente un radical CH, R₁, R₂ et R₃, identiques représentent tous trois un radical méthyle, et -Y représente un radical phényle,
lesdits produits de formule I étant sous toutes les formes isomères possibles racémiques, énantiomères et diastéréoisomères, ainsi que les sels d'addition avec les acides minéraux et organiques ou avec les bases minérales et organiques, desdits produits de formule (I).

2. Les produits suivants :
. Acide 4'-((3-butyl-1,4-dihydro-7-formyl-4-oxo-1-quinoléinyl)méthyl)-(1,1'-biphényl)-2-carboxylique.
. Acide 3-butyl-1-(2'-carboxy-(1,1'-biphényl)-4-yl)méthyl)-1,4-dihydro-4-oxo-7-quinoléinepropanoique.
. Acide 4'-((3-butyl-6-(2-carboxyéthenyl)-1,4-dihydro-4-oxo-1-quinoléinyl) méthyl)-(1,1'-biphényl)-2-carboxylique.
. Acide 4'-((3-butyl-1,4-dihydro-6-formyl-4-oxo-1-quinoléinyl)méthyl)-(1,1'-biphényl)-2-carboxylique.
. Acide 4'-(3-butyl-1,4-dihydro-7-(3-hydroxy-3-oxo-1-propényl)-4-oxo-1-quinoléinyl)méthyl)-(1,1'-biphényl)-2-carboxylique.
. Acide 3-butyl-1-((2'-carboxy-(1,1'-biphényl)-4-yl)méthyl)-1,4-dihydro-4-oxo-7-quinoléineacétique.
. Acide 4'-((3-butyl-1,4-dihydro-4-oxo-7-(2-(1-pyrrolidinyl)-2-oxoéthyl)-1-quinoléinyl)méthyl)-(1,1'-biphényl)-2-carboxylique.
. Acide 1,4-dihydro-4-oxo-1((2'-(1H-tétrazol-5-yl)(1,1'-biphényl)-4-yl)méthyl-quinoléine-3,8-dicarboxylique.
. 3-butyl-1-((2'-(1H-tétrazol-5-yl)-1,1'-biphényl)-4-yl)méthyl)-6,7,8-trifluoro-4(1H)-quinoléinone.
. 3-butyl-6,8-difluoro-7-(1-pyrrolidinyl)-1-((2'-(1H-tétrazol5-yl)-(1,1'-biphényl)-4-yl)méthyl)-4-(1H)-quinoléinone
ces produits étant sous toutes les formes isomères possibles racémiques, énantiomères et diastéréoisomères, ainsi que les sels d'addition avec les acides minéraux et organiques ou avec les bases minérales et organiques, de ces produits.

3. Les produits selon la revendication 1 repondant aux définitions suivantes :
. l'acide 4'-[(3-butyl 1,4-dihydro-4-oxo-1-quinoléinyl) méthyl] (1,1'-biphényl)-2-carboxylique,
. l'acide 4'-[(3-butyl 1,4-dihydro-4-thioxo-1-quinoléinyl) méthyl] (1,1'-biphényl) 2-carboxylique,
. l'acide 3-butyl 1-[(2'-carboxy (1,1'-biphényl) 4-yl) méthyl] 1,4-dihydro-4-oxo-7-quinoléinecarboxylique,
. l'acide 4'-[(3-butyl 1,4-dihydro 7-(hydroxyméthyl) 4-oxo 1-quinoléinyl) méthyl] (1,1'-biphényl) 2-carboxylique,
. l'acide 4'-[(3-butyl 1,4-dihydro 4-oxo 7-[(1-pyrrolidinyl) carbonyl] 1-quinoléinyl) méthyl] (1,1'-biphényl) 2-carboxylique,
. l'acide 4'-[(3-butyl 1,4,5,6,7,8-hexahydro 4-oxo 1-[(1-quinoléinyl) méthyl] (1,1'-biphényl) 2-carboxylique,
. l'acide 4'-[(3-butyl 1,4,5,6,7,8-hexahydro 6-méthyl 4-oxo 1-quinoléinyl) méthyl] (1,1'-biphényl) 2-carboxylique,
. l'acide 3-butyl 1,4-dihydro 1-[[2'-(1H-tétrazol-5-yl) (1,1'-biphényl) 4-yl] méthyl]4-oxo 8-quinoléinecarboxylique,
. l'acide 1,4-dihydro 3-méthyl 4-oxo 1-[[2'-(1H-tétrazol-5-yl) (1,1'-biphényl) 4-yl] méthyl] 8-quinoléinecarboxylique,
. l'acide 1,4-dihydro 3-éthyl 4-oxo 1-[[2'-(1H-tétrazol-5-yl) (1,1'-biphényl) 4-yl] méthyl] 8-quinoléinecarboxylique.

4. Procédé de préparation de produits de formule (I) telle que définie à la revendication 1 et des produits tels que définis à la revendication 2, caractérisé en ce que :
**soit** l'on fait réagir un produit de formule (II) : 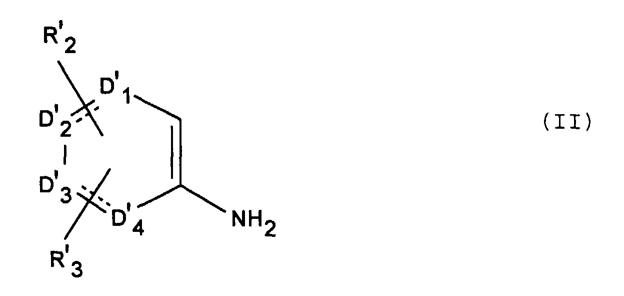 dans laquelle D'₁, D'₂, D'₃, D'₄, R'₂ et R'₃ ont les significations indiquées à la revendication 1, respectivement pour D₁, D₂, D₃, D₄, R₂ et R₃, ou correspondant aux produits tels que définis à la revendication 2, dans lesquelles les éventuelles fonctions réactives sont éventuellement protégées par des groupements protecteurs, avec un produit de formule (III) : 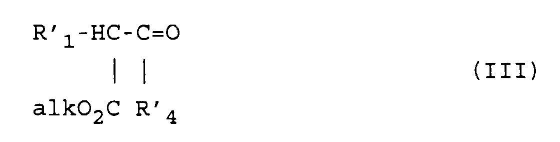 dans laquelle R'₁ a la signification indiquée à la revendication 1 pour R₁ et R,₄ représente un atome d'hydrogène ou le radical carboxy, dans lesquelles les éventuelles fonctions réactives sont éventuellement protégées par des groupements protecteurs et alk représente un radical alkyle renfermant au plus 6 atomes de carbone, pour obtenir des produits de formule (IV) : 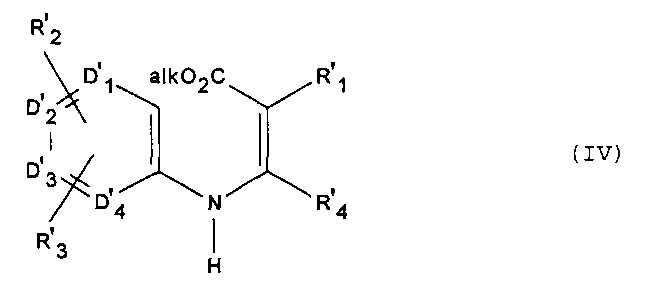 dans laquelle D'₁, D'₂, D'₃, D'₄, R'₁, R'₂, R'₃, R'₄ et alk ont les significations indiquées ci-dessus, que l'on cyclise pour obtenir des produits de formule (V) : 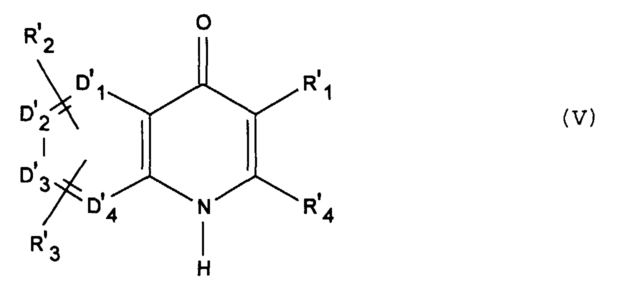 dans laquelle D'₁, D'₂, D'₃, D'₄, R'₁, R'₂, R'₃ et R'₄ ont lessignifications indiquées ci-dessus,
**soit** l'on fait réagir un produit de formule (VI) : 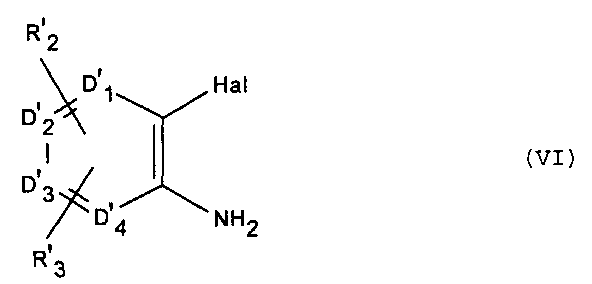 dans laquelle D'₁, D'₂, D'₃, D'₄, R'₂ et R'₃ ont les significations indiquées ci-dessus et Hal représente un atome d'halogène, avec un produit de formule (VII) :
R'₁-C≡CH (VII)
dans laquelle R'₁ a la signification indiquée ci-dessus, pour obtenir des produits de formule (VIII) : 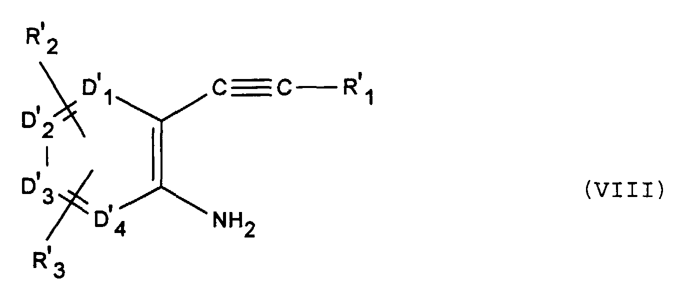 dans laquelle D'₁, D'₂, D'₃, D'₄, R'₁, R'₂ et R'₃ ont les significations indiquées ci-dessus, que l'on soumet à une réaction de cyclisation pour obtenir un produit de formule (IX): 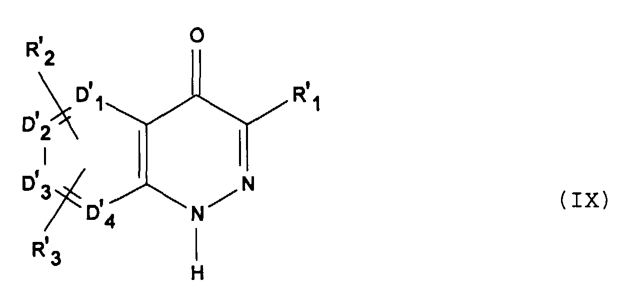 dans laquelle D'₁, D'₂, D'₃, D'₄, R'₁, R'₂ et R'₃ ont les significations indiquées ci-dessus, produits de formules (V) et (IX) que, si désiré, l'on soumet à une réaction d'hydrogénation pour obtenir respectivement les produits de formule (V_{B}) : 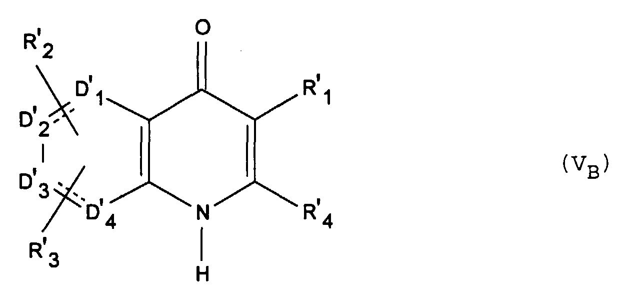 et les produits de formule (IX_{B}) : 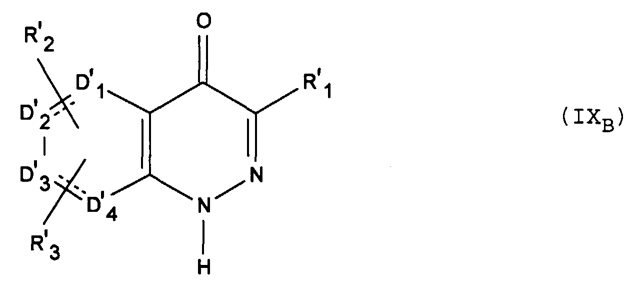 produits de formules (V_{B}) et (IX_{B}) dans lesquels D'₁, D'₂, D'₃, D'₄, R'₁, R'₂, R'₃ et R'₄ ont les significations indiquées ci-dessus, et produits de formules (V), (VB), (IX) et (IX) que si désiré, l'on soumet à une réaction de transformation de la fonction oxo en une fonction thioxo et que l'on fait réagir avec un composé de formule (X) :
Hal-CH₂-Y' (X)
dans laquelle Hal représente un atome d'halogène et Y' a la signification indiquée ci-dessus, pour Y dans laquelle les éventuelles fonctions réactives sont éventuellement protégées par des groupements protecteurs, pour obtenir un produit de formule (I') : 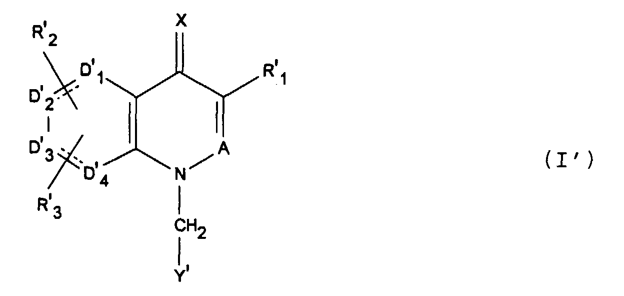 dans laquelle : 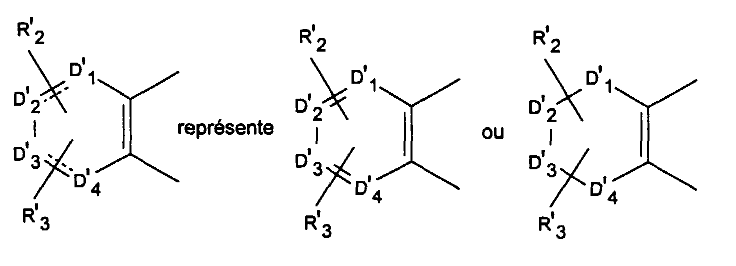 D'₁, D'₂, D'₃, D'₄, R'₁, R'₂, R'₃, R'₄, R'₅ et Y' ont la signification indiquée ci-dessus, A représente le radical C-R'₄ ou un atome d'azote, et X a la signification indiquée ci-dessus, produits de formule (I') dans laquelle 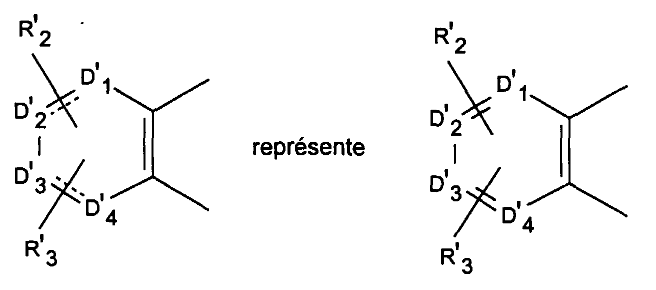 que l'on peut, si désiré, soumettre à une réaction d'hydrogénation pour obtenir des produits de formule (I') dans laquelle 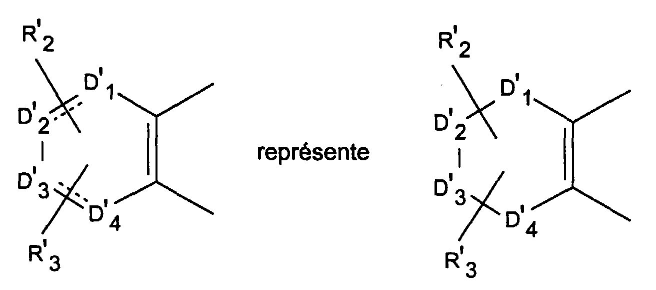 produit de formule (I') que l'on soumet, si désiré et si nécessaire, à l'une ou plusieurs des réactions suivantes, dans un ordre quelconque :
- une réaction de transformation de fonction oxo C=O en fonction thioxo C=S,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
les produits ainsi obtenus étant sous toutes les formes isomères possibles racémiques, énantiomères et diastéréoisomè res.

5. Procédé de préparation de produits de formule(I), telle que définie à la revendication 1 et des produits tels que définis à la revendication 2, dans laquelle A représente CH caractérisé en ce que :
**soit** l'on fait réagir un produit de formule (II_{E}) : 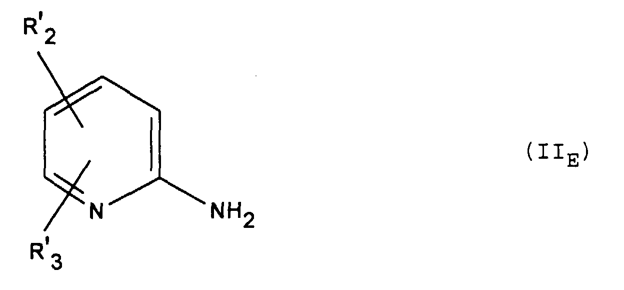 dans laquelle R'₂ et R'₃ ont les significations indiquées à la revendication 1, respectivement pour R₂ et R3, ou correspondant aux produits tels que définis à la revendication 2, dans lesquelles les éventuelles fonctions réactives sont éventuellement proté gées par des groupements protecteurs, avec un produit de formule (XI) : 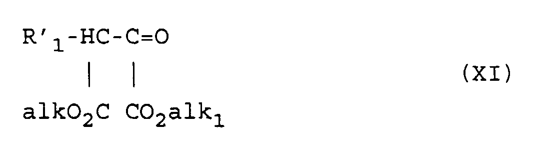 dans laquelle R'₁ a la signification indiquée à la revendication 1, pour R₁ dans laquelle les éventuelles fonctions réactives sont éventuellement protégées par des groupements protecteurs et alk et alk₁, identiques ou différents, représentent un radical alkyle renfermant au plus 6 atomes de carbone, pour obtenir des produits de formule (XII) : 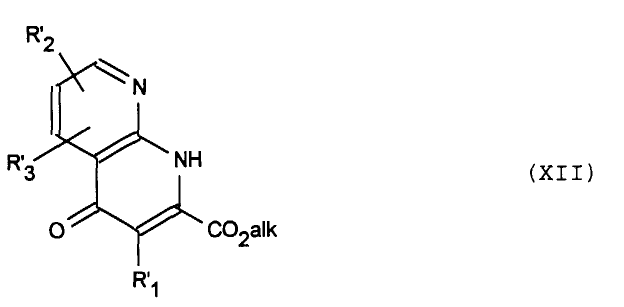 dans laquelle R'₁, R'₂, R'₃ et alk ont les significations indiquées ci-dessus,
**soit** l'on fait réagir un produit de formule (II_{F}) : 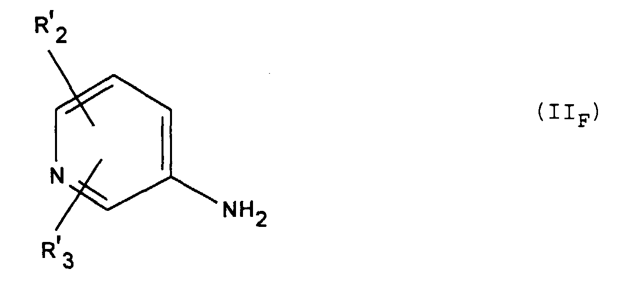 dans laquelle R'₂ et R'3 ont les significations indiquées ci-dessus, avec le produit de formule (XI) tel que défini ci-dessus, pour obtenir des produits de formule (XIII) : 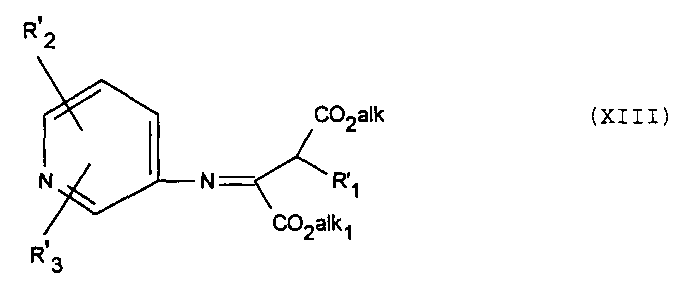 dans laquelle R'1, R'₂', R'₃, alk et alk₁ ont les significations indiquées ci-dessus, produits de formules (XII) et (XIII) que l'on transforme en produit de formule (XIV) : 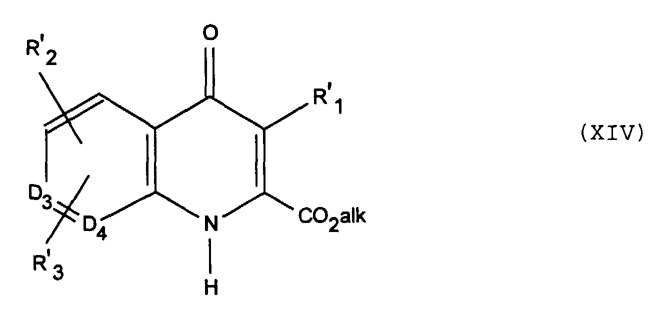 dans laquelle R'₁, R'₂, R'₃ et alk₁ ont les significations indiquées ci-dessus et D₃ et D₄ ont les significations indiquées à la revendication 1, produits de formule (XIV) que, si désiré, l'on soumet à une réaction d'hydrogénation pour obtenir les produits de formule (XV) : 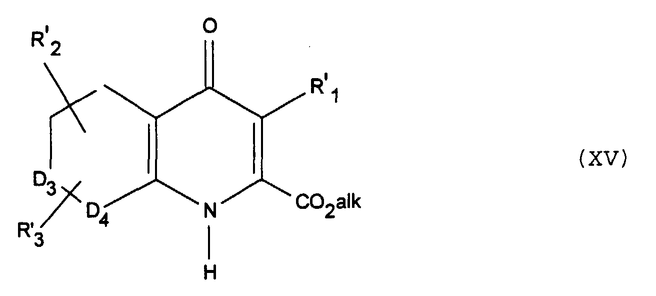 dans laquelle D₃, D₄,, R'₁, R'₂ et R'₃ et alk₁ ont les significations indiquées ci-dessus, produits de formules (XIV) et (XV) que l'on soumet à une réaction de saponification puis de décarboxylation, pour obtenir les produits correspondants de formule (XIV') : 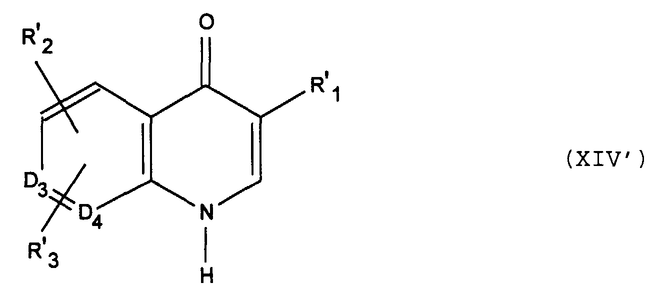 et de formule (XV') : 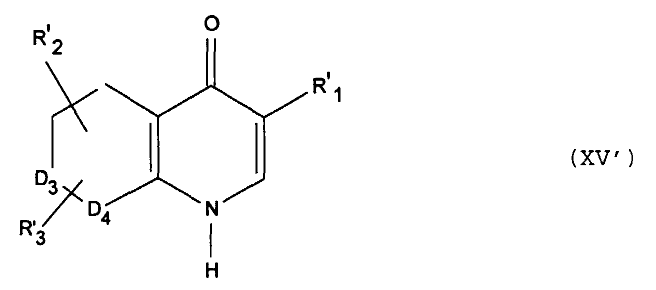 produits de formules (XIV') et (XV') que si désiré, l'on soumet à une réaction de transformation de la fonction oxo en une fonction thioxo et que l'on fait réagir avec un composé de formule (X) :
Hal-CH2-Y' (X)
dans laquelle Y' a la signification indiquée à la revendication 1 pour Y dans laquelle les éventuelles fonctions réactives sont éventuellement protégées par des groupements protecteurs, pour obtenir un produit de formule (I'_{EB}) : 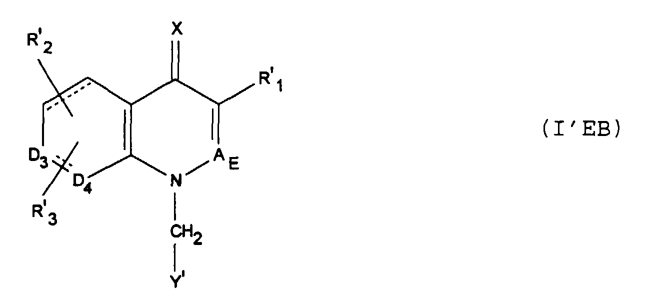 dans laquelle : 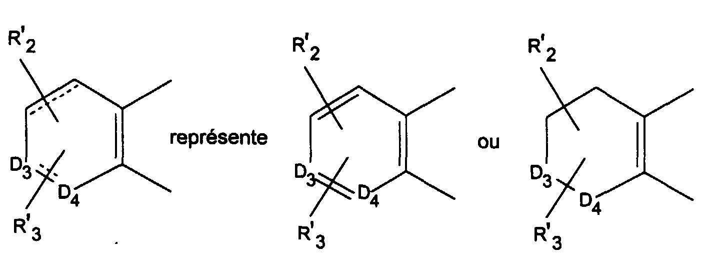 D₃, D₄, R'₁, R'₂, R'₃ et Y' ont les significations indiquées ci-dessus, A_{E} représente le radical CH et X a la signification indiquée ci-dessus, produits de formule (I'_{EB}) dans laquelle : 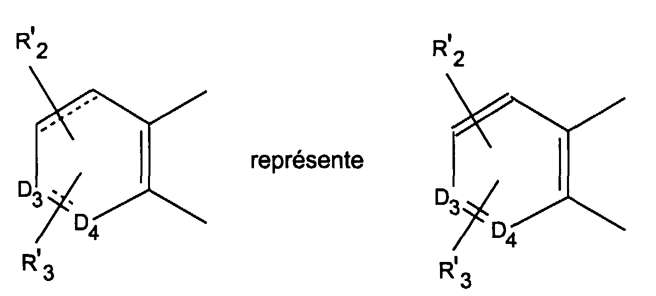 que l'on peut, si désiré, soumettre à une réaction d'hydrogénation pour obtenir des produits de formule (I'_{EB}) dans laquelle 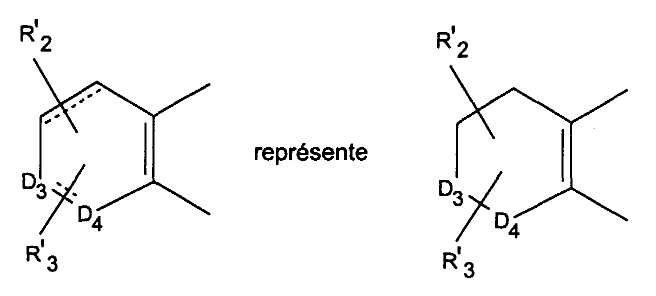 produit de formule (I'_{EB}) que l'on soumet, si désiré et si nécessaire, à l'une ou plusieurs des réactions suivantes, dans un ordre quelconque :
- une réaction de transformation de fonction oxo C=O en fonction thioxo C=S,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
les produits ainsi obtenus étant sous toutes les formes isomères possibles racémiques, énantiomères et diastéréoisomères.

6. A titre de médicaments, les produits tels que définis aux revendications 1 à 3, ces produits étant sous toutes les formes isomères possibles racémiques, énantiomères et diastéréoisomères, ainsi que les sels d'addition avec les acides minéraux et organiques ou avec les bases minérales et organiques pharmaceutiquement acceptables de ces produits.

7. A titre de médicaments, les produits tels que définis à la revendication 2, ainsi que les sels d'addition avec les acides minéraux et organiques ou avec les bases minérales et organiques pharmaceutiquement acceptables de ces produits.

8. Les compositions pharmaceutiques contenant à titre de principe actif, l'un au moins des médicaments tels que définis à l'une quelconque des revendications 6 et 7.

9. Utilisation des produits tels que définis aux revendications 1 à 3,
ces produits étant sous toutes les formes isomères possibles racémiques, énantiomères et diastéréoisomères, ainsi que les sels d'addition avec les acides minéraux et organiques ou avec les bases minérales et organiques de ces produits,
pour la préparation de médicaments, destinés :
**soit** au traitement de l'hypertension artérielle, des insuffisances cardiaques, du glaucome, des insuffisances rénales et dans la prévention des resténoses post-angioplastie,
**soit** au traitement de certains désordres gastro-intestinaux ou gynécologiques.
**soit** au traitement des troubles de la mémoire et des fonctions cognitives, de l'anxiété, de la dépression, de la démence sénile et de la maladie d'Alzheimer.

## Revendications (Revendications pour l'(les) Etat(s) contractant(s) suivant(s): ES, GR)

1. Procédé de préparation des produits de formule (I) : 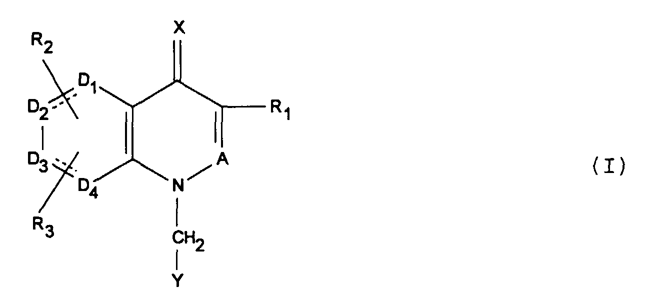 dans laquelle : 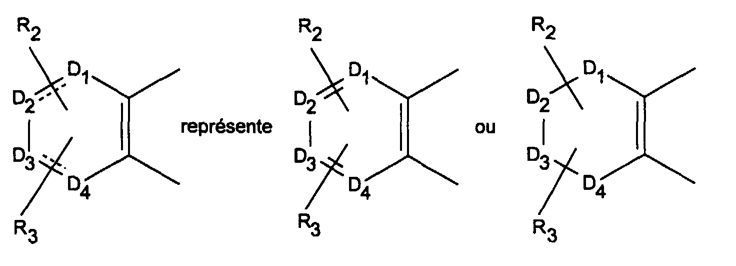
avec D₁, D₂ représentant tous deux un radical méthine ou un radical méthylène, éventuellement substitués par un radical choisi parmi R₂ et R₃,
D₃ et D₄, identiques ou différents, représentant un atome d'azote, un radical méthine ou un radical méthylène, éventuellement substitués par un radical choisi parmi R₂ et R₃,
l'un au moins de D₃ et D₄ représentent un radical méthine ou méthylène, éventuellement substitué par un radical choisi parmi R₂ et R₃,
R₁ représente un radical méthyle, éthyle, n-propyle, n-propényle, n-butyle,
R₂ et R₃, identiques ou différents, sont choisis dans le groupe formé par :
- l'atome d'hydrogène,
- les radicaux mercapto et alkylthio,
- les radicaux alkyle linéaires ou ramifiés renfermant au plus 6 atomes de carbone et eventuellement substitués par un ou plusieurs substituants identiques ou différents choisis parmi les atomes d'halogène, le radical hydroxyle, le radical amino, mono et dialkylamino, pyrrolidinyle, morpholinyle, pipéridinyle,
- le radical carboxy libre, salifié ou estérifié par un radical alkyle linéaire ou ramifié renfermant au plus 4 atomes de carbone,
- le radical pyrrolidinyl-carbonyle, morpholinyl-carbonyle, carbamoyle, dialkylcarbamoyle, pipéridynyl-carbonyle,
A représente un atome d'azote ou un radical CH,
X représente un atome d'oxygène ou un atome de soufre,
Y représente un radical phényle ou biphényle éventuellement substitué par un ou plusieurs radicaux choisis parmi les radicaux cyano, carboxy libre, salifié et estérifié, tétrazole et isoxazole,
à l'exclusion des produits de formule (I) dans laquelle 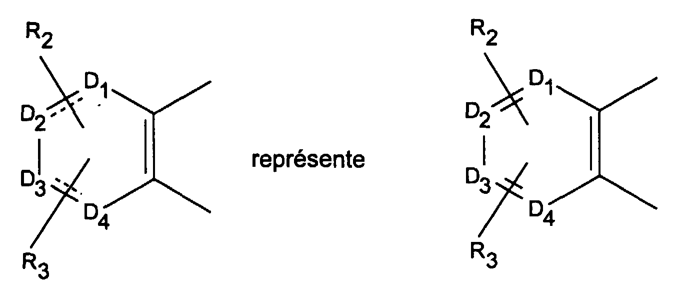
avec D₁, D₂, D₃ et D₄ représentant chacun un radical méthine X représente un atome d'oxygène et A représente un radical CH, R₁, R₂ et R₃, identiques représentent tous trois un radical méthyle, et -Y représente un radical phényle,
lesdits produits de formule (I) étant sous toutes les formes isomères possibles racémiques, énantiomères et diastéréoisomères, ainsi que les sels d'addition avec les acides minéraux et organiques ou avec les bases minérales et organiques, desdits produits de formule (I)caractérisé en ce que :
**soit** l'on fait réagir un produit de formule (II) : 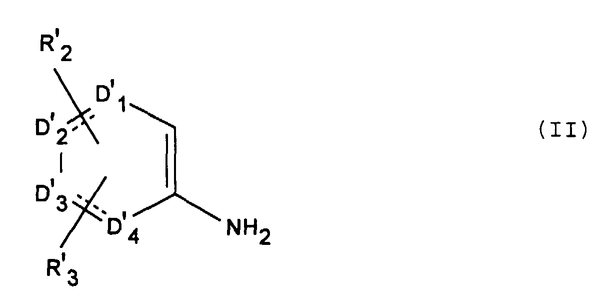 dans laquelle D'₁, D'₂, D'₃, D'₄, R'₂ et R'₃ ont les significations indiquées ci-dessus, respectivement pour D₁, D₂, D₃, D₄, R₂ et R₃ dans lesquelles les éventuelles fonctions réactives sont éventuellement protégées par des groupements protecteurs, avec un produit de formule (III) : 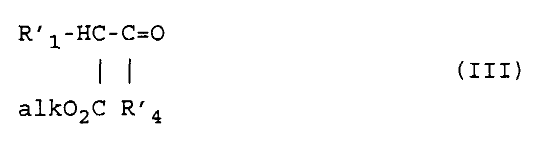 dans laquelle R'₁ a la signification indiquée ci-dessus pour R₁ et R,₄ représente un atome d'hydrogène ou le radical carboxy, dans lesquelles les éventuelles fonctions réactives sont éventuellement protégées par des groupements protecteurs et alk représente un radical alkyle renfermant au plus 6 atomes de carbone, pour obtenir des produits de formule (IV): 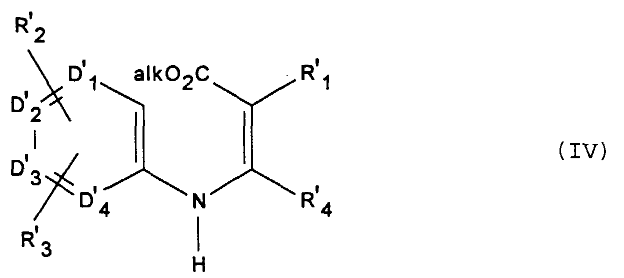 dans laquelle D'₁, D'₂, D'₃, D'₄, R'₁, R'₂, R'₃, R'₄ et alk ont les significations indiquées ci-dessus, que l'on cyclise pour obtenir des produits de formule (V) : 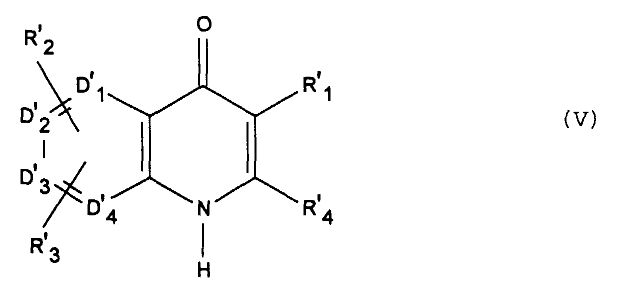 dans laquelle D'₁, D'₂, D'₃, D'₄, R'₁, R'₂, R'₃ et R'₄ ont les significations indiquées ci-dessus,
**soit** l'on fait réagir un produit de formule (VI) : 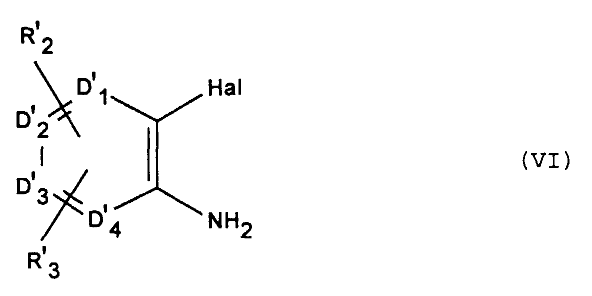 dans laquelle D'₁, D'₂, D'₃, D'₄, R'₂ et R'₃ ont les significations indiquées ci-dessus et Hal représente un atome d'halogène, avec un produit de formule (VII) :
R'₁-C≡CH (VII)
dans laquelle R'₁ a la signification indiquée ci-dessus, pour obtenir des produits de formule (VIII) : 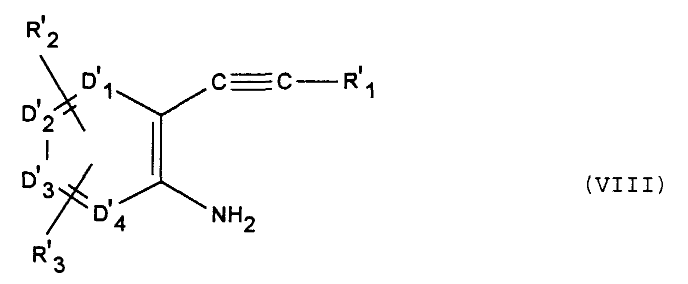 dans laquelle D'₁, D'₂, D'₃, D'₄, R'₁, R'₂ et R'₃ ont les significations indiquées ci-dessus, que l'on soumet à une réaction de cyclisation pour obtenir un produit de formule (IX) : 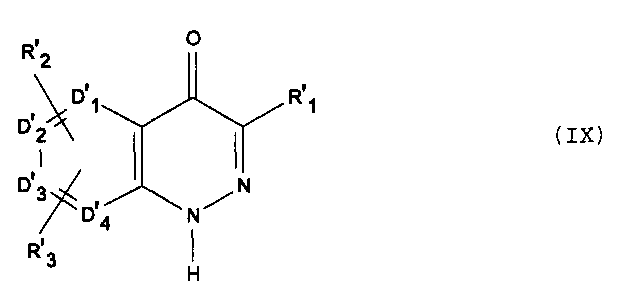 dans laquelle D'₁, D'₂, D'₃, D'₄, R'₁, R'₂ et R'₃ ont les significations indiquées ci-dessus, produits de formules (V) et (IX) que, si désiré, l'on soumet à une réaction d'hydrogénation pour obtenir respectivement les produits de formule (V_{B}) : 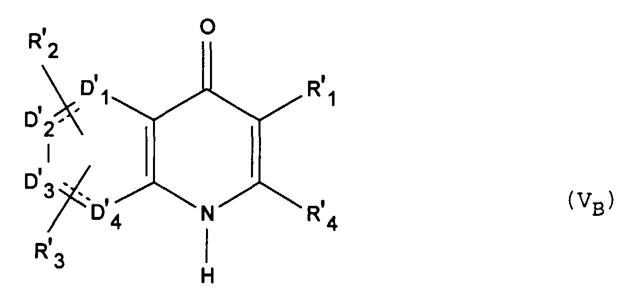 et les produits de formule (IX_{B}) : 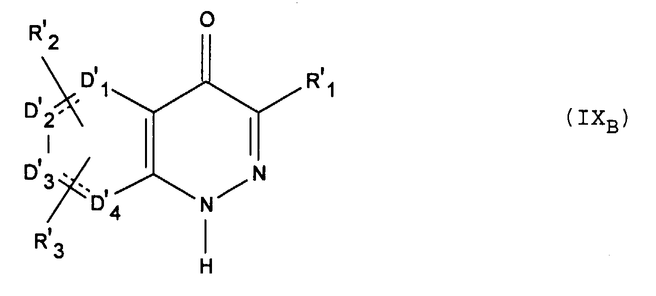 produits de formules (V_{B}) et (IX_{B}) dans lesquels D'₁, D'₂, D'₃, D'₄, R'₁, R'₂, R'₃ et R'₄ ont les significations indiquées ci-dessus, et produits de formules (V, (VB), (IX) et (IX) que si désiré, l'on soumet à une réaction de transformation de la fonction oxo en une fonction thioxo et que l'on fait réagir avec un composé de formule (X) :
Hal-CH₂-Y' (X)
dans laquelle Hal représente un atome d'halogène et Y' a la signification indiquée ci-dessus pour Y, dans laquelle les éventuelles fonctions réactives sont éventuellement protégées par des groupements protecteurs, pour obtenir un produit de formule (I') : 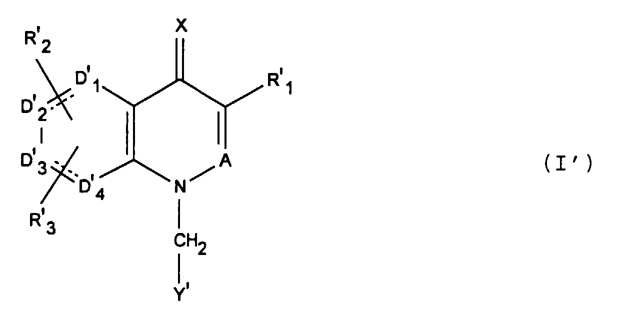 dans laquelle : 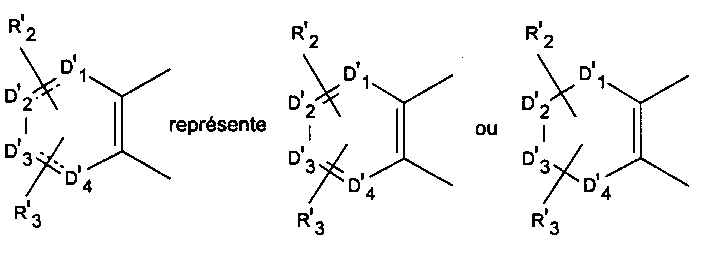 D'₁, D'₂, D'₃, D'₄, R'₁, R'₂, R'₃, R'₄, R'₅ et Y' ont la signification indiquée ci-dessus, A représente le radical C-R'₄ ou un atome d'azote, et X a la signification indiquée ci-dessus, produits de formule (I') dans laquelle 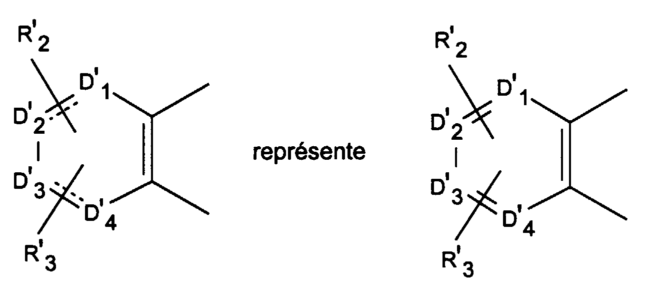 que l'on peut, si désiré, soumettre à une réaction d'hydrogénation pour obtenir des produits de formule (I') dans laquelle 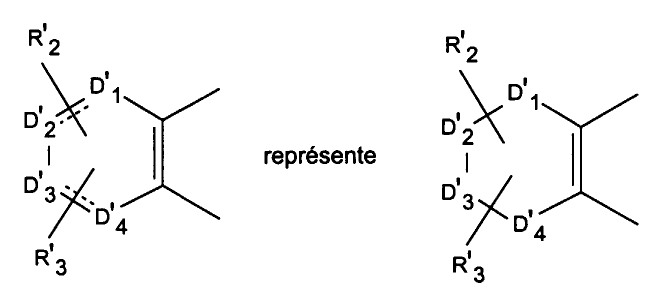 produit de formule (I') que l'on soumet, si désiré et si nécessaire, à l'une ou plusieurs des réactions suivantes, dans un ordre quelconque :
- une réaction de transformation de fonction oxo C=O en fonction thioxo C=S,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
les produits ainsi obtenus étant sous toutes les formes isomères possibles racémiques, énantiomères et diastéréoisomè res.

2. Procédé tel que défini à la revendication 1 de préparation des produits suivants :
. Acide 4'-((3-butyl-1,4-dihydro-7-formyl-4-oxo-1-quinoléinyl)méthyl)-(1,1'-biphényl)-2-carboxylique.
. Acide 3-butyl-1-(2'-carboxy-(1,1'-biphényl)-4-yl)méthyl)-1,4-dihydro-4-oxo-7-quinoléinepropanoique.
. Acide 4'-((3-butyl-6-(2-carboxyéthenyl)-1,4-dihydro-4-oxo-1-quinoléinyl) méthyl)-(1,1'-biphényl)-2-carboxylique.
. Acide 4'-((3-butyl-1,4-dihydro-6-formyl-4-oxo-1-quinoléinyl)méthyl)-(1,1'-biphényl)-2-carboxylique.
. Acide 4'-(3-butyl-1,4-dihydro-7-(3-hydroxy-3-oxo-1-propényl)-4-oxo-1-quinoléinyl)méthyl)-(1,1'-biphényl)-2-carboxylique.
. Acide 3-butyl-1-((2'-carboxy-(1,1'-biphényl)-4-yl)méthyl)-1,4-dihydro-4-oxo-7-quinoléineacétique.
. Acide 4'-((3-butyl-1,4-dihydro-4-oxo-7-(2-(1-pyrrolidinyl)-2-oxoéthyl)-1-quinoléinyl)méthyl)-(1,1'-biphényl)-2-carboxylique.
. Acide 1,4-dihydro-4-oxo-1((2'-(1H-tétrazol-5-yl)(1,1'-biphényl)-4-yl)méthyl-quinoléine-3,8-dicarboxylique.
. 3-butyl-1-((2'-(1H-tétrazol-5-yl)-1,1'-biphényl)-4-yl)méthyl)-6,7,8-trifluoro-4(1H)-quinoléinone.
. 3-butyl-6,8-difluoro-7-(1-pyrrolidinyl)-1-((2'-(1H-tétrazol-5-yl)-(1,1'-biphényl)-4-yl)méthyl)-4-(1H)-quinoléinone
Ces produits étant sous toutes les formes isomères possibles racémiques, énantiomères et diastéréoisomères, ainsi que les sels d'addition avec les acides minéraux et organiques ou avec les bases minérales et organiques de ces produits.

3. Procédé de préparation des produits de formule (I) : 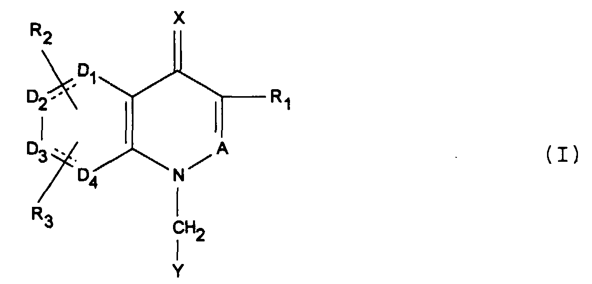 dans laquelle : 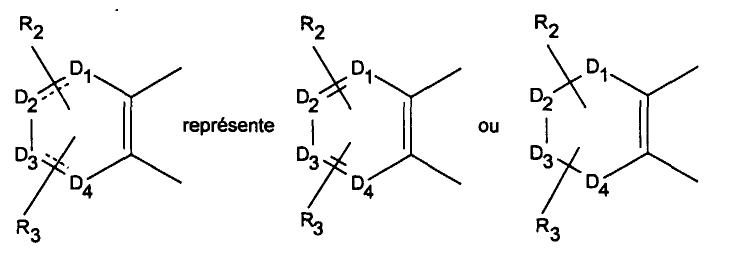
avec D₁, D₂ représentant tous deux un radical méthine ou un radical méthylène, éventuellement substitués par un radical choisi parmi R₂ et R₃,
D₃ et D₄, identiques ou différents, représentant un atome d'azote, un radical méthine ou un radical méthylène, éventuellement substitués par un radical choisi parmi R₂ et R₃,
l'un au moins de D₃ et D₄ représentent un radical méthine ou méthylène, éventuellement substitué par un radical choisi parmi R₂ et R₃,
R₁ représente un radical méthyle, éthyle, n-propyle, n-propényle, n-butyle,
R₂ et R₃, identiques ou différents, sont choisis dans le groupe formé par :
- l'atome d'hydrogène,
- les radicaux mercapto et alkylthio,
- les radicaux alkyle linéaires ou ramifiés renfermant au plus 6 atomes de carbone et éventuellement substitués par un ou plusieurs substituants identiques ou différents choisis parmi les atomes d'halogène, le radical hydroxyle, le radical amino, mono et dialkylamino, pyrrolidinyle, morpholinyle, pipéridinyle,
- le radical carboxy libre, salifié ou estérifié par un radical alkyle linéaire ou ramifié renfermant au plus 4 atomes de carbone,
- le radical pyrrolidinyl-carbonyle, morpholinyl-carbonyle, carbamoyle, dialkylcarbamoyle, pipéridynyl-carbonyle,
A représente un atome d'azote ou un radical CH,
X représente un atome d'oxygène ou un atome de soufre,
Y représente un radical phényle ou biphényle éventuellement substitué par un ou plusieurs radicaux choisis parmi les radicaux cyano, carboxy libre, salifié et estérifié, tétrazole et isoxazole,
à l'exclusion des produits de formule (I) dans laquelle 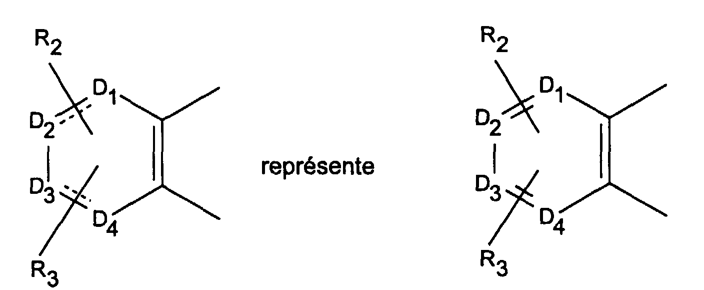
avec D₁, D₂, D₃ et D₄ représentant chacun un radical méthine X représente un atome d'oxygène et A représente un radical CH, R₁, R₂ et R₃, identiques représentent tous trois un radical méthyle, et -Y représente un radical phényle,
lesdits produits de formule I étant sous toutes les formes isomères possibles racémiques, énantiomères et diastéréoisomères, ainsi que les sels d'addition avec les acides minéraux et organiques ou avec les bases minérales et organiques, desdits produits de formule (I)caractérisé en ce que :
**soit** l'on fait réagir un produit de formule (II_{E}) : 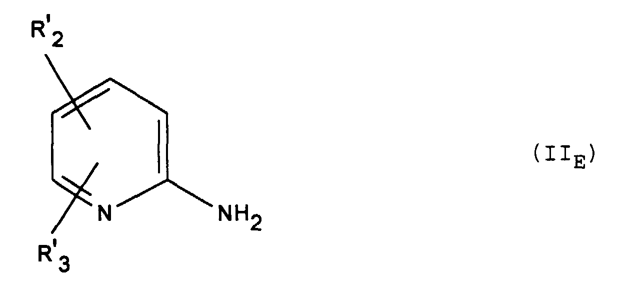 dans laquelle R'₂ et R'₃ ont les significations indiquées ci-dessus, respectivement pour R₂ et R3 dans lesquelles les éventuelles fonctions réactives sont éventuellement protégées par des groupements protecteurs, avec un produit de formule (XI) : 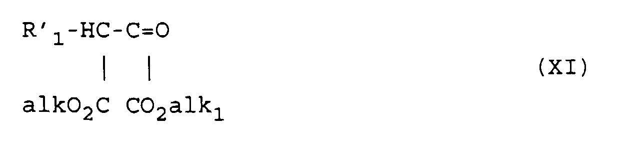 dans laquelle R'₁ a la signification indiquée à la revendication 1, pour R₁ dans laquelle les éventuelles fonctions réactives sont éventuellement protégées par des groupements protecteurs et alk et alk₁, identiques ou différents, représentent un radical alkyle renfermant au plus 6 atomes de carbone, pour obtenir des produits de formule (XII) : 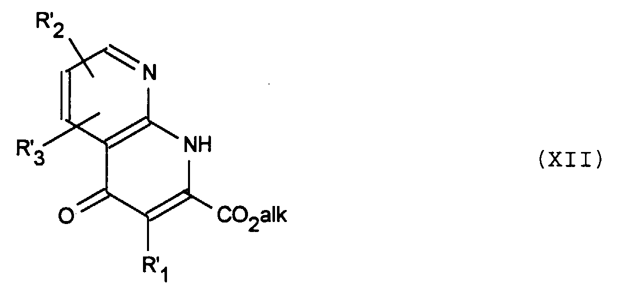 dans laquelle R'₁, R'₂, R'₃ et alk ont les significations indiquées ci-dessus,
**soit** l'on fait réagir un produit de formule (II_{F}) : 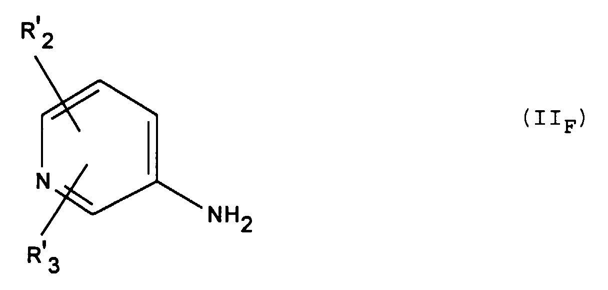 dans laquelle R'₂ et R'3 ont les significations indiquées ci-dessus, avec le produit de formule (XI) tel que défini ci-dessus, pour obtenir des produits de formule (XIII) : 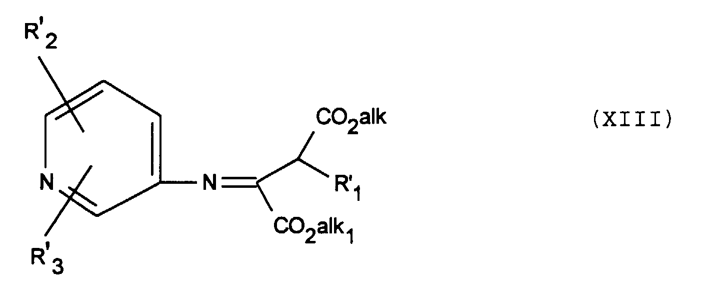 dans laquelle R'1, R'₂', R'₃, alk et alk₁ ont les significations indiquées ci-dessus, produits de formules (XII) et (XIII) que l'on transforme en produit de formule (XIV) : 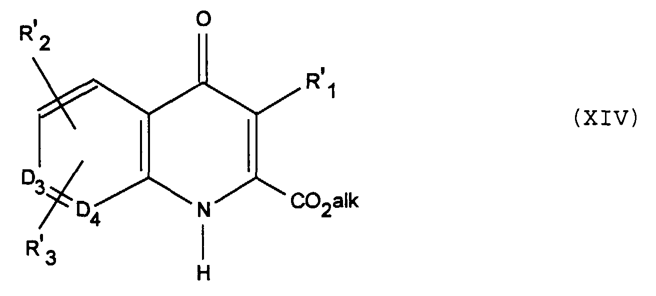 dans laquelle R'₁, R'₂, R'₃ et alk₁ ont les significations indiquées ci-dessus et D₃ et D₄ ont les significations indiquées à la revendication 1, produits de formule (XIV) que, si désiré, l'on soumet à une réaction d'hydrogénation pour obtenir les produits de formule (XV) : 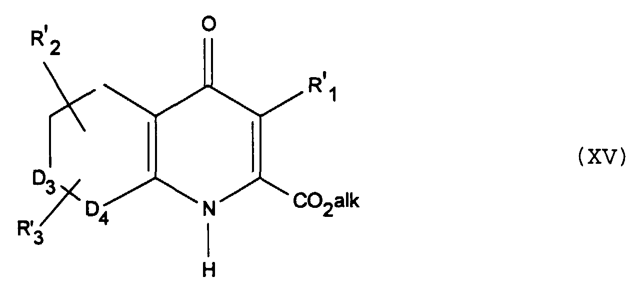 dans laquelle D₃, D₄, R'₁, R'₂ et R'₃ et alk₁ ont les significations indiquées ci-dessus, produits de formules (XIV) et (XV) que l'on soumet à une réaction de saponification puis de décarboxylation, pour obtenir les produits correspondants de formule (XIV') : 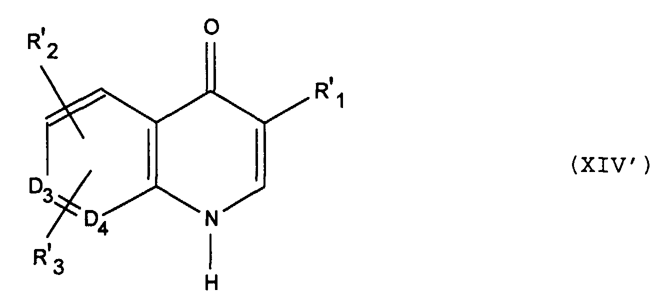 et de formule (XV') : 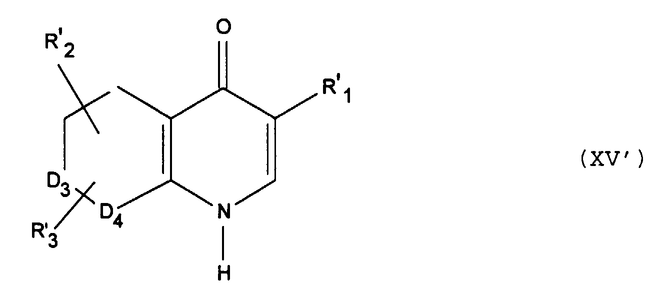 produits de formules (XIV') et (XV') que si désiré, l'on soumet à une réaction de transformation de la fonction oxo en une fonction thioxo et que l'on fait réagir avec un composé de formule (X) :
Hal-CH2-Y' (X)
dans laquelle Y'a la signification indiquée à la revendication 1 pour Y dans lesquelles les éventuelles fonctions réactives sont éventuellement protégées par des groupements protecteurs, pour obtenir un produit de formule (I'_{EB}) : 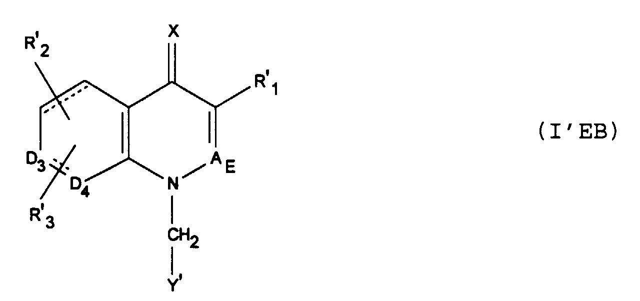 dans laquelle : 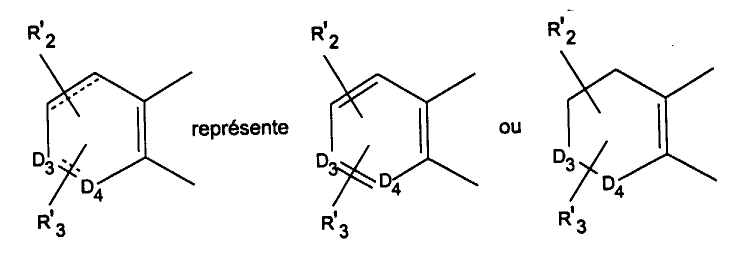 D₃, D₄, R'₁, R'₂, R'₃, R'₅ et Y' ont les significations indiquées ci-dessus, A_{E} représente le radical CH et X a la signification indiquée ci-dessus, produits de formule (I'_{EB}) 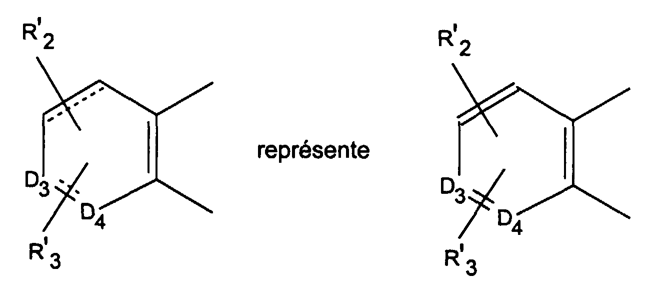 que l'on peut, si désiré, soumettre à une réaction d'hydrogénation pour obtenir des produits de formule (I'_{EB}) dans laquelle 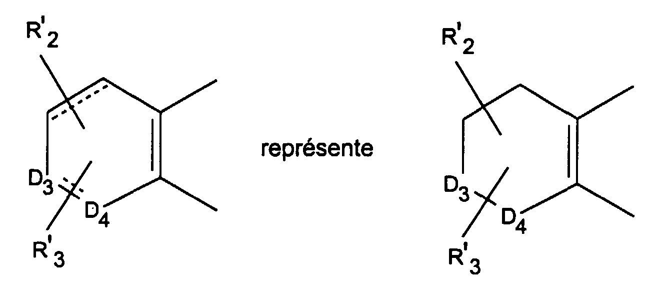 produit de formule (I'_{EB}) que l'on soumet, si désiré et si nécessaire, à l'une ou plusieurs des réactions suivantes, dans un ordre quelconque :
- une réaction de transformation de fonction oxo C=O en fonction thioxo C=S,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide, - une réaction de saponification de fonction ester en fonction acide,
- une réaction de transformation de fonction alkyloxy en fonction hydroxyle,
- une réaction de transformation de la fonction cyano en fonction acide,
- une réaction de réduction de la fonction carboxy en fonction alcool,
- une réaction de dédoublement des formes racémiques,
les produits ainsi obtenus étant sous toutes les formes isomères possibles racémiques, énantiomères et diastéréoisomères.

4. Procédé tel que défini à la revendication 3 de préparation des produits suivants :
. Acide 4'-((3-butyl-1,4-dihydro-7-formyl-4-oxo-1-quinoléinyl)méthyl)-(1,1'-biphényl)-2-carboxylique.
. Acide 3-butyl-1-(2'-carboxy-(1,1'-biphényl)-4-yl)méthyl)-1,4-dihydro-4-oxo-7-quinoléinepropanoique.
. Acide 4'-((3-butyl-6-(2-carboxyéthenyl)-1,4-dihydro-4-oxo-1-quinoléinyl) méthyl)-(1,1'-biphényl)-2-carboxylique.
. Acide 4'-((3-butyl-1,4-dihydro-6-formyl-4-oxo-1-quinoléinyl)méthyl)-(1,1'-biphényl)-2-carboxylique.
. Acide 4'-(3-butyl-1,4-dihydro-7-(3-hydroxy-3-oxo-1-propényl)-4-oxo-1-quinoléinyl)méthyl)-(1,1'-biphényl)-2-carboxylique.
. Acide 3-butyl-1-((2'-carboxy-(1,1'-biphényl)-4-yl)méthyl)-1,4-dihydro-4-oxo-7-quinoléineacétique.
. Acide 4'-((3-butyl-1,4-dihydro-4-oxo-7-(2-(1-pyrrolidinyl)-2-oxoéthyl)-1-quinoléinyl)méthyl)-(1,1'-biphényl)-2-carboxylique.
. Acide 1,4-dihydro-4-oxo-1((2'-(1H-tétrazol-5-yl)(1,1'-biphényl)-4-yl)méthyl-quinoléine-3,8-dicarboxylique.
. 3-butyl-1-((2'-(1H-tétrazol-5-yl)-1,1'-biphényl)-4-yl)méthyl)-6,7,8-trifluoro-4(1H)-quinoléinone.
. 3-butyl-6,8-difluoro-7-(1-pyrrolidinyl)-1-((2'-(1H-tétrazol-5-yl)-(1,1'-biphényl)-4-yl)méthyl)-4-(1H)-quinoléinone,
Ces produits étant sous toutes les formes isomères possibles racémiques, énantiomères et diastéréoisomères, ainsi que les sels d'addition avec les acides minéraux et organiques ou avec les bases minérales et organiques de ces produits, dans lesquelles A représente CH.

5. Procédé selon l'une quelconque des revendications 1 à 4 dans lequel les produits répondent aux définitions suivantes
. L'acide 4'-[(3-butyl 1,4-dihydro-4-oxo-1-quinoléinyl)-méthyl]-(1,1'-biphényl)-2-carboxylique.
. l'acide 4'-[(3-butyl 1,4-dihydro-4-thioxo-1-quinoléinyl)-méthyl](1,1'-biphényl)-2-carboxylique.
. l'acide 3-butyl 1-[(2'-carboxy (1,1'-biphényl)4-yl)méthyl] 1,4-dihydro-4-oxo-7-quinoléinecarboxylique.
. l'acide 4'-[(3-butyl 1,4-dihydro-7-(hydroxyméthyl) 4-oxo-1-quinoléinyl)-méthyl](1,1'-biphényl)-2-carboxylique
. l'acide 4'-[(3-butyl 1,4-dihydro- 4-oxo 7-pyrrolidiyl)-carbonyl]-1-quinoléinyl)-méthyl](1,1'-biphényl)-2-carboxylique
. l'acide 4'-[(3-butyl 1,4,5,6,7,8-hexahydro 4-oxo [(1-quinoléinyl)-méthyl](1,1'-biphényl)-2-carboxylique
. l'acide 4'-[(3-butyl 1,4,5,6,7,8-hexahydro 6-méthyl 4-oxo 1-quinoléinyl)-méthyl](1,1'-biphényl)-2-carboxylique
. l'acide 3-butyl 1,4-dihydro 1-[[2'-(1H-tétrazol-5-yl)(1,1-biphényl) 4-yl]méthyl]4-oxo-8-quinoléinecarboxylique
. l'acide 1,4-dihydro 3-méthyl 4-oxo 1-[[2'-(1H-tétrazol-5-yl)(1,1-biphényl) 4-yl]méthyl]-8-quinoléinecarboxylique
. l'acide 1,4-dihydro 3-éthyl 4-oxo 1-[[2'-(1H-tétrazol-5-yl)(1,1-biphényl) 4-yl]méthyl]-8-quinoléinecarboxylique

6. Procédé selon l'une quelconque des revendications 1 à 5 dans lequel les produits sont pour la préparation de médicaments, destines :
**soit** au traitement de l'hypertension artérielle, des insuffisances cardiaques, du glaucome, des insuffisances rénales et dans la prévention des resténoses post-angioplastie,
**soit** au traitement de certains désordres gastro-intestinaux ou gynécologiques.
**soit** au traitement des troubles de la mémoire et des fonctions cognitives, de l'anxiété, de la dépression, de la démence sénile et de la maladie d'Alzheimer.

## Claims (Claims for the following Contracting State(s): AT, BE, CH, LI, DE, DK, FR, GB, IT, LU, MC, NL, PT, SE)

1. The products of formula (I): 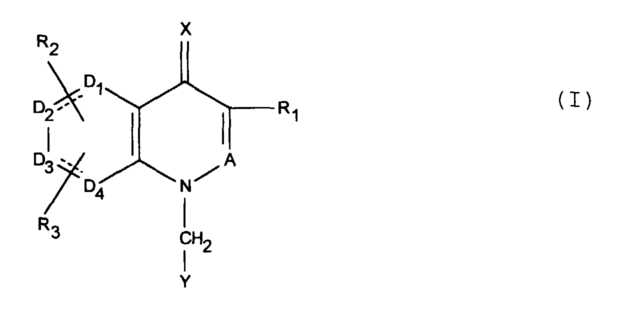 in which: 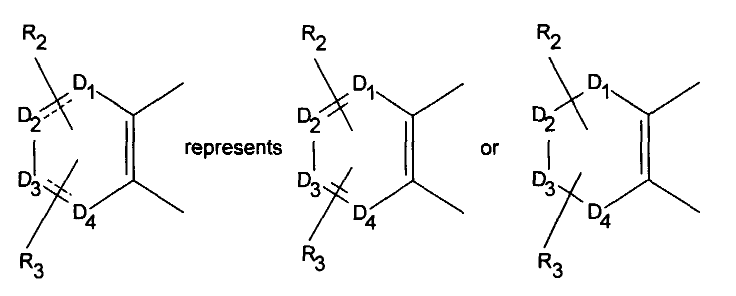
with D₁, D₂ both representing a methine radical or a methylene, radical, optionally substituted by a radical chosen from R₂ and R₃,
D₃ and D₄, identical or different, represent a nitrogen atom, a methine radical or a methylene radical, optionally substituted by a radical chosen from R₂ and R₃,
at least one of D₃ and D₄ represents a methine or methylene radical, optionally substituted by a radical chosen from R₂ and R₃,
R₁ represents a methyl, ethyl, n-propyl, n-propenyl, n-butyl radical,
R₂ and R₃, identical or different, are chosen from the group formed by:
- the hydrogen atom,
- the mercapto and alkylthio radicals,
- linear or branched alkyl radicals containing at most 6 carbon atoms and optionally substituted by one or more identical or different substituents chosen from halogen atoms, the hydroxyl radical, the amino, mono and dialkylamino, pyrrolidinyl, morpholinyl, piperidinyl radicals,
- the carboxy radical free, salified or esterified by a linear or branched alkyl radical containing at most 4 carbon atoms,
- the pyrrolidinyl-carbonyl, morpholinyl-carbonyl, carbamoyl, dialkylcarbamoyl, piperidynyl-carbonyl radicals,
A represents a nitrogen atom or a CH radical,
X represents an oxygen atom or a sulphur atom,
Y represents a phenyl or biphenyl radical optionally substituted by one or more radicals chosen from the cyano, free, salified and esterified carboxy, tetrazole and isoxazole radicals,
with the exception of the products of formula (I) in which 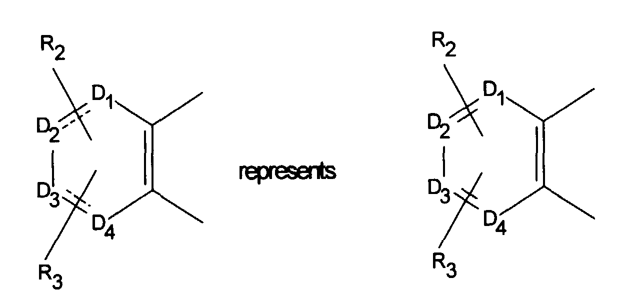
with D₁, D₂, D₃ and D₄ each representing a methine radical X represents an oxygen atom and A represents a CH radical, R₁, R₂ and R₃, being identical, all three represent a methyl radical, and -Y represents a phenyl radical,
the said products of formula I being in all possible racemic enantiomeric and diastereoisomeric isomer forms, as well as the addition salts with mineral and organic acids or with mineral and organic bases, of said products of formula (I).

2. The following products:
. 4'-((3-butyl-1,4-dihydro-7-formyl-4-oxo-1-quinolinyl)methyl)-(1,1'-biphenyl)-2-carboxylic acid.
. 3-butyl-1-(2'-carboxy-(1,1'-biphenyl)-4-yl)methyl)-1,4-dihydro-4-oxo-7-quinolinepropanoic acid.
. 4'-((3-butyl-6-(2-carboxyethenyl)-1,4-dihydro-4-oxo-1-quinolinyl) methyl)-(1,1'-biphenyl)-2-carboxylic acid.
. 4'-((3-butyl-1,4-dihydro-6-formyl-4-oxo-1-quinolinyl)methyl)-(1,1'-biphenyl)-2-carboxylic acid.
. 4'-(3-butyl-1,4-dihydro-7-(3-hydroxy-3-oxo-1-propenyl)-4-oxo-1-quinolinyl)methyl)-(1,1'-biphenyl)-2-carboxylic acid.
. 3-butyl-1-((2'-carboxy-(1,1'-biphenyl)-4-yl)methyl)-1,4-dihydro-4-oxo-7-quinolineacetic acid.
. 4'-((3-butyl-1,4-dihydro-4-oxo-7-(2-(1-pyrrolidinyl)-2-oxoethyl)-1-quinolinyl)methyl)-(1,1'-biphenyl)-2-carboxylic acid.
. 1,4-dihydro-4-oxo-1((2'-(1H-tetrazol-5-yl)(1,1'-biphenyl)-4-yl)methyl-quinoline-3,8-dicarboxylic acid.
. 3-butyl-1-((2'-(1H-tetrazol-5-yl)-1,1'-biphenyl)-4-yl)methyl)-6,7,8-trifluoro-4(1H)-quinolinone.
. 3-butyl-6,8-difluoro-7-(1-pyrrolidinyl)-1-((2'-(1H-tetrazol-5-yl)-(1,1'-biphenyl)-4-yl)methyl)-4-(1H)-quinolinone
these products being in all possible racemic, enantiomeric and diastereoisomeric isomer forms, as well as the addition salts with mineral and organic acids or with mineral and organic bases, of these products.

3. The products according to claim 1 corresponding to the following definitions:
. 4'-[(3-butyl 1,4-dihydro-4-oxo-1-quinolinyl) methyl] (1,1'-biphenyl)-2-carboxylic acid,
. 4'-[(3-butyl 1,4-dihydro-4-thioxo-1-quinolinyl) methyl] (1,1'-biphenyl) 2-carboxylic acid,
. 3-butyl 1-[(2'-carboxy (1,1'-biphenyl) 4-yl) methyl] 1,4-dihydro-4-oxo-7-quinolinecarboxylic acid,
. 4'-[(3-butyl 1,4-dihydro 7-(hydroxymethyl) 4-oxo 1-quinolinyl) methyl] (1,1'-biphenyl) 2-carboxylic acid,
. 4'-[(3-butyl 1,4-dihydro 4-oxo 7-[(1-pyrrolidinyl) carbonyl]-1-quinolinyl) methyl] (1,1'-biphenyl) 2-carboxylic acid,
. 4'-[(3-butyl 1,4,5,6,7,8-hexahydro 4-oxo 1-[(1-quinolinyl) methyl] (1,1'-biphenyl) 2-carboxylic acid,
. 4'-[(3-butyl 1,4,5,6,7,8-hexahydro 6-methyl 4-oxo 1-quinolinyl) methyl] (1,1'-biphenyl) 2-carboxylic acid,
. 3-butyl 1,4-dihydro 1-[[2'-(1H-tetrazol-5-yl) (1,1'-biphenyl) 4-yl] methyl]4-oxo 8-quinolinecarboxylic acid,
. 1,4-dihydro 3-methyl 4-oxo 1-[[2'-(1H-tetrazol-5-yl) (1,1'-biphenyl) 4-yl] methyl] 8-quinolinecarboxylic acid,
. 1,4-dihydro 3-ethyl 4-oxo 1-[[2'-(1H-tetrazol-5-yl) (1,1'-biphenyl) 4-yl] methyl] 8-quinolinecarboxylic acid.

4. Preparation process for the products of formula (I) as defined in claim 1 and the products as defined in claim 2, characterized in that:
either a product of formula (II): 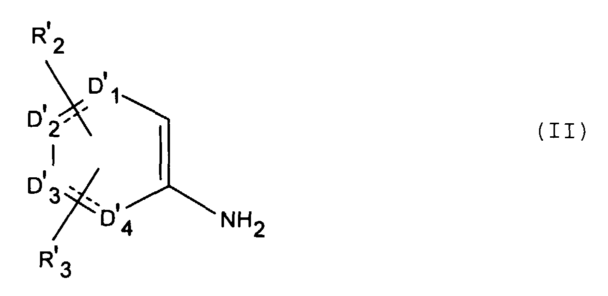 in which D'₁, D'₂, D'₃, D'₄, R'₂ and R'₃ have the meanings indicated in claim 1, respectively for D₁, D₂, D₃, D₄, R₂ and R₃ or corresponding to the products defined in claim 3 in which the optional reactive functions are optionally protected by protective groups, is reacted with a product of formula (III): 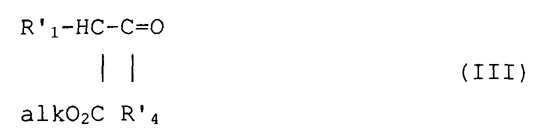 in which R'₁ has the meaning indicated in claim 1 for R₁ and R_{'4} represents a hydrogen atom or the carboxy radical, in which the optional reactive functions are optionally protected by protective groups and alk represents an alkyl radical containing at most 6 carbon atoms, in order to obtain the products of formula (IV): 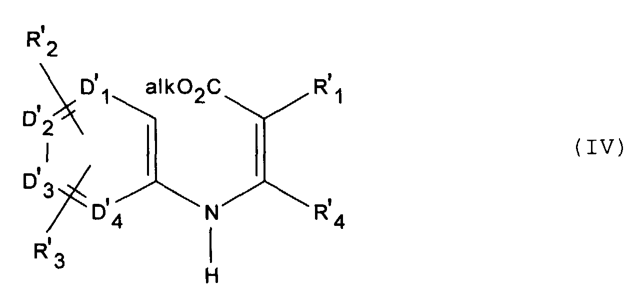 in which D'₁, D'₂, D'₃, D'₄, R'₁, R'₂, R'₃, R'₄ and alk have the meanings indicated above, which are cyclized in order to obtain the products of formula (V): 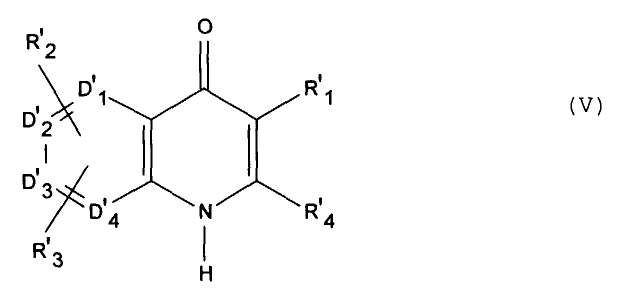 in which D'₁, D'₂, D'₃, D'₄, R'₁, R'₂, R'₃ and R'₄ have the meanings indicated above,
or a product of formula (VI): 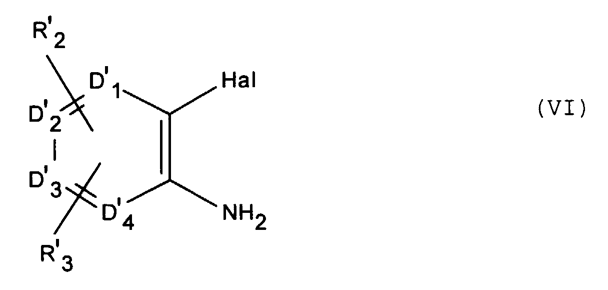 in which D'₁, D'₂, D'₃, D'₄, R'₂ and R'₃ have the meanings indicated above and Hal represents a halogen atom, is reacted with a product of formula (VII):
R'₁-C≡CH (VII)
in which R'₁ has the meaning indicated above, in order to obtain the products of formula (VIII): 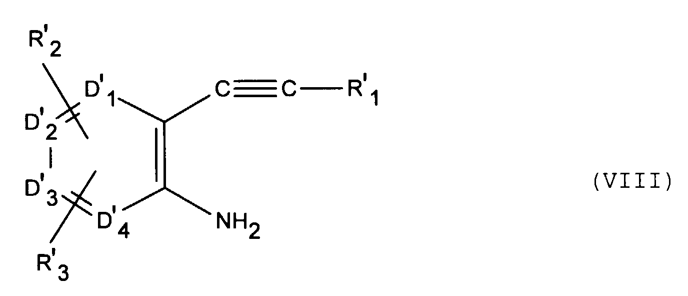 in which D'₁, D'₂, D'₃, D'₄, R'₁, R'₂ and R'₃ have the meanings indicated above, which is subjected to a cyclisation reaction in order to obtain a product of formula (IX): 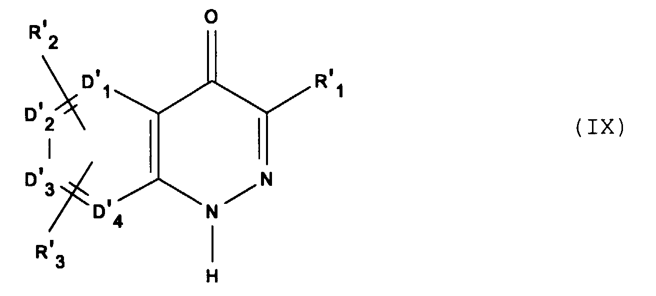 in which D'₁, D'₂, D'₃, D'₄, R'₁, R'₂ and R'₃ have the meanings indicated above, which products of formulae (V) and (IX), if desired, are subjected to a hydrogenation reaction in order to obtain respectively the products of formula (V_{B}): 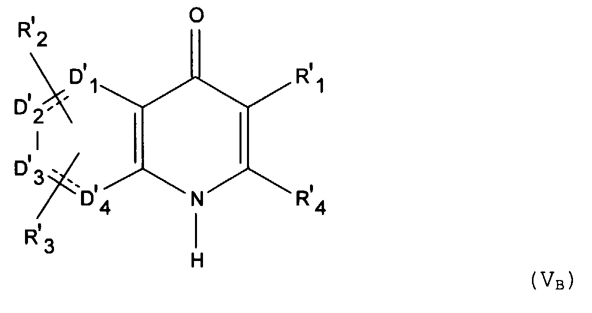 and the products of formula (IX_{B}) : 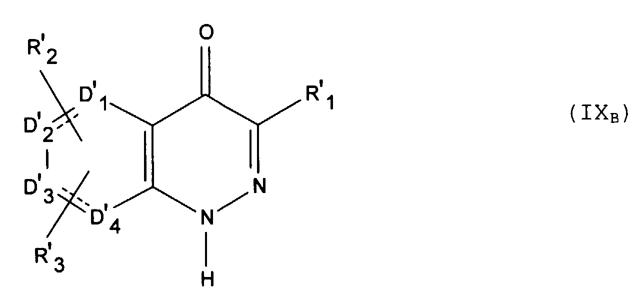 the products of formulae (V_{B}) and (IX_{B}) in which D'₁, D'₂, D'₃, D'₄, R'₁, R'₂, R'₃ and R'₄ have the meanings indicated above, and products of formulae (V), (VB), (IX) and (IX) which if desired, are subjected to a reaction which converts the oxo function into a thioxo function and which are reacted with a compound of formula (X):
Hal-CH₂-Y' (X)
in which Hal represents a halogen atom and Y' has the meaning indicated above for Y in which the optional reactive functions are optionally protected by protective groups, in order to obtain a product of formula (I'): 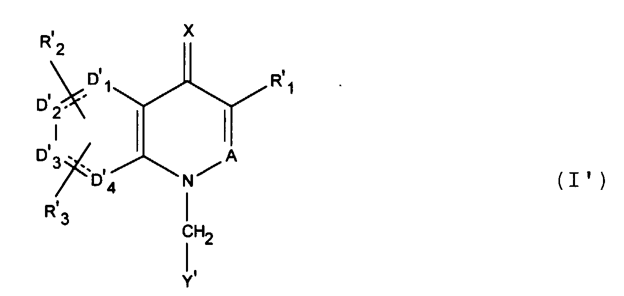 in which: 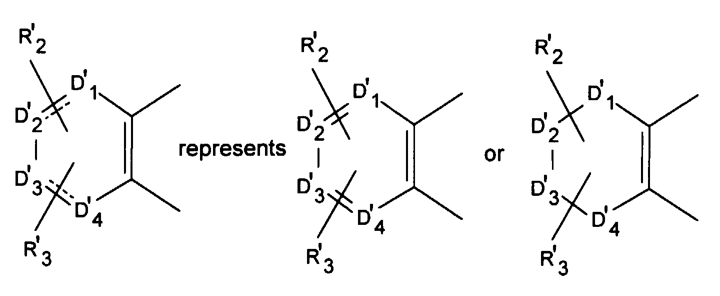 D'₁, D'₂, D'₃, D'₄, R'₁, R'₂, R'₃, R'₄, R'₅ and Y' have the meaning indicated above, A represents the C-R'₄ radical or a nitrogen atom, and X has the meaning indicated above, products of formula (I') in which 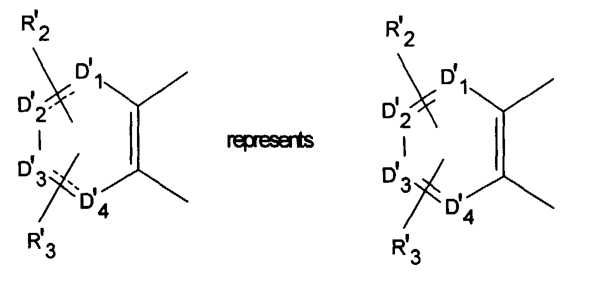 which can, if desired, be subjected to a hydrogenation reaction in order to obtain the products of formula (I') in which 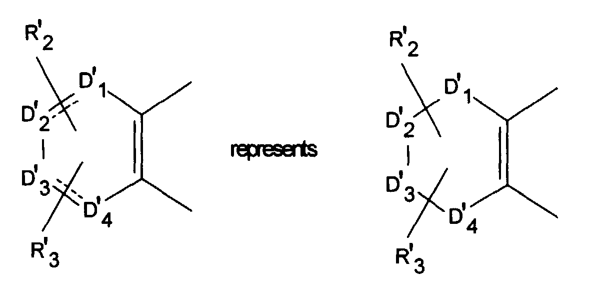 which product of formula (I') is subjected, if desired and if necessary, to one or more of the following reactions, in any order:
- a reaction which converts the oxo C=O function into a thioxo C=S function,
- a reaction which eliminates the protective groups which can be carried by the protected reactive functions,
- a salification reaction by a mineral or organic acid or by a mineral or organic base in order to obtain the corresponding salt,
- an esterification reaction of the acid function,
- a saponification reaction of the ester function into an acid function,
- a conversion reaction of the alkyloxy function into a hydroxyl function,
- a conversion reaction of the cyano function into an acid function,
- a reduction reaction of the carboxy function into an alcohol function,
- a resolution reaction of the racemic forms,
the products thus obtained being in all possible racemic, enantiomeric and diastereoisomeric isomer forms.

5. Preparation process for the products of formula (I), as defined in claim 1 and the products as defined in claim 2, in which A represents CH characterized in that:
either a product of formula (II_{E}): 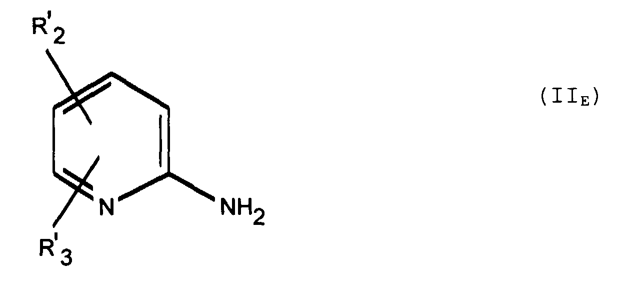 in which R'₂ and R'₃ have the meanings indicated in claim 1, respectively for R₂ and R3, or corresponding to the products as defined in claim 2, in which the optional reactive functions are optionally protected by protective groups, is reacted with a product of formula (XI): 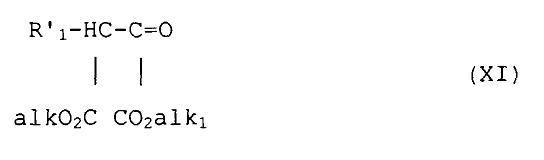 in which R'₁ has the meaning indicated in claim 1 for R₁ in which the optional reactive functions are optionally protected by protective groups and alk and alk₁, identical or different, represent an alkyl radical containing at most 6 carbon atoms, in order to obtain the products of formula (XII): 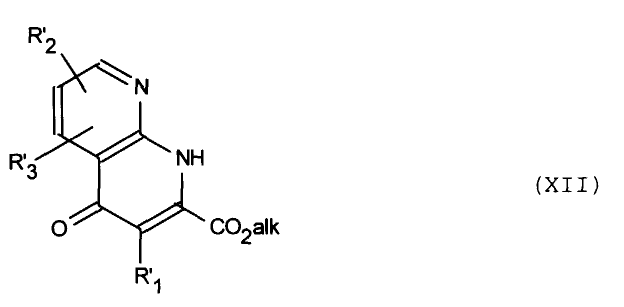 in which R'₁, R'₂, R'₃ and alk have the meanings indicated above,
or a product of formula (II_{F}): 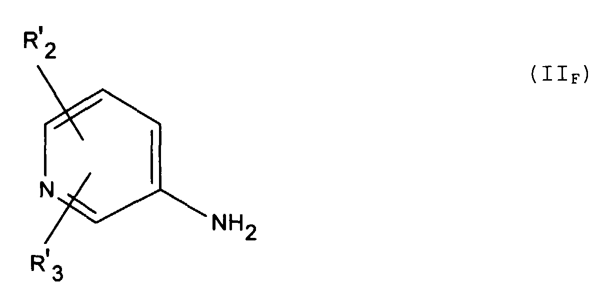 in which R'₂ and R'3 have the meanings indicated above, is reacted with the product of formula (XI) as defined above, in order to obtain the products of formula (XIII): 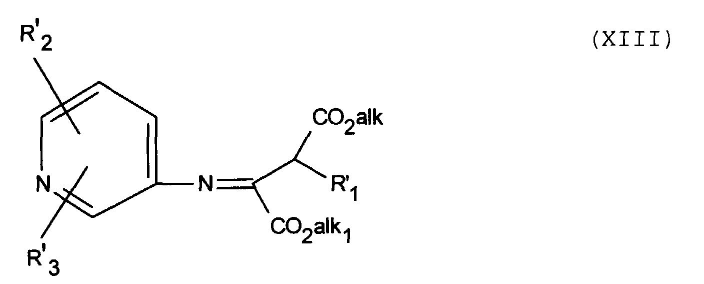 in which R'1, R'₂', R'₃, alk and alk₁ have the meanings indicated above, which products of formulae (XII) and (XIII) are converted into the product of formula (XIV): 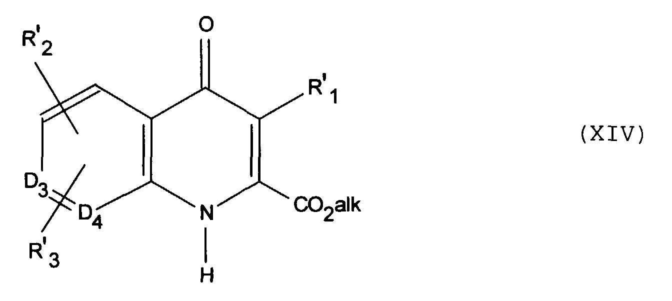 in which R'₁, R'₂, R'₃ and alk₁ have the meanings indicated above and D₃ and D₄ have the meanings indicated in claim 1, which products of formula (XIV), if desired, are subjected to a hydrogenation reaction in order to obtain the products of formula (XV): 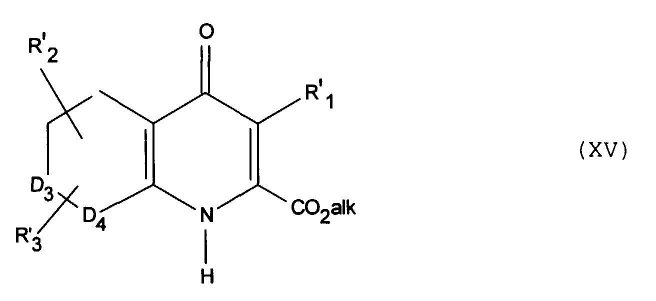 in which D₃, D₄,, R'₁, R'₂ and R'₃ and alk₁ have the meanings indicated above, which products of formulae (XIV) and (XV) are subjected to a saponification reaction then to a decarboxylation reaction, in order to obtain the corresponding products of formula (XIV'): 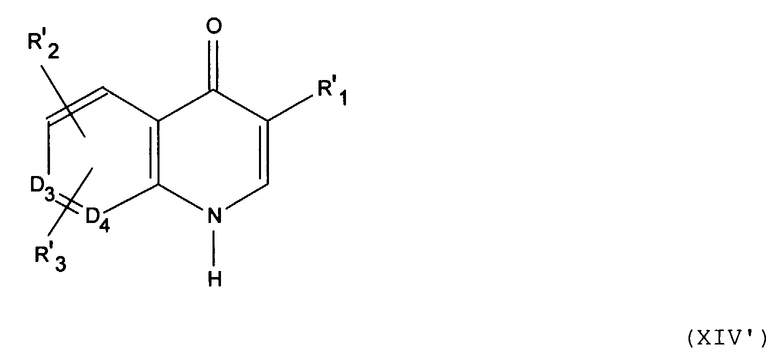 and of formula (XV'): 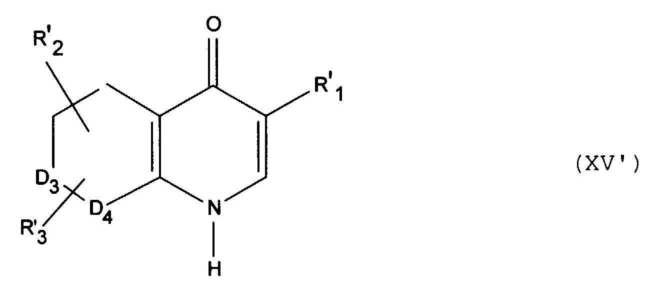 which products of formulae (XIV') and (XV') if desired, are subjected to a conversion reaction of the oxo function into a thioxo function and which is reacted with a compound of formula (X):
Hal-CH2-Y' (X)
in which Y' has the meaning indicated in claim 1 for Y in which the optional reactive functions are optionally protected by protective groups, in order to obtain a product of formula (I'_{EB}): 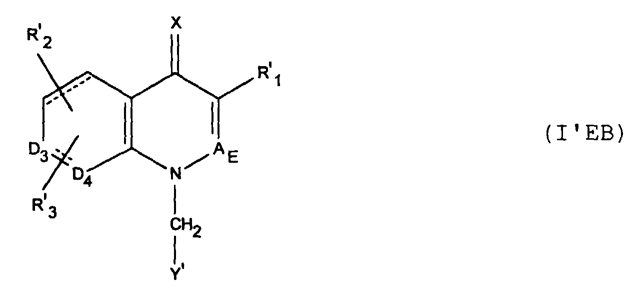 in which: 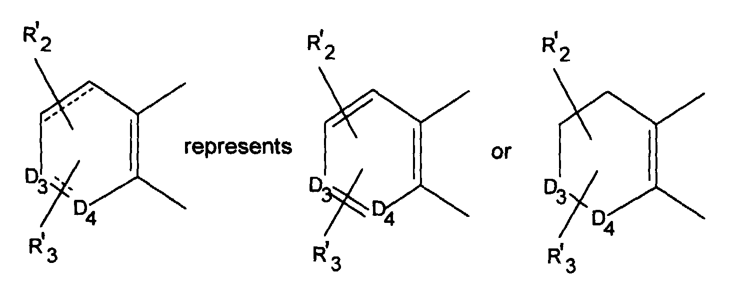 D₃, D₄, R'₁, R'₂, R'₃ and Y' have the meanings indicated above, A_{E} represent the CH radical and X has the meaning indicated above, products of formula (I'_{EB}) in which: 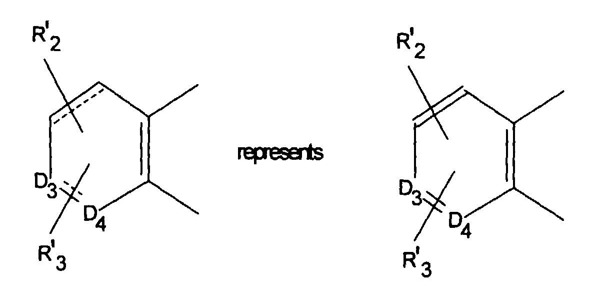 which can, if desired, be subjected to a hydrogenation reaction in order to obtain the products of formula (I'_{EB}) in which 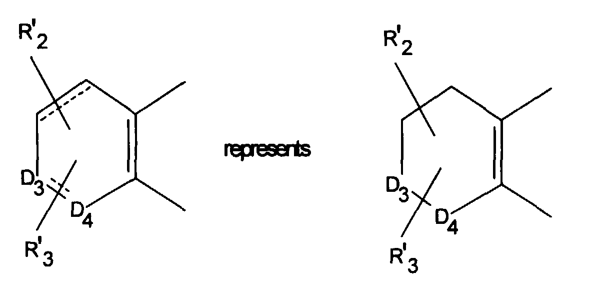 which product of formula (I'_{EB}) can be subjected, if desired and if necessary, to one or more of the following reactions, in any order:
- a reaction which converts the oxo C=O function into a thioxo C=S function,
- a reaction which eliminates the protective groups which can be carried by the protected reactive functions,
- a salification reaction by a mineral or organic acid or by a mineral or organic base in order to obtain the corresponding salt,
- an esterification reaction of the acid function,
- a saponification reaction of the ester function into an acid function,
- a conversion reaction of the alkyloxy function into a hydroxyl function,
- a conversion reaction of the cyano function into an acid function,
- a reduction reaction of the carboxy function into an alcohol function,
- a resolution reaction of the racemic forms,
the products thus obtained being in all possible racemic, enantiomeric and diastereoisomeric isomer forms.

6. As medicaments, the products defined in claims 1 to 3, these products being in all possible racemic, enantiomeric and diastereoisomeric isomer forms, as well as the addition salts with pharmaceutically acceptable mineral and organic acids or mineral and organic bases of these products.

7. As medicaments, the products defined in claim 2, as well as the addition salts with pharmaceutically acceptable mineral and organic acids or mineral and organic bases of these products.

8. The pharmaceutical compositions containing at least one of the medicaments defined in any one of claims 6 and 7 as active ingredient.

9. Use of the products defined in claims 1 to 3, these products being in all possible racemic, enantiomeric and diastereoisomeric isomer forms, as well as the addition salts with pharmaceutically acceptable mineral and organic acids or mineral and organic bases of these products,
for the preparation of medicaments, intended:
either for the treatment of arterial hypertension, cardiac insufficiencies, glaucoma, renal insufficiencies and in the prevention of post-angioplasty recurrence of stenosis,
or for the treatment of certain gastro-intestinal or gynecological disorders.
or for the treatment of memory disorders and disorders of the cognitive functions, anxiety, depression, senile dementia and Alzheimer's disease.

## Claims (Claims for the following Contracting State(s): ES, GR)

1. Preparation process for the products of formula (I): 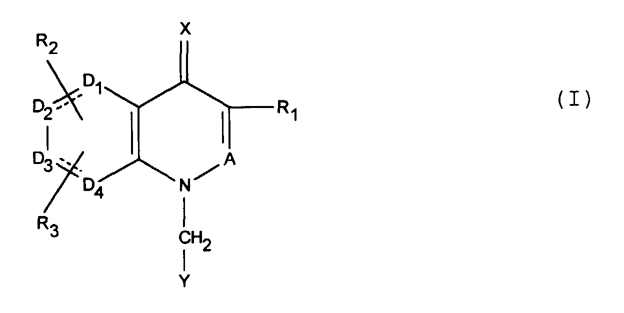 in which: 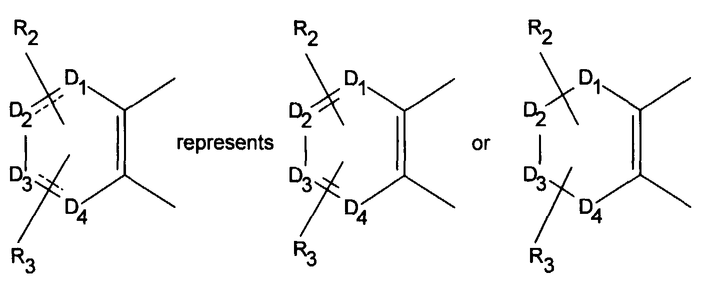
with D₁, D₂ both representing a methine radical or a methylene, radical, optionally substituted by a radical chosen from R₂ and R₃,
D₃ and D₄, identical or different, represent a nitrogen atom, a methine radical or a methylene radical, optionally substituted by a radical chosen from R₂ and R₃,
at least one of D₃ and D₄ represents a methine or methylene radical, optionally substituted by a radical chosen from R₂ and R₃,
R₁ represents a methyl, ethyl, n-propyl, n-propenyl, n-butyl radical,
R₂ and R₃, identical or different, are chosen from the group formed by:
- the hydrogen atom,
- the mercapto and alkylthio radicals,
- linear or branched alkyl radicals containing at most 6 carbon atoms and optionally substituted by one or more identical or different substituents chosen from halogen atoms, the hydroxyl radical, the amino, mono and dialkylamino, pyrrolidinyl, morpholinyl, piperidinyl radicals,
- the carboxy radical free, salified or esterified by a linear or branched alkyl radical containing at most 4 carbon atoms,
- the pyrrolidinyl-carbonyl, morpholinyl-carbonyl, carbamoyl, dialkylcarbamoyl, piperidynyl-carbonyl radicals,
A represents a nitrogen atom or a CH radical,
X represents an oxygen atom or a sulphur atom,
Y represents a phenyl or biphenyl radical optionally substituted by one or more radicals chosen from the cyano, free, salified and esterified carboxy, tetrazole and isoxazole radicals,
with the exception of the products of formula (I) in which 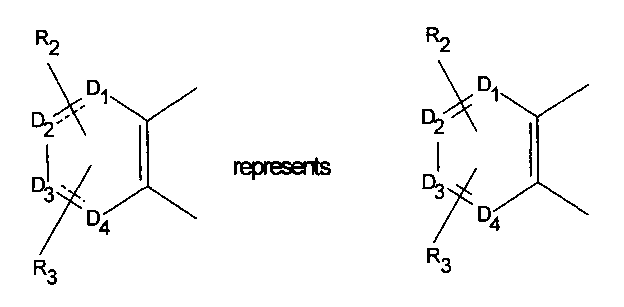
with D₁, D₂, D₃ and D₄ each representing a methine radical X represents an oxygen atom and A represents a CH radical, R₁, R₂ and R₃, being identical all three represent a methyl radical, and -Y represents a phenyl radical,
the said products of formula (I) being in all possible racemic, enantiomeric and diastereoisomeric isomer forms, as well as the addition salts with mineral and organic acids or with mineral and organic bases, of said products of formula (I) characterized in that:
either a product of formula (II): 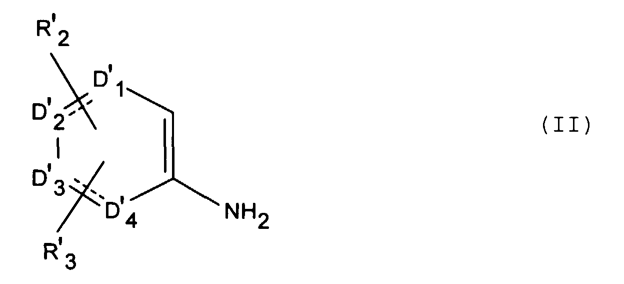 in which D'₁, D'₂, D'₃, D'₄, R'₂ and R'₃ have the meanings indicated above, respectively for D₁, D₂, D₃, D₄, R₂ and R₃ in which the optional reactive functions are optionally protected by protective groups, is reacted with a product of formula (III): 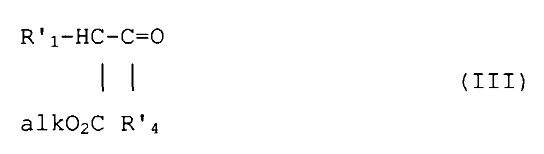 in which R'₁ has the meaning indicated above for R₁ and R_{'4} represents a hydrogen atom or the carboxy radical, in which the optional reactive functions are optionally protected by protective groups and alk represents an alkyl radical containing at most 6 carbon atoms, in order to obtain the products of formula (IV): 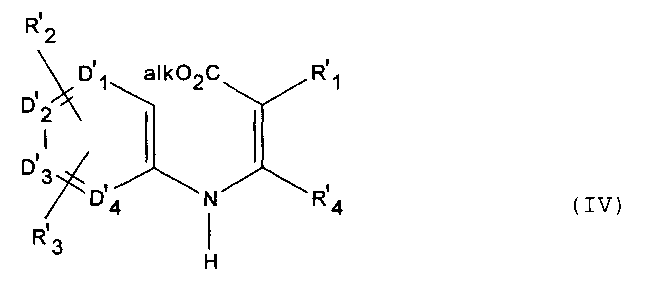 in which D'₁, D'₂, D'₃, D'₄, R'₁, R'₂, R'₃, R'₄ and alk have the meanings indicated above, which are cyclized in order to obtain the products of formula (V): 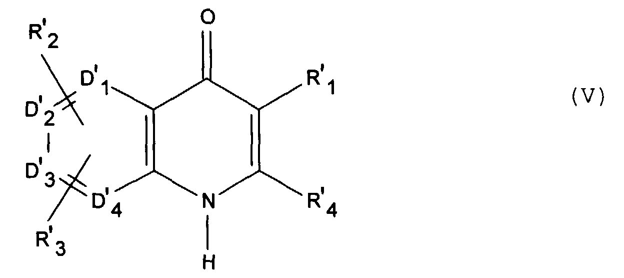 in which D'₁, D'₂, D'₃, D'₄, R'₁, R'₂, R'₃ and R'₄ have the meanings indicated above,
or a product of formula (VI): 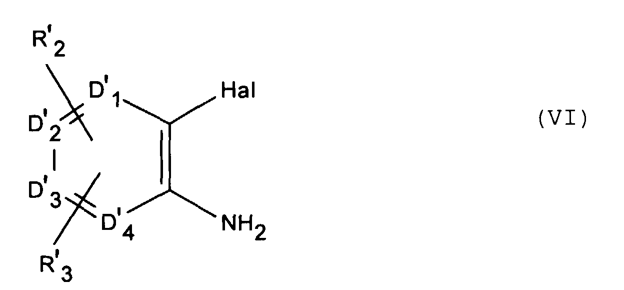 in which D'₁, D'₂, D'₃, D'₄, R'₂ and R'₃ have the meanings indicated above and Hal represents a halogen atom, is reacted with a product of formula (VII):
R'₁-C≡CH (VII)
in which R'₁ has the meaning indicated above, in order to obtain the products of formula (VIII): 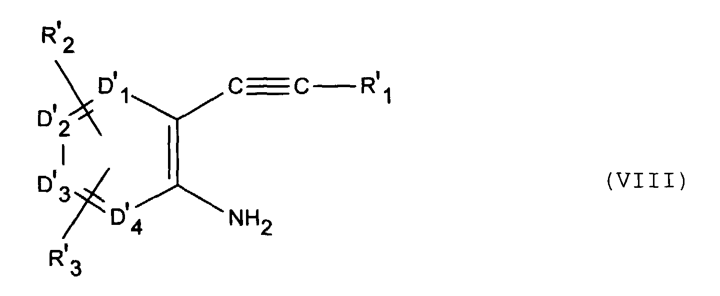 in which D'₁, D'₂, D'₃, D'₄, R'₁, R'₂ and R'₃ have the meanings indicated above, which is subjected to a cyclisation reaction in order to obtain a product of formula (IX): 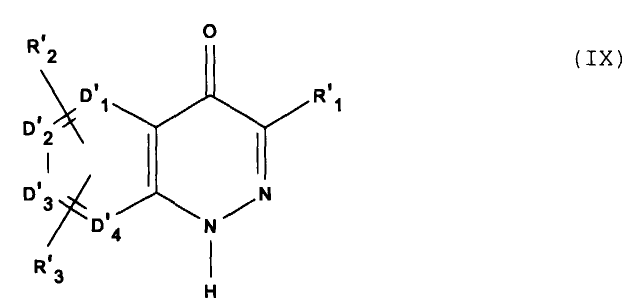 in which D'₁, D'₂, D'₃, D'₄, R'₁, R'₂ and R'₃ have the meanings indicated above, which products of formulae (V) and (IX), if desired, are subjected to a hydrogenation reaction in order to obtain respectively the products of formula (V_{B}): 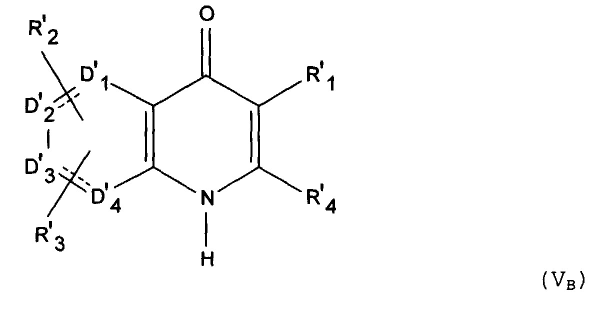 and the products of formula (IX_{B}) : 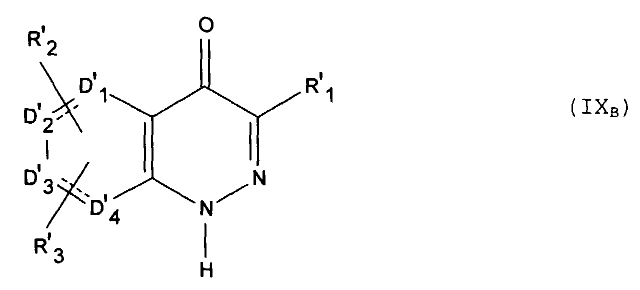 the products of formulae (V_{B}) and (IX_{B}) in which D'₁, D'₂, D'₃, D'₄, R'₁, R'₂, R'₃ and R'₄ have the meanings indicated above, and products of formulae (V), (VB), (IX) and (IX) which if desired, are subjected to a reaction which converts the oxo function into a thioxo function thioxo and which are reacted with a compound of formula (X):
Hal-CH₅-Y' (X)
in which Hal represents a halogen atom and Y' has the meaning indicated above, for Y in which the optional reactive functions are optionally protected by protective groups, in order to obtain a product of formula (I'): 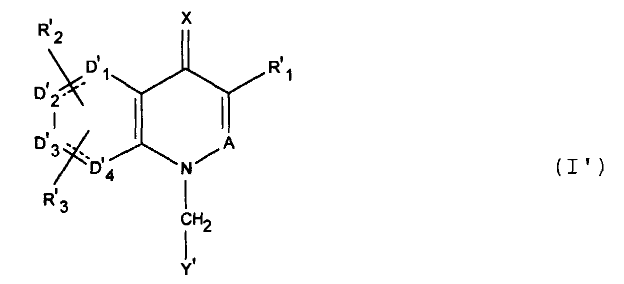 in which: 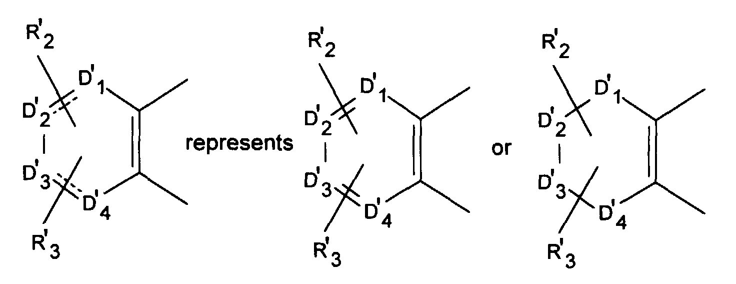 D'₁, D'₂, D'₃, D'₄, R'₁, R'₂, R'₃, R'₄, R'₅ and Y' have the meaning indicated above, A represents the C-R'₄ radical or a nitrogen atom, and X has the meaning indicated above, products of formula (I') in which 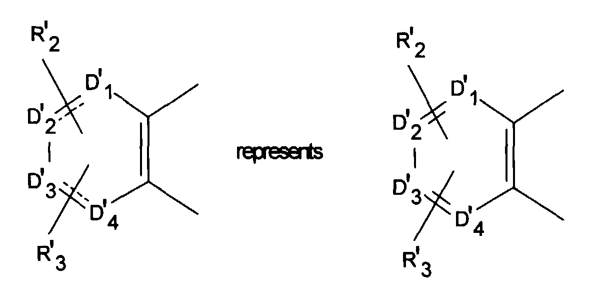 which can, if desired, be subjected to a hydrogenation reaction in order to obtain the products of formula (I') in which 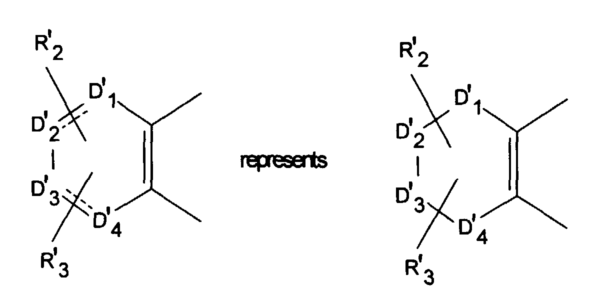 which product of formula (I') is subjected, if desired and if necessary, to one or more of the following reactions, in any order:
- a reaction which converts the oxo C=O function into a thioxo C=S function,
- a reaction which eliminates the protective groups which can be carried by the protected reactive functions,
- a salification reaction by a mineral or organic acid or by a mineral or organic base in order to obtain the corresponding salt,
- an esterification reaction of the acid function,
- a saponification reaction of the ester function into an acid function,
- a conversion reaction of the alkyloxy function into a hydroxyl function,
- a conversion reaction of the cyano function into an acid function,
- a reduction reaction of the carboxy function into an alcohol function,
- a resolution reaction of the racemic forms,
the products thus obtained being in all possible racemic, enantiomeric and diastereoisomeric isomer forms.

2. Process as defined in claim 1 for the preparation of the following products :
. 4'-((3-butyl-1,4-dihydro-7-formyl-4-oxo-1-quinolinyl)methyl)-(1,1'-biphenyl)-2-carboxylic acid.
. 3-butyl-1-(2'-carboxy-(1,1'-biphenyl)-4-yl)methyl)-1,4-dihydro-4-oxo-7-quinolinepropanoic acid.
. 4'-((3-butyl-6-(2-carboxyethenyl)-1,4-dihydro-4-oxo-1-quinolinyl) methyl)-(1,1'-biphenyl)-2-carboxylic acid.
. 4'-((3-butyl-1,4-dihydro-6-formyl-4-oxo-1-quinolinyl)methyl)-(1,1'-biphenyl)-2-carboxylic acid.
. 4'-(3-butyl-1,4-dihydro-7-(3-hydroxy-3-oxo-1-propenyl)-4-oxo-1-quinolinyl)methyl)-(1,1'-biphenyl)-2-carboxylic acid.
. 3-butyl-1-((2'-carboxy-(1,1'-biphenyl)-4-yl)methyl)-1,4-dihydro-4-oxo-7-quinolineacetic acid.
. 4'-((3-butyl-1,4-dihydro-4-oxo-7-(2-(1-pyrrolidinyl)-2-oxoethyl)-1-quinolinyl)methyl)-(1,1'-biphenyl)-2-carboxylic acid.
. 1,4-dihydro-4-oxo-1((2'-(1H-tetrazol-5-yl)(1,1'-biphenyl)-4-yl)methyl-quinoline-3,8-dicarboxylic acid.
. 3-butyl-1-((2'-(1H-tetrazol-5-yl)-1,1'-biphenyl)-4-yl)methyl)-6,7,8-trifluoro-4(1H)-quinolinone.
. 3-butyl-6,8-difluoro-7-(1-pyrrolidinyl)-1-((2'-(1H-tetrazol-5-yl)-(1,1'-biphenyl)-4-yl)methyl)-4-(1H)-quinolinone
these products being in all possible racemic, enantiomeric and diastereoisomeric isomer forms, as well as the addition salts with mineral and organic acids or with mineral and organic bases of these products.

3. Preparation process for the products of formula (I) : 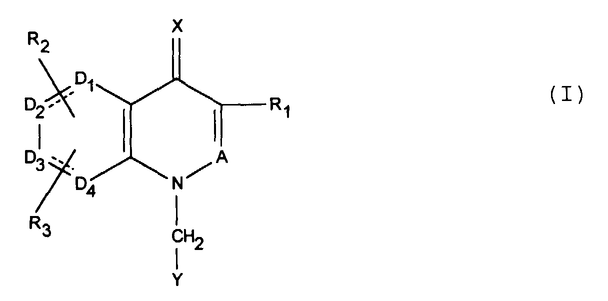 in which: 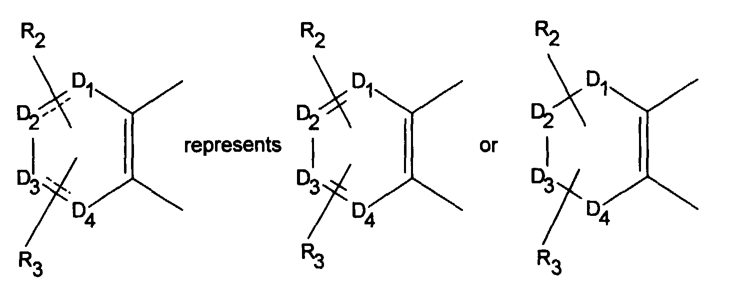
with D₁, D₂ both representing a methine radical or a methylene radical optionally substituted by a radical chosen from R₂ and R₃,
D₃ and D₄, identical or different, represent a nitrogen atom, a methine radical or a methylene radical, optionally substituted by a radical chosen from R₂ and R₃,
at least one of D₃ and D₄ represents a methine or methylene radical, optionally substituted by a radical chosen from R₂ and R₃,
R₁ represents a methyl, ethyl, n-propyl, n-propenyl, n-butyl radical,
R₂ and R₃, identical or different, are chosen from the group formed by:
- the hydrogen atom,
- the mercapto and alkylthio radicals,
- linear or branched alkyl radicals containing at most 6 carbon atoms and optionally substituted by one or more identical or different substituents chosen from halogen atoms, the hydroxyl radical, the amino, mono and dialkylamino, pyrrolidinyl, morpholinyl, piperidinyl radicals,
- the carboxy radical free, salified or esterified by a linear or branched alkyl radical containing at most 4 carbon atoms,
- the pyrrolidinyl-carbonyl, morpholinyl-carbonyl, carbamoyl, dialkylcarbamoyl, piperidynyl-carbonyl radicals,
A represents a nitrogen atom or a CH radical,
X represents an oxygen atom or a sulphur atom,
Y represents a phenyl or biphenyl radical optionally substituted by one or more radicals chosen from the cyano, free, salified and esterified carboxy, tetrazole and isoxazole radicals,
with the exception of the products of formula (I) in which 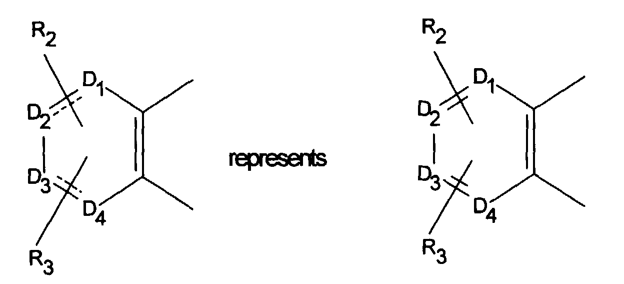
with D₁, D₂, D₃ and D₄ each representing a methine radical X represents an oxygen atom and A represents a CH radical, R₁, R₂ and R₃, being identical all three represent a methyl radical, and -Y represents a phenyl radical,
the said products of formula I being in all possible racemic, enantiomeric and diastereoisomeric isomer forms, as well as the addition salts with mineral and organic acids or with mineral and organic bases of said products of formula (I) characterized in that:
either a product of formula (II_{E}): 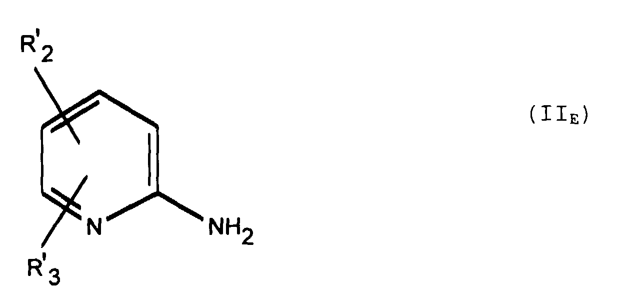 in which R'₂ and R'₃ have the meanings indicated above, respectively for R₂ and R3, in which the optional reactive functions are optionally protected by protective groups, is reacted with a product of formula (XI): 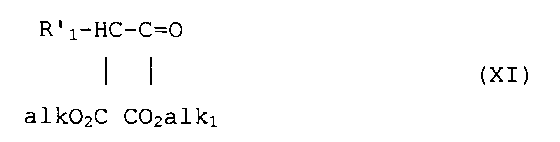 in which R'₁ has the meaning indicated in claim 1 for R₁ in which the optional reactive functions are optionally protected by protective groups and alk and alk₁, identical or different, represent an alkyl radical containing at most 6 carbon atoms, in order to obtain the products of formula (XII): 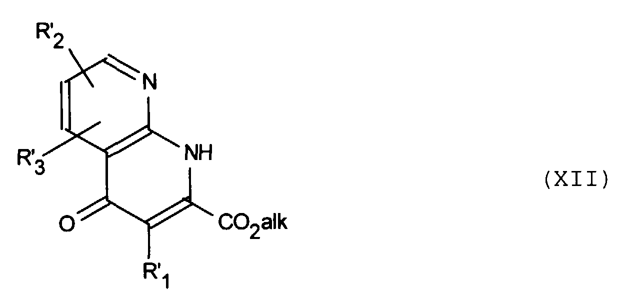 in which R'₁, R'₂, R'₃ and alk have the meanings indicated above,
or a product of formula (II_{F}): 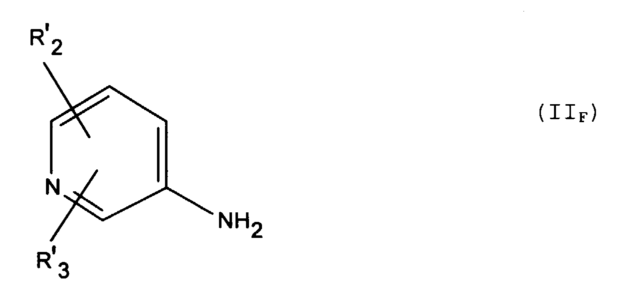 in which R'₂ and R'3 have the meanings indicated above, is reacted with the product of formula (XI) as defined above, in order to obtain the products of formula (XIII): 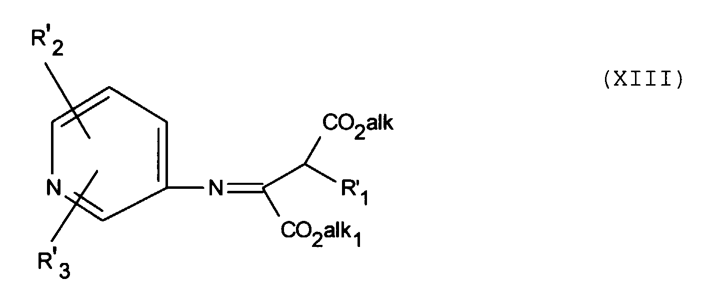 in which R'1, R'₂', R'₃, alk and alki have the meanings indicated above, which products of formulae (XII) and (XIII) are converted into the product of formula (XIV): 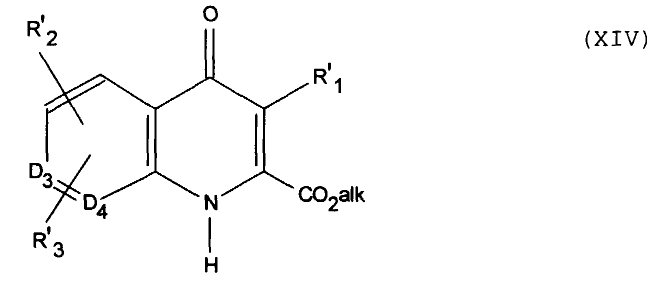 in which R'₁, R'₂, R'₃ and alk₁ have the meanings indicated above and D₃ and D₄ have the meanings indicated in claim 1, which products of formula (XIV), if desired, are subjected to a hydrogenation reaction in order to obtain the products of formula (XV): 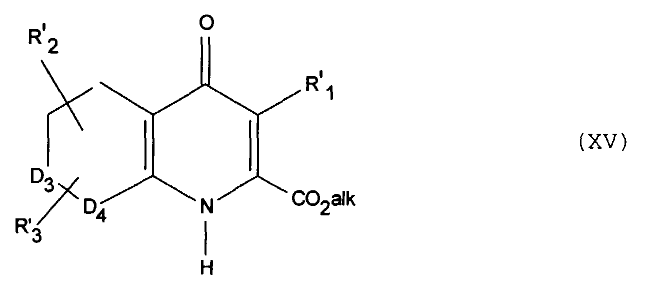 in which D₃, D₄, R'₁, R'₂ and R'₃ and alk₁ have the meanings indicated above, which products of formulae (XIV) and (XV) are subjected to a saponification reaction then to a decarboxylation reaction, in order to obtain the corresponding products of formula (XIV'): 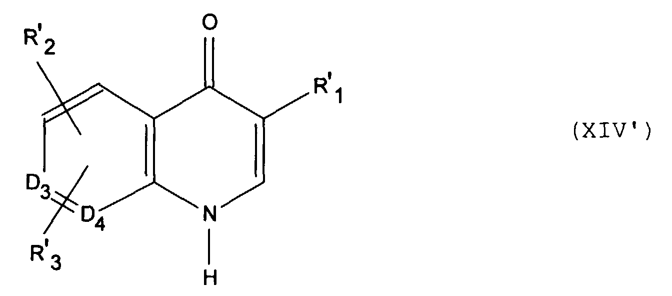 and of formula (XV'): 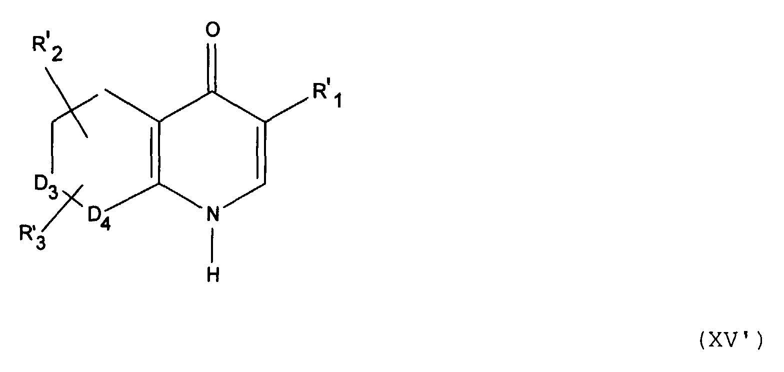 which products of formulae (XIV') and (XV') if desired, are subjected to a conversion reaction of the oxo function into a thioxo function and which is reacted with a compound of formula (X):
Hal-CH2-Y' (X)
in which Y' has the meaning indicated in claim 1 for Y in which the optional reactive functions are optionally protected by protective groups, in order to obtain a product of formula (I'_{EB}): 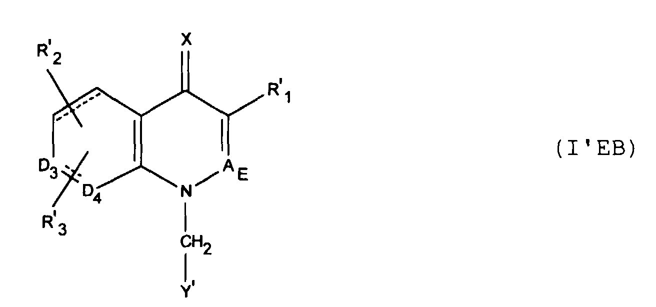 in which: 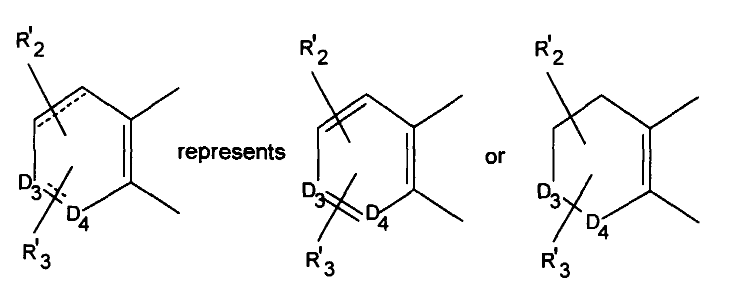 D₃, D₄, R'₁, R'₂, R'₃ and Y' have the meanings indicated above, A_{E} represents the CH radical and X has the meaning indicated above, products of formula (I'_{EB}) in which: 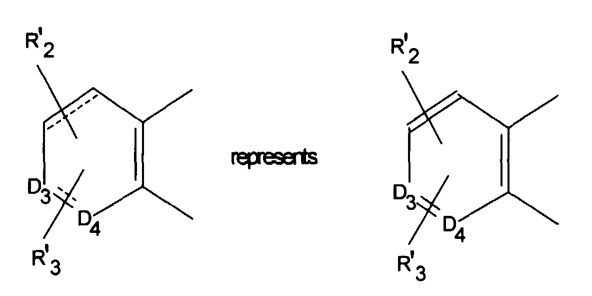 which can, if desired, be subjected to a hydrogenation reaction in order to obtain the products of formula (I'_{EB}) in which 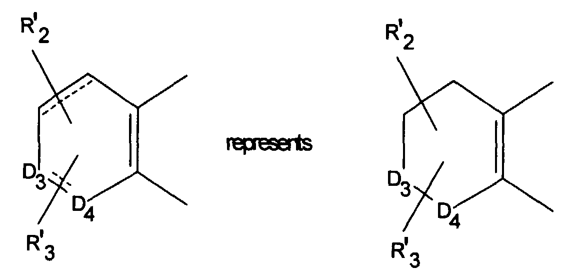 which product of formula (I'_{EB}) can be subjected, if desired and if necessary, to one or more of the following reactions, in any order:
- a reaction which converts the oxo C=O function into a thioxo C=S function,
- a reaction which eliminates the protective groups which can be carried by the protected reactive functions,
- a salification reaction by a mineral or organic acid or by a mineral or organic base in order to obtain the corresponding salt,
- an esterification reaction of the acid function,
- a saponification reaction of the ester function into an acid function,
- a conversion reaction of the alkyloxy function into a hydroxyl function,
- a conversion reaction of the cyano function into an acid function,
- a reduction reaction of the carboxy function into an alcohol function,
- a resolution reaction of the racemic forms,
the products thus obtained being in all possible racemic, enantiomeric and diastereoisomeric isomer forms.

4. Process as defined in claim 3 for the preparation of the following products :
. 4'-((3-butyl-1,4-dihydro-7-formyl-4-oxo-1-quinolinyl)methyl)-(1,1'-biphenyl)-2-carboxylic acid.
. 3-butyl-1-(2'-carboxy-(1,1'-biphenyl)-4-yl)methyl)-1,4-dihydro-4-oxo-7-quinolinepropanoic acid.
. 4'-((3-butyl-6-(2-carboxyethenyl)-1,4-dihydro-4-oxo-1-quinolinyl) methyl)-(1,1'-biphenyl)-2-carboxylic acid.
. 4'-((3-butyl-1,4-dihydro-6-formyl-4-oxo-1-quinolinyl)methyl)-(1,1'-biphenyl)-2-carboxylic acid.
. 4'-(3-butyl-1,4-dihydro-7-(3-hydroxy-3-oxo-1-propenyl)-4-oxo-1-quinolinyl)methyl)-(1,1'-biphenyl)-2-carboxylic acid.
. 3-butyl-1-((2'-carboxy-(1,1'-biphenyl)-4-yl)methyl)-1,4-dihydro-4-oxo-7-quinolineacetic acid.
. 4'-((3-butyl-1,4-dihydro-4-oxo-7-(2-(1-pyrrolidinyl)-2-oxoethyl)-1-quinolinyl)methyl)-(1,1'-biphenyl)-2-carboxylic acid.
. 1,4-dihydro-4-oxo-1((2'-(1H-tetrazol-5-yl)(1,1'-biphenyl)-4-yl)methyl-quinoline-3,8-dicarboxylic acid.
. 3-butyl-1-((2'-(1H-tetrazol-5-yl)-1,1'-biphenyl)-4-yl)methyl)-6,7,8-trifluoro-4(1H)-quinolinone.
. 3-butyl-6,8-difluoro-7-(1-pyrrolidinyl)-1-((2'-(1H-tetrazol-5-yl)-(1,1'-biphenyl)-4-yl)methyl)-4-(1H)-quinolinone
These products being in all possible racemic, enantiomeric and diastereoisomeric isomer forms, as well as the addition salts with mineral and organic acids or with mineral and organic bases of these products, in which A represents CH.

5. Process according to any one of claims 1 to 4 in which the products correspond to the following definitions:
. 4'-[(3-butyl 1,4-dihydro-4-oxo-1-quinolinyl) methyl] (1,1'-biphenyl)-2-carboxylic acid,
. 4'-[(3-butyl 1,4-dihydro-4-thioxo-1-quinolinyl) methyl] (1,1'-biphenyl) 2-carboxylic acid,
. 3-butyl 1-[(2'-carboxy (1,1'-biphenyl) 4-yl) methyl] 1,4-dihydro-4-oxo-7-quinolinecarboxylic acid,
. 4'-[(3-butyl 1,4-dihydro 7-(hydroxymethyl) 4-oxo 1-quinolinyl) methyl] (1,1'-biphenyl) 2-carboxylic acid,
. 4'-[(3-butyl 1,4-dihydro 4-oxo 7-pyrrolidinyl) carbonyl] 1-quinolinyl) methyl] (1,1'-biphenyl) 2-carboxylic acid,
. 4'-[(3-butyl 1,4,5,6,7,8-hexahydro 4-oxo 1-[(1-quinolinyl) methyl] (1,1'-biphenyl) 2-carboxylic acid,
. 4'-[(3-butyl 1,4,5,6,7,8-hexahydro 6-methyl 4-oxo 1-quinolinyl) methyl] (1,1'-biphenyl) 2-carboxylic acid,
. 3-butyl 1,4-dihydro 1-[[2'-(1H-tetrazol-5-yl) (1,1'-biphenyl) 4-yl] methyl]4-oxo 8-quinolinecarboxylic acid,
. 1,4-dihydro 3-methyl 4-oxo 1-[[2'-(1H-tetrazol-5-yl) (1,1' biphenyl) 4-yl] methyl] 8-quinolinecarboxylic acid,
. 1,4-dihydro 3-ethyl 4-oxo 1-[[2'-(1H-tetrazol-5-yl) (1,1'-biphenyl) 4-yl] methyl] 8-quinolinecarboxylic acid.

6. Process according to any one of claims 1 to 5 in which the products are for the preparation of medicaments, intended:
either for the treatment of arterial hypertension, cardiac insufficiencies, glaucoma, renal insufficiencies and in the prevention of post-angioplasty recurrence of stenosis,
or for the treatment of certain gastro-intestinal or gynecological disorders.
or for the treatment of memory disorders and disorders of the cognitive functions, anxiety, depression, senile dementia and Alzheimer's disease.

## Patentansprüche (Patentansprüche für folgende(n) Vertragsstaat(en): AT, BE, CH, LI, DE, DK, FR, GB, IT, LU, MC, NL, PT, SE)

1. Produkte der Formel (I) 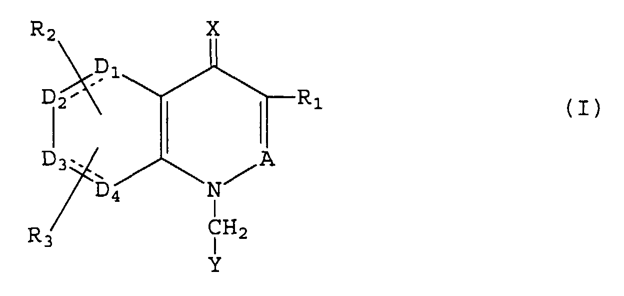 in der 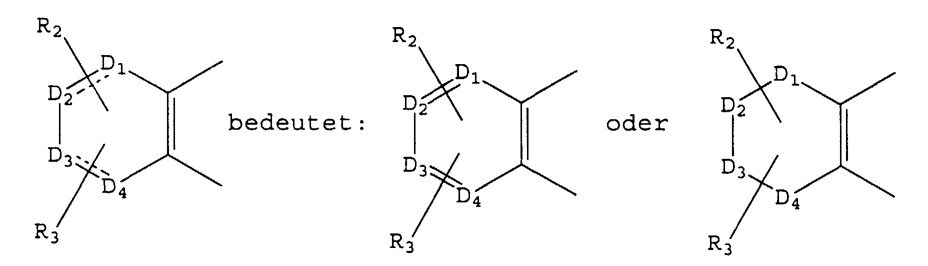
mit D₁, D₂, die beide einen Rest Methin oder einen Rest Methylen darstellen, gegebenenfalls substituiert durch einen unter R₂ und R₃ ausgewählten Rest,
D₃ und D₄, gleich oder verschieden, ein Stickstoffatom, einen Rest Methin oder einen Rest Methylen bedeuten, gegebenenfalls substituiert durch einen unter R₂ und R₃ ausgewählten Rest,
mindestens eines von D₃ und D₄ einen Rest Methin oder Methylen darstellt, gegebenenfalls substituiert durch einen unter R₂ und R₃ ausgewählten Rest,
R₁ einen Rest Methyl, Ethyl, n-Propyl, n-Propenyl, n-Butyl bedeutet,
R₂ und R₃, gleich oder verschieden, aus der Gruppe gewählt werden die gebildet wird durch:
- das Wasserstoffatom,
- die Reste Mercapto und Alkylthio,
- die linearen oder verzweigten Reste Alkyl mit höchstens 6 Kohlenstoffatomen und gegebenenfalls substituiert durch einen oder mehrere, gleiche oder verschiedene Substituenten, ausgewählt unter den Halogenatomen, dem Rest Hydroxyl, dem Rest Amino, Mono- und Dialkylamino, Pyrrolidinyl, Morpholinyl, Piperidinyl,
- den Rest Carboxy, frei, in Salz überführt oder verestert durch einen linearen oder verzweigten Rest Alkyl mit höchstens 4 Kohlenstoffatomen,
- den Rest Pyrrolidinyl-carbonyl, Morpholinyl-carbonyl, Carbamoyl, Dialkylcarbamoyl, Piperidinyl-carbonyl,
A ein Stickstoffatom oder einen Rest CH darstellt,
X ein Sauerstoffatom oder ein Schwefelatom bedeutet,
Y ein Rest Phenyl oder Biphenyl ist, gegebenenfalls substituiert durch einen oder mehrere Reste, ausgewählt unter den Resten Cyano, Carboxy, frei, in Salz überführt und verestert, Tetrazol und Isoxazol,
mit Ausnahme der Produkte der Formel (I), in der 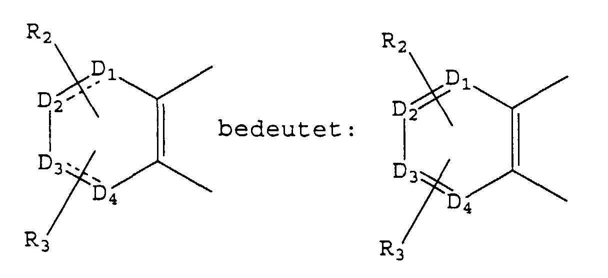
mit D₁, D₂, D₃ und D₄, die jeweils einen Rest Methin darstellen, X ein Sauerstoffatom ist und A einen Rest CH bedeutet, R₁, R₂ und R₃ gleich sind und alle drei einen Rest Methyl darstellen, und -Y ein Rest Phenyl ist,
die genannten Produkte der Formel (I) in allen ihren möglichen isomeren racemischen Formen, Enantiomeren und Diastereoisomeren, sowie die Additionssalze der genannten Produkte der Formel (I) mit Mineralsäuren und organischen Säuren oder mit Mineralbasen und organischen Basen.

2. Die folgenden Produkte:
. 4'-[(3-Butyl-1,4-dihydro-7-formyl-4-oxo-1-chinolinyl)-methyl]-(1,1'-biphenyl)-2-carbonsäure;
. 3-Butyl-1-{[2'-carboxy-(1,1'-biphenyl)-4-yl]-methyl}-1,4-dihydro-4-oxo-7-chinolin-propansäure;
. 4'-{[3-Butyl-6-(2-carboxyethenyl)-1,4-dihydro-4-oxo-1-chinolinyl]-methyl}-(1,1'-biphenyl)-2-carbonsäure;
. 4'-[(3-Butyl-1,4-dihydro-6-formyl-4-oxo-1-chinolinyl)-methyl]-(1,1'-biphenyl)-2-carbonsäure;
. 4'-{[3-Butyl-1,4-dihydro-7-(3-hydroxy-3-oxo-1-propenyl)-4-oxo-1-chinolinyl]-methyl}-(1,1'-biphenyl)-2-carbonsäure;
. 3-Butyl-1-{[2'-carboxy-(1,1'-biphenyl)-4-yl]-methyl}-1,4-dihydro-4-oxo-7-chinolin-essigsäure;
. 4'-{{3-Butyl-1,4-dihydro-4-oxo-7-[2-(1-pyrrolidinyl)-2-oxo-ethyl]-1-chinolinyl}-methyl}-(1,1'-biphenyl)-2-carbonsäure;
. 1,4-Dihydro-4-oxo-1-{[2'-(1H-tetrazol-5-yl)-(1,1'-biphenyl)-4-yl]-methyl}-chinolin-3,8-dicarbonsäure;
. 3-Butyl-1-{[2'-(1H-tetrazol-5-yl)-(1,1'-biphenyl)-4-yl]-methyl}-6,7,8-trifluor-4-(1H)-chinolinon;
. 3-Butyl-6,8-difluor-7-(1-pyrrolidinyl)-1-{[2'-(1H-tetrazol-5-yl)-(1,1'-biphenyl)-4-yl]-methyl}-4-(1H)-chinolinon;
diese Produkte in allen ihren möglichen isomeren racemischen Formen, Enantiomeren und Diastereoisomeren, sowie die Additionssalze dieser Produkte mit Mineralsäuren und organischen Säuren oder mit Mineralbasen und organischen Basen.

3. Die Produkte nach Anspruch 1, die den folgenden Definitionen entsprechen:
. 4'-[(3-Butyl-1,4-dihydro-4-oxo-1-chinolinyl)-methyl]-(1,1'-biphenyl)-2-carbonsäure;
. 4'-[(3-Butyl-1,4-dihydro-4-thioxo-1-chinolinyl)-methyl]-(1,1' biphenyl)-2-carbonsäure;
. 3-Butyl-1-{[2'-carboxy-(1,1'-biphenyl)-4-yl]-methyl}-1,4-dihydro-4-oxo-7-chinolincarbonsäure;
. 4'-{[3-Butyl-1,4-dihydro-7-(hydroxymethyl)-4-oxo-1-chinolinyl]-methyl}-(1,1'-biphenyl)-2-carbonsäure;
. 4'-{{3-Butyl-1,4-dihydro-4-oxo-7-[(1-pyrrolidinyl)-carbonyl]-1-chinolinyl}-methyl}-(1,1'-biphenyl)-2-carbonsäure;
. 4'-[3-Butyl-1,4,5,6,7,8-hexahydro-4-oxo-1-(1-chinolinyl)-methyl]-(1,1'-biphenyl)-2-carbonsäure;
. 4'-[3-Butyl-1,4,5,6,7,8-hexahydro-6-methyl-4-oxo-(1-chinolinyl)-methyl]-(1,1'-biphenyl)-2-carbonsäure;
. 3-Butyl-1,4-dihydro-1-{[2'-(1H-tetrazol-5-yl)-(1,1'-biphenyl)-4-yl]-methyl}-4-oxo-8-chinolincarbonsäure;
. 1,4-Dihydro-3-methyl-4-oxo-1-{[2'-(1H-tetrazol-5-yl)-(1,1'-biphenyl)-4-yl]-methyl}-8-chinolincarbonsäure;
. 1,4-Dihydro-3-ethyl-4-oxo-1-{[2'-(1H-tetrazol-5-yl)-(1,1'-biphenyl)-4-yl]-methyl}-8-chinolincarbonsäure.

4. Verfahren zur Herstellung von Produkten der Formel (I) wie in Anspruch 1 definiert und von Produkten wie in Anspruch 2 definiert, dadurch gekennzeichnet, daß man entweder
ein Produkt der Formel (II) 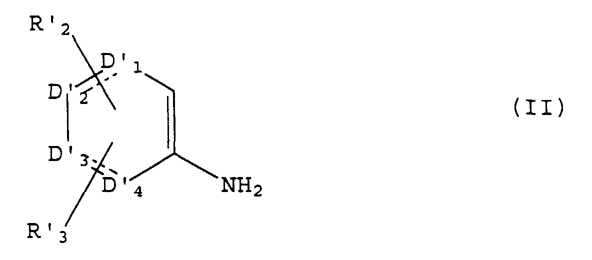 in der D'₁, D'₂, D'₃, D'₄, R'₂ und R'₃ die gleichen Bedeutungen wie in Anspruch 1 bei D₁, D₂, D₃, D₄, R₂ und R₃ angegeben besitzen oder den Produkten wie in Anspruch 3 definiert entsprechen, worin die eventuellen reaktiven Funktionen gegebenenfalls durch Schutzgruppen geschützt sind, mit einem Produkt der Formel (III) 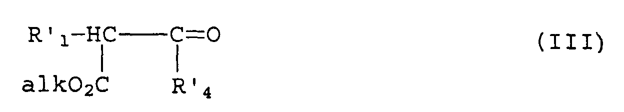 zur Reaktion bringt, worin R'₁ die gleiche Bedeutung wie in Anspruch 1 bei R₁ angegeben besitzt und R'₄ ein Wasserstoffatom oder der Rest Carboxy ist, bei dem die eventuellen reaktiven Funktionen gegebenenfalls durch Schutzgruppen geschützt sind und alk einen Rest Alkyl mit höchstens 6 Kohlenstoffatomen darstellt, um Produkte der Formel (IV) zu erhalten 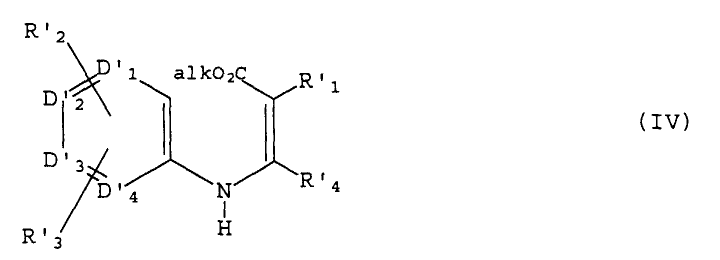 in der D'₁, D'₂, D'₃, D'₄, R'₁, R'₂, R'₃, R'₄ und alk die gleichen Bedeutungen wie oben angegeben besitzen, die man dann cyclisiert, um Produkte der Formel (V) 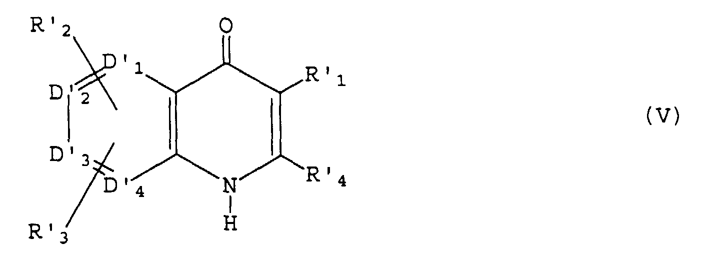 zu erhalten, worin D'₁, D'₂, D'₃, D'₄, R'₁, R'₂, R'₃ und R'₄ die oben angegebenen Bedeutungen besitzen, oder
ein Produkt der Formel (VI) 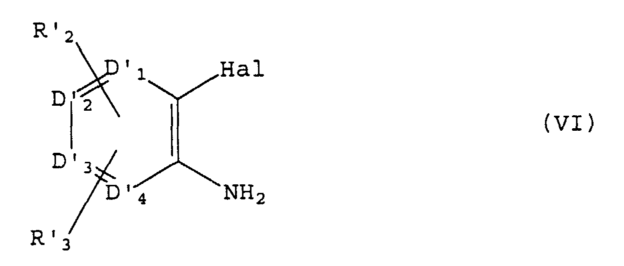 in der D'₁, D'₂, D'₃, D'₄, R'₂ und R'₃ die oben angegebenen Bedeutungen besitzen und Hal ein Halogenatom ist, mit einem Produkt der Formel (VII)
R'₁-C≡CH (VII)
zur Reaktion bringt, in der R'₁ die oben angegebene Bedeutung besitzt, um Produkte der Formel (VIII) 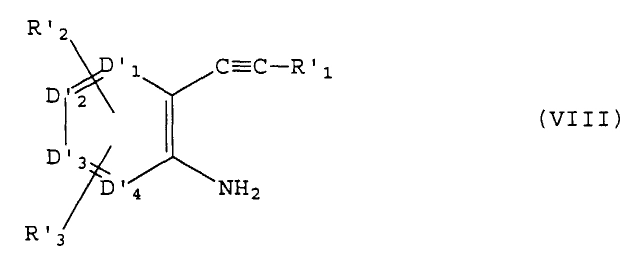 zu erhalten, in der D'₁, D'₂, D'₃, D'₄, R'₁, R'₂ und R'₃ die oben angegebenen Bedeutungen besitzen, das man anschließend einer Reaktion der Cyclisierung unterzieht, um ein Produkt der Formel (IX) 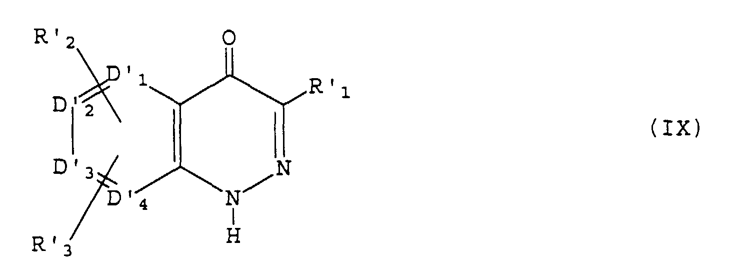 zu erhalten, in der D'₁, D'₂, D'₃, D'₄, R'₁, R'₂ und R'₃ die oben angegebenen Bedeutungen besitzen,
und man die Produkte der Formeln (V) und (IX), wenn erwünscht, einer Reaktion der Hydrierung unterzieht, um jeweils Produkte der Formel (V_{B}) 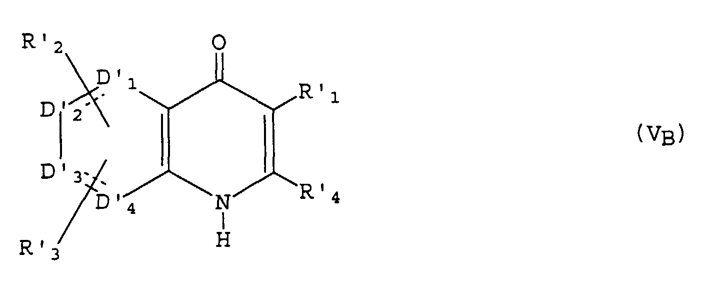 und Produkte der Formel (IX_{B}) 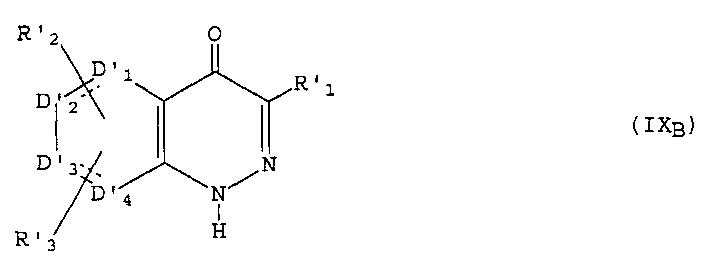 zu erhalten, wobei in diesen Produkten der Formeln (V_{B}) und (IX_{B}) D'₁, D'₂, D'₃, D'₄, R'₁, R'₂, R'₃ und R'₄ die oben angegebenen Bedeutungen besitzen, und man, wenn erwünscht, die Produkte der Formeln (V), (V_{B}), (IX) und (IX_{B}) einer Reaktion zur Umwandlung der Funktion Oxo in eine Funktion Thioxo unterzieht, und daß man sie mit einer Verbindung der Formel (X)
Hal-CH₂-Y' (X)
zur Reaktion bringt, in der Hal ein Halogenatom ist und Y' die gleiche Bedeutung wie oben bei Y angegeben besitzt, bei der die eventuellen reaktiven Funktionen gegebenenfalls durch Schutzgruppen geschützt sind, um ein Produkt der Formel (I') 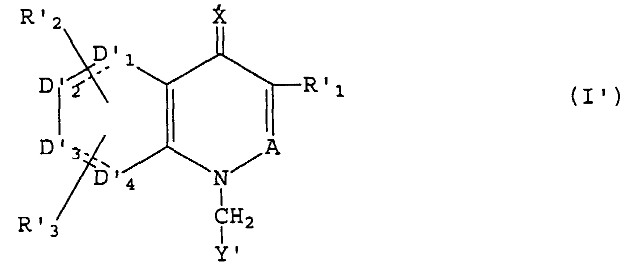 zu erhalten, in der 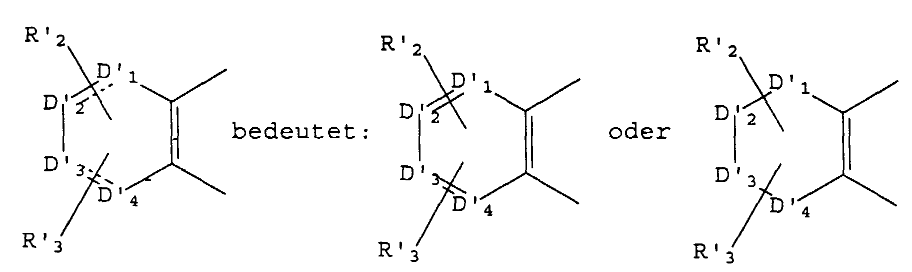 D'₁, D'₂, D'₃, D'₄, R'₁, R'₂, R'₃, R'₄, R'₅ und Y' die oben angegebenen Bedeutungen besitzen, A den Rest C-R'₄ oder ein Stickstoffatom darstellt und X die oben angegebene Bedeutung besitzt, wobei man die Produkte der Formel (I') in der 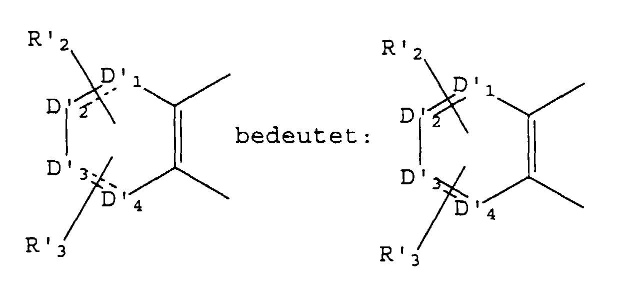 wenn erwünscht, einer Reaktion der Hydrierung unterziehen kann, um Produkte der Formel (I') zu erhalten, in der 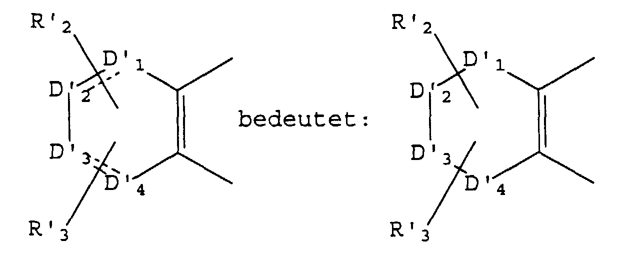 und man das Produkt der Formel (I'), wenn erwünscht und wenn erforderlich, einer oder mehreren der folgenden Reaktionen in irgendeiner Reihenfolge unterzieht:
- einer Reaktion zur Umwandlung der Funktion Oxo C=O in die Funktion Thioxo C=S,
- einer Reaktion zur Entfernung der Schutzgruppen, die von den geschützten, reaktiven Funktionen getragen werden können,
- einer Reaktion zur Salzbildung durch eine Mineralsäure oder organische Säure oder durch eine Mineralbase oder organische Base, um das entsprechende Salz zu erhalten,
- einer Reaktion zur Veresterung der Säurefunktion,
- einer Reaktion zur Verseifung der Esterfunktion in die Säurefunktion,
- einer Reaktion zur Umwandlung der Funktion Alkyloxy in die Funktion Hydroxyl,
- einer Reaktion zur Umwandlung der Funktion Cyano in die Säurefunktion,
- einer Reaktion zur Reduzierung der Funktion Carboxy in die Alkoholfunktion,
- einer Reaktion zur Spaltung der racemischen Formen,
und die auf diese Weise erhaltenen Produkte in allen ihren möglichen isomeren racemischen Formen, Enantiomeren und Diastereoisomeren.

5. Verfahren zur Herstellung der Produkte der Formel (I) wie in Anspruch 1 definiert und der Produkte wie in Anspruch 2 definiert, worin A CH darstellt, dadurch gekennzeichnet,
daß man entweder ein Produkt der Formel (II_{E}) 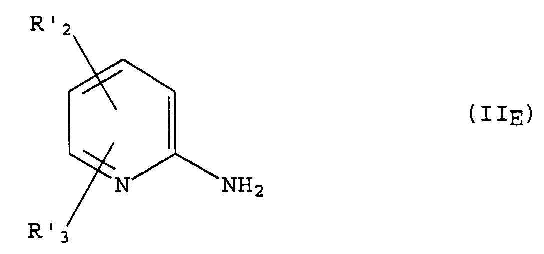 in der R'₂ und R'₃ die jeweils in Anspruch 1 bei R₂ und R₃ angegebenen Bedeutungen besitzen oder den Produkten wie in Anspruch 2 definiert entsprechen, bei denen die eventuellen reaktiven Funktionen gegebenenfalls durch Schutzgruppen geschützt sind, mit einem Produkt der Formel (XI) 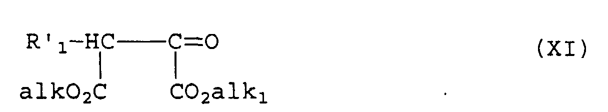 zur Reaktion bringt, in der R'₁ die in Anspruch 1 bei R₁ angegebene Bedeutung besitzt, worin die eventuellen reaktiven Funktionen gegebenenfalls durch Schutzgruppen geschützt sind und alk und alk₁, gleich oder verschieden, einen Rest Alkyl mit höchstens 6 Kohlenstoffatomen darstellen, um Produkte der Formel (XII) 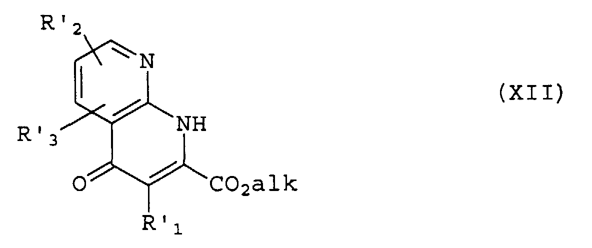 zu erhalten, in der R'₁, R'₂, R'₃ und alk die oben angegebenen Bedeutungen besitzen,
oder ein Produkt der Formel (II_{F}) 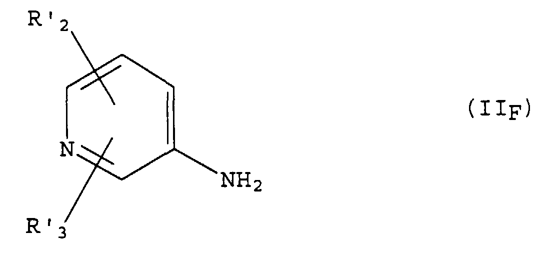 in der R'₂ und R'₃ die oben angegebenen Bedeutungen besitzen, mit dem wie oben definierten Produkt der Formel (XI) zur Reaktion bringt, um Produkte der Formel (XIII) 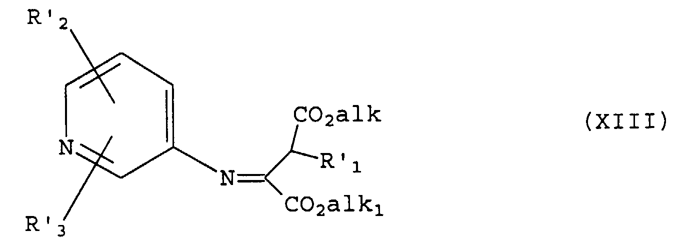 zu erhalten, in der R'₁, R'₂, R'₃, alk und alk₁ die oben angegebenen Bedeutungen besitzen, und man die Produkte der Formeln (XII) und (XIII) in ein Produkt der Formel (XIV) 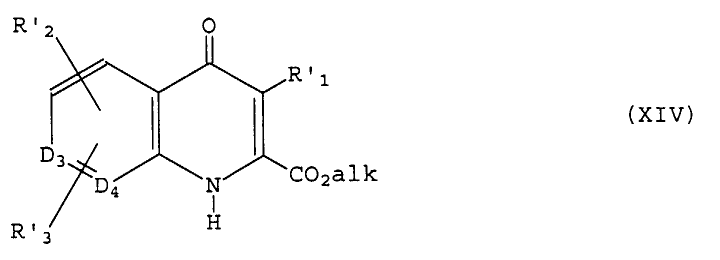 umwandelt, in der R'₁, R'₂, R'₃ und alk₁ die oben angegebenen Bedeutungen besitzen und D₃ und D₄ die in Anspruch 1 angegebenen Bedeutungen besitzen, und man die Produkte der Formel (XIV), wenn erwünscht, einer Reaktion zur Hydrierung unterzieht, um die Produkte der Formel (XV) 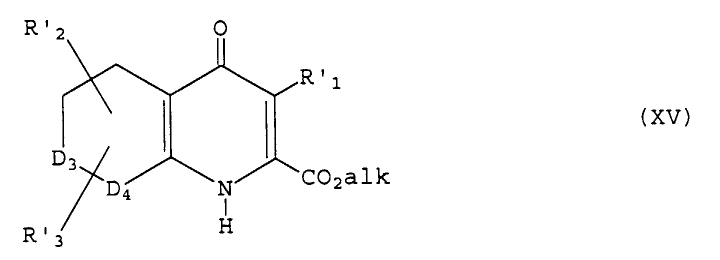 zu erhalten, in der D₃, D₄ , R'₁, R'₂, R'₃ und alk₁ die oben angegebenen Bedeutungen besitzen, und man die Produkte der Formel (XIV) und (XV) einer Reaktion der Verseifung und anschließend einer Decarboxylierung unterzieht, um die entsprechenden Produkte der Formeln (XIV') 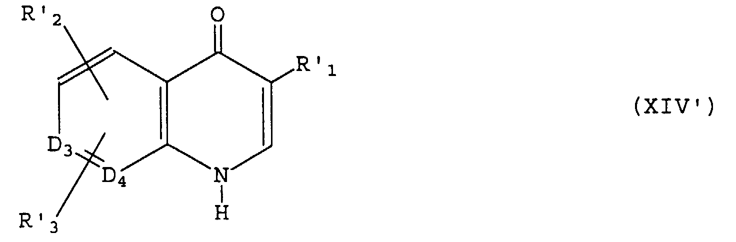 und der Formel (XV') 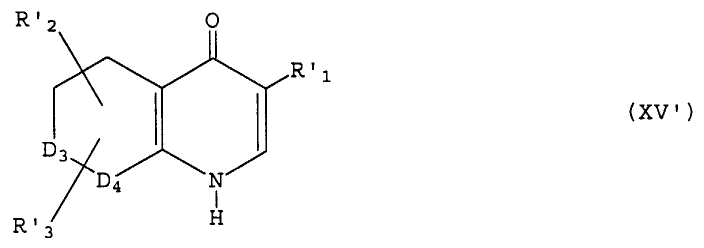 zu erhalten, und man die Produkte der Formeln (XIV') und (XV'), wenn erwünscht, einer Reaktion zu Umwandlung der Funktion Oxo in die Funktion Thioxo unterzieht, und daß man sie mit einer Verbindung der Formel (X)
Hal-CH₂-Y' (X)
zur Reaktion bringt, in der Y' die in Anspruch 1 bei Y angegebene Bedeutung besitzt, worin die eventuellen reaktiven Funktionen gegebenenfalls durch Schutzgruppen geschützt sind, um ein Produkt der Formel (I'_{EB}) 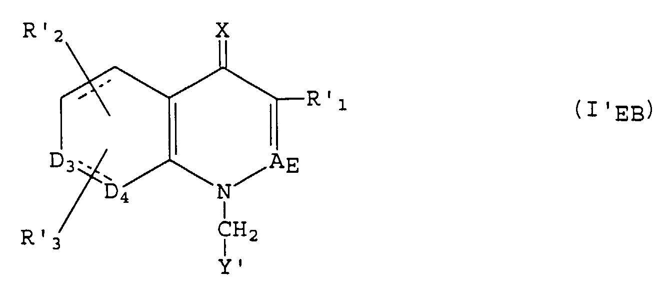 zu erhalten, in der 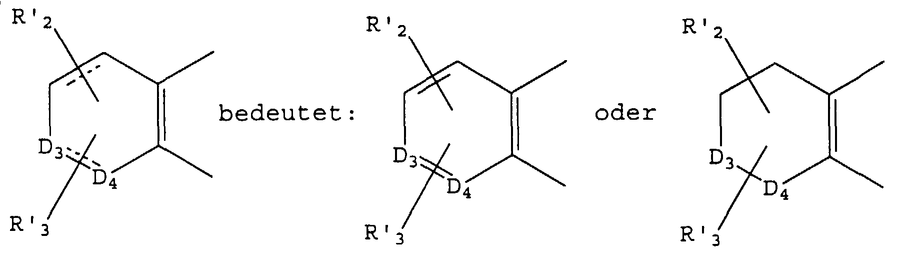 D₃, D₄, R'₁, R'₂, R'₃ und Y' die oben angegebenen Bedeutungen besitzen, A_{E} den Rest CH darstellt und X die oben angegebene Bedeutung besitzt, und man die Produkte der Formel (I'_{EB}), in der 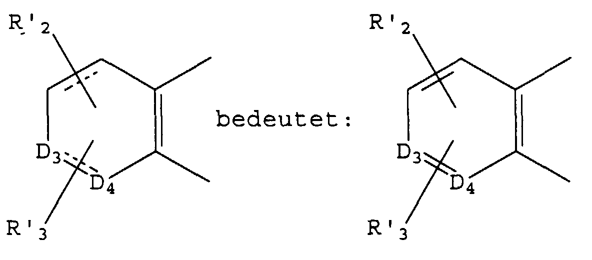 wenn erwünscht, einer Reaktion der Hydrierung unterziehen kann, um Produkte der Formel (I'_{EB}) zu erhalten, in der 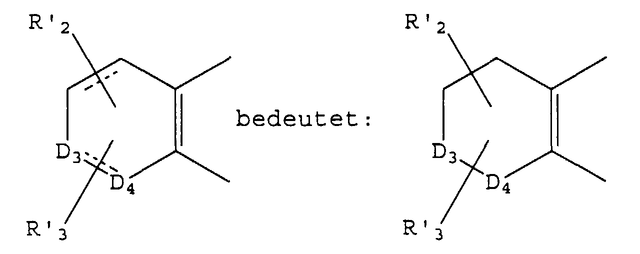 und man das Produkt der Formel (I'_{EB}), wenn erwünscht und wenn erforderlich, einer oder mehreren der folgenden Reaktionen in irgendeiner Reihenfolge unterzieht:
- einer Reaktion zur Umwandlung der Funktion Oxo C=O in die Funktion Thioxo C=S,
- einer Reaktion zur Entfernung der Schutzgruppen, die von den geschützten, reaktiven Funktionen getragen werden können,
- einer Reaktion zur Salzbildung durch eine Mineralsäure oder organische Säure oder durch eine Mineralbase oder organische Base, um das entsprechende Salz zu erhalten,
- einer Reaktion zur Veresterung der Säurefunktion,
- einer Reaktion zur Verseifung der Esterfunktion in die Säurefunktion,
- einer Reaktion zur Umwandlung der Funktion Alkyloxy in die Funktion Hydroxyl,
- einer Reaktion zur Umwandlung der Funktion Cyano in die Säurefunktion,
- einer Reaktion zur Reduzierung der Funktion Carboxy in die Alkoholfunktion,
- einer Reaktion zur Spaltung der racemischen Formen,
und die auf diese Weise erhaltenen Produkte in allen ihren möglichen isomeren racemischen Formen, Enantiomeren und Diastereoisomeren.

6. Als Arzneimittel die Produkte wie in den Ansprüchen 1 bis 3 definiert, und diese Produkte in allen ihren möglichen isomeren racemischen Formen, Enantiomeren und Diastereoisomeren, sowie die Additionssalze dieser Produkte mit pharmazeutisch akzeptablen Mineralsäuren und organischen Säuren oder mit Mineralbasen und organischen Basen.

7. Als Arzneimittel die Produkte wie im Anspruch 2 definiert, sowie die Additionssalze dieser Produkte mit pharmazeutisch akzeptablen Mineralsäuren und organischen Säuren oder mit Mineralbasen und organischen Basen.

8. Pharmazeutische Zusammensetzungen, enthaltend als Wirkstoff mindestens eines der Arzneimittel wie in irgendeinem der Ansprüche 6 und 7 definiert.

9. Verwendung der Produkte wie in den Ansprüchen 1 bis 3 definiert, und diese Produkte in allen ihren möglichen isomeren racemischen Formen, Enantiomeren und Diastereoisomeren, sowie der Additionssalze dieser Produkte mit Mineralsäuren und organischen Säuren oder mit Mineralbasen und organischen Basen für die Herstellung von Arzneimitteln, vorgesehen für:
entweder die Behandlung von arteriellem Überdruck, Herzinsuffizienzen, Glaukom, Niereninsuffizienzen und die Vorbeugung von postangioplastischen Restenosen,
oder die Behandlung von einigen gastro-intestinalen oder gynäkologischen Störungen,
oder die Behandlung von Störungen des Gedächtnisses oder der kognitiven Funktionen, von Angst, Depression, seniler Demenz oder der Alzheimer-Erkrankung.

## Patentansprüche (Patentansprüche für folgende(n) Vertragsstaat(en): ES, GR)

1. Verfahren zur Herstellung von Produkten der Formel (I) 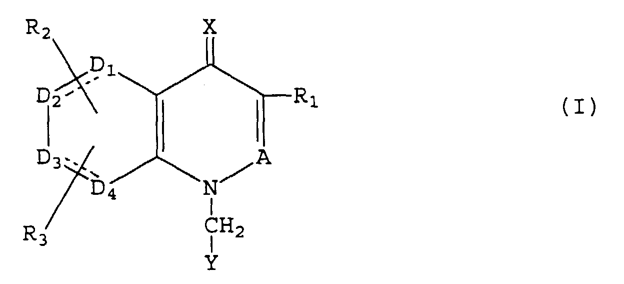 in der 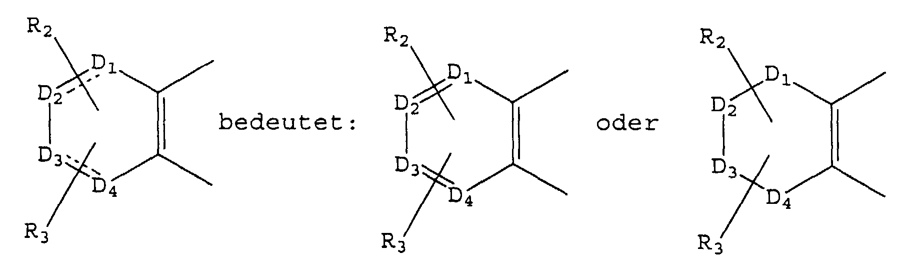
mit D₁, D₂, die beide einen Rest Methin oder einen Rest Methylen darstellen, gegebenenfalls substituiert durch einen unter R₂ und R₃ ausgewählten Rest,
D₃ und D₄, gleich oder verschieden, ein Stickstoffatom, einen Rest Methin oder einen Rest Methylen bedeuten, gegebenenfalls substituiert durch einen unter R₂ und R₃ ausgewählten Rest,
mindestens eines von D₃ und D₄ einen Rest Methin oder Methylen darstellt, gegebenenfalls substituiert durch einen unter R₂ und R₃ ausgewählten Rest,
R₁ einen Rest Methyl, Ethyl, n-Propyl, n-Propenyl, n-Butyl bedeutet,
R₂ und R₃, gleich oder verschieden, aus der Gruppe gewählt werden, die gebildet wird durch:
- das Wasserstoffatom,
- die Reste Mercapto und Alkylthio,
- die linearen oder verzweigten Reste Alkyl mit höchstens 6 Kohlenstoffatomen und gegebenenfalls substituiert durch einen oder mehrere, gleiche oder verschiedene Substituenten, ausgewählt unter den Halogenatomen, dem Rest Hydroxyl, dem Rest Amino, Mono- und Dialkylamino, Pyrrolidinyl, Morpholinyl, Piperidinyl,
- den Rest Carboxy, frei, in Salz überführt oder verestert durch einen linearen oder verzweigten Rest Alkyl mit höchstens 4 Kohlenstoffatomen,
- den Rest Pyrrolidinyl-carbonyl, Morpholinyl-carbonyl, Carbamoyl, Dialkylcarbamoyl, Piperidinyl-carbonyl,
A ein Stickstoffatom oder einen Rest CH darstellt,
X ein Sauerstoffatom oder ein Schwefelatom bedeutet,
Y ein Rest Phenyl oder Biphenyl ist, gegebenenfalls substituiert durch einen oder mehrere Reste, ausgewählt unter den Resten Cyano, Carboxy, frei, in Salz überführt und verestert, Tetrazol und Isoxazol,
mit Ausnahme der Produkte der Formel (I), in der 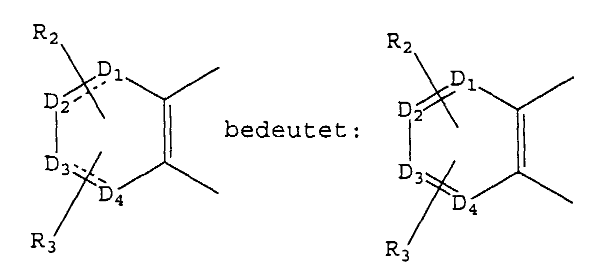
mit D₁, D₂, D₃ und D₄, die jeweils einen Rest Methin darstellen, X ein Sauerstoffatom ist und A einen Rest CH bedeutet, R₁, R₂ und R₃ gleich sind und alle drei einen Rest Methyl darstellen, und -Y ein Rest Phenyl ist,
die genannten Produkte der Formel (I) in allen ihren möglichen isomeren racemischen Formen, Enantiomeren und Diastereoisomeren, sowie die Additionssalze der genannten Produkte der Formel (I) mit Mineralsäuren und organischen Säuren oder mit Mineralbasen und organischen Basen, dadurch gekennzeichnet,
daß man entweder ein Produkt der Formel (II) 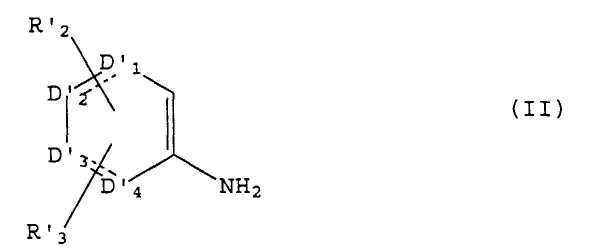 in der D'₁, D'₂, D'₃, D'₄, R'₂ und R'₃ die oben bei D₁, D₂, D₃, D₄, R₂ und R₃ angegebenen Bedeutungen besitzen, worin die eventuellen reaktiven Funktionen gegebenenfalls durch Schutzgruppen geschützt sind, mit einem Produkt der Formel (III) 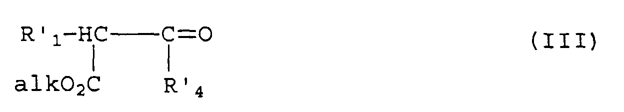 zur Reaktion bringt, worin R'₁ die gleiche Bedeutung wie oben bei R₁ angegeben besitzt und R'₄ ein Wasserstoffatom oder der Rest Carboxy ist, bei dem die eventuellen reaktiven Funktionen gegebenenfalls durch Schutzgruppen geschützt sind und alk einen Rest Alkyl mit höchstens 6 Kohlenstoffatomen darstellt, um Produkte der Formel (IV) zu erhalten 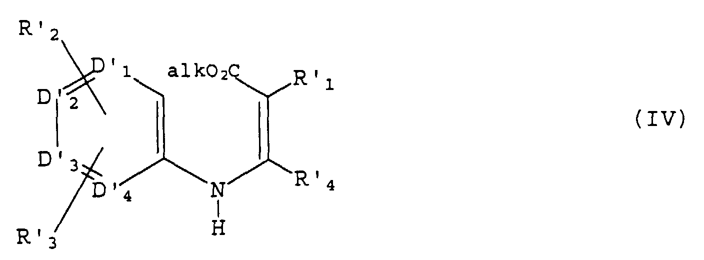 in der D'₁, D'₂, D'₃, D'₄, R'₁, R'₂, R'₃, R'₄ und alk die gleichen Bedeutungen wie oben angegeben besitzen, die man dann cyclisiert, um Produkte der Formel (V) 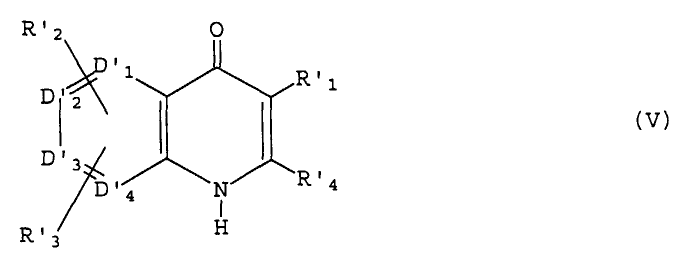 zu erhalten, worin D'₁, D'₂, D'₃, D'₄, R'₁, R'₂, R'₃ und R'₄ die oben angegebenen Bedeutungen besitzen, oder
ein Produkt der Formel (VI) 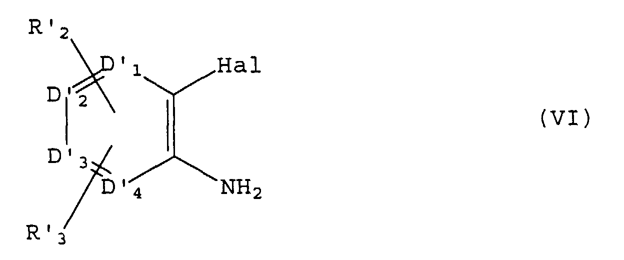 in der D'₁, D'₂, D'₃, D'₄, R'₂ und R'₃ die oben angegebenen Bedeutungen besitzen und Hal ein Halogenatom ist, mit einem Produkt der Formel (VII)
R'₁-C≡CH (VII)
zur Reaktion bringt, in der R'₁ die oben angegebene Bedeutung besitzt, um Produkte der Formel (VIII) 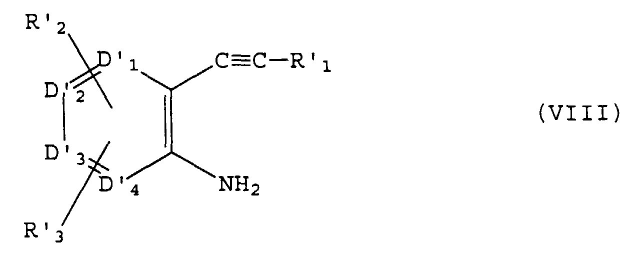 zu erhalten, in der D'₁, D'₂, D'₃, D'₄, R'₁, R'₂ und R'₃ die oben angegebenen Bedeutungen besitzen, das man anschließend einer Reaktion der Cyclisierung unterzieht, um ein Produkt der Formel (IX) 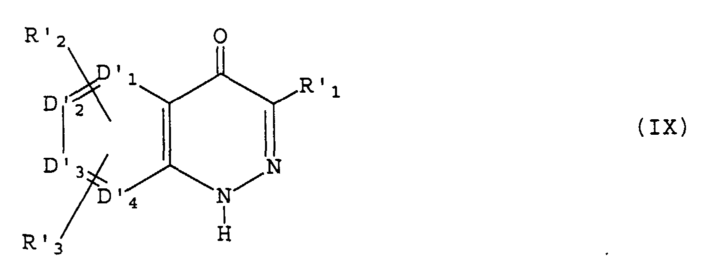 zu erhalten, in der D'₁, D'₂, D'₃, D'₄, R'₁, R'₂ und R'₃ die oben angegebenen Bedeutungen besitzen,
und man die Produkte der Formeln (V) und (IX), wenn erwünscht, einer Reaktion der Hydrierung unterzieht, um jeweils Produkte der Formel (V_{B}) 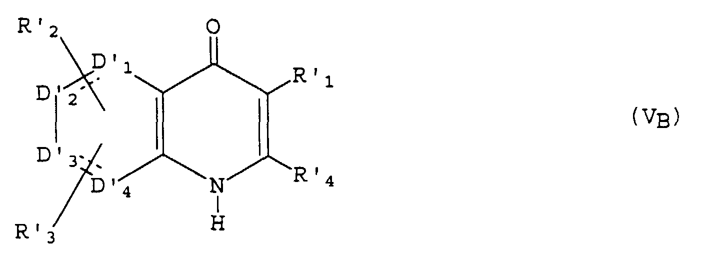 und Produkte der Formel (IX_{B}) 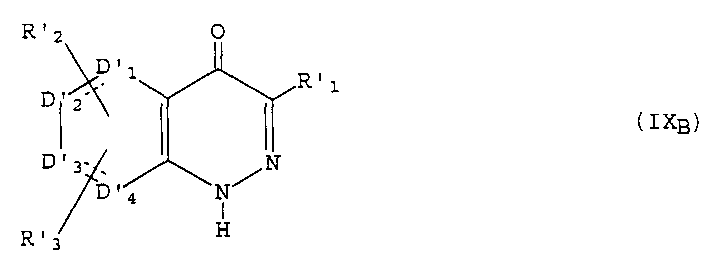 zu erhalten, wobei in diesen Produkten der Formeln (V_{B}) und (IX_{B}) D'₁, D'₂, D'₃, D'₄, R'₁, R'₂, R'₃ und R'₄ die oben angegebenen Bedeutungen besitzen, und man, wenn erwünscht, die Produkte der Formeln (V), (V_{B}), (IX) und (IX_{B}) einer Reaktion zur Umwandlung der Funktion Oxo in eine Funktion Thioxo unterzieht, und daß man sie mit einer Verbindung der Formel (X)
Hal-CH₂-Y' (X)
zur Reaktion bringt, in der Hal ein Halogenatom ist und Y' die gleiche Bedeutung wie oben bei Y angegeben besitzt, bei der die eventuellen reaktiven Funktionen gegebenenfalls durch Schutzgruppen geschützt sind, um ein Produkt der Formel (I') 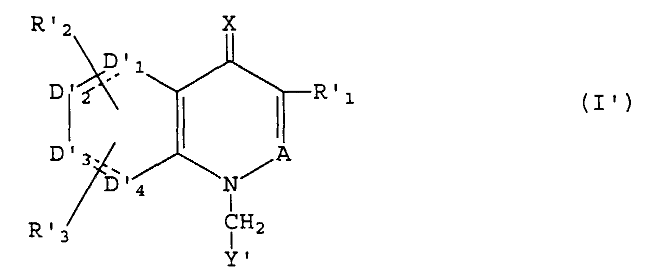 zu erhalten, in der 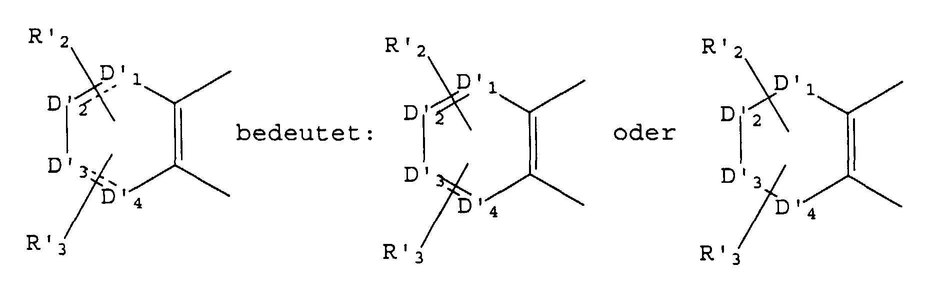 D'₁, D'₂, D'₃, D'₄, R'₁, R'₂, R'₃, R'₄, R'₅ und Y' die oben angegebenen Bedeutungen besitzen, A den Rest C-R'₄ oder ein Stickstoffatom darstellt und X die oben angegebene Bedeutung besitzt, wobei man die Produkte der Formel (I') in der 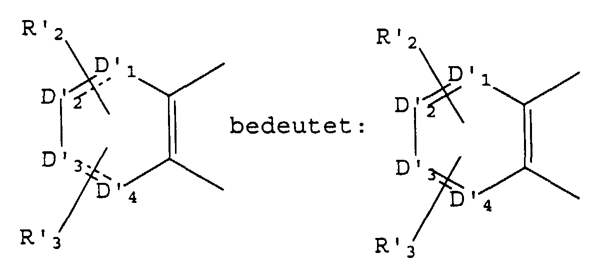 wenn erwünscht, einer Reaktion der Hydrierung unterziehen kann, um Produkte der Formel (I') zu erhalten, in der 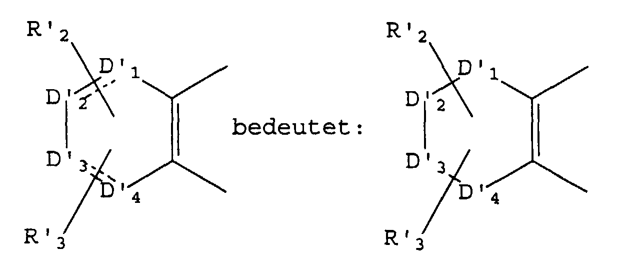 und man das Produkt der Formel (I'), wenn erwünscht und wenn erforderlich, einer oder mehreren der folgenden Reaktionen in irgendeiner Reihenfolge unterzieht:
- einer Reaktion zur Umwandlung der Funktion Oxo C=O in die Funktion Thioxo C=S,
- einer Reaktion zur Entfernung der Schutzgruppen, die von den geschützten, reaktiven Funktionen getragen werden können,
- einer Reaktion zur Salzbildung durch eine Mineralsäure oder organische Säure oder durch eine Mineralbase oder organische Base, um das entsprechende Salz zu erhalten,
- einer Reaktion zur Veresterung der Säurefunktion,
- einer Reaktion zur Verseifung der Esterfunktion in die Säurefunktion,
- einer Reaktion zur Umwandlung der Funktion Alkyloxy in die Funktion Hydroxyl,
- einer Reaktion zur Umwandlung der Funktion Cyano in die Säurefunktion,
- einer Reaktion zur Reduzierung der Funktion Carboxy in die Alkoholfunktion,
- einer Reaktion zur Spaltung der racemischen Formen,
und die auf diese Weise erhaltenen Produkte in allen ihren möglichen isomeren racemischen Formen, Enantiomeren und Diastereoisomeren.

2. Verfahren wie in Anspruch 1 definiert zur Herstellung der folgenden Produkte:
. 4'-[(3-Butyl-1,4-dihydro-7-formyl-4-oxo-1-chinolinyl)-methyl]-(1,1'-biphenyl)-2-carbonsäure;
. 3-Butyl-1-{[2'-carboxy-(1,1'-biphenyl)-4-yl]-methyl}-1,4-dihydro-4-oxo-7-chinolin-propansäure;
. 4'-{[3-Butyl-6-(2-carboxyethenyl)-1,4-dihydro-4-oxo-1-chinolinyl]-methyl}-(1,1'-biphenyl)-2-carbonsäure;
. 4'-[(3-Butyl-1,4-dihydro-6-formyl-4-oxo-1-chinolinyl)-methyl]-(1,1'-biphenyl)-2-carbonsäure;
. 4'-{[3-Butyl-1,4-dihydro-7-(3-hydroxy-3-oxo-1-propenyl)-4-oxo-1-chinolinyl]-methyl}-(1,1'-biphenyl)-2-carbonsäure;
. 3-Butyl-1-{[2'-carboxy-(1,1'-biphenyl)-4-yl]-methyl}-1,4-dihydro-4-oxo-7-chinolin-essigsäure;
. 4'-{{3-Butyl-1,4-dihydro-4-oxo-7-[2-(1-pyrrolidinyl)-2-oxo-ethyl]-1-chinolinyl}-methyl}-(1,1'-biphenyl)-2-carbonsäure;
. 1,4-Dihydro-4-oxo-1-{[2'-(1H-tetrazol-5-yl)-(1,1'-biphenyl)-4-yl]-methyl}-chinolin-3,8-dicarbonsäure;
. 3-Butyl-1-{[2'-(1H-tetrazol-5-yl)-(1,1'-biphenyl)-4-yl]-methyl}-6,7,8-trifluor-4-(1H)-chinolinon;
. 3-Butyl-6,8-difluor-7-(1-pyrrolidinyl)-1-{[2'-(1H-tetrazol-5-yl)-(1,1'-biphenyl)-4-yl]-methyl}-4-(1H)-chinolinon;
diese Produkte in allen ihren möglichen isomeren racemischen Formen, Enantiomeren und Diastereoisomeren, sowie die Additionssalze dieser Produkte mit Mineralsäuren und organischen Säuren oder mit Mineralbasen und organischen Basen.

3. Verfahren zur Herstellung von Produkten der Formel (I) 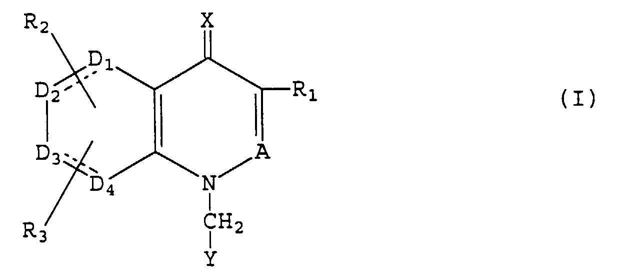 in der 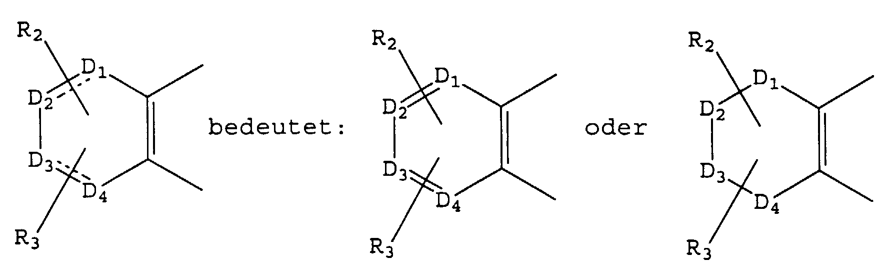
mit D₁, D₂, die beide einen Rest Methin oder einen Rest Methylen darstellen, gegebenenfalls substituiert durch einen unter R₂ und R₃ ausgewählten Rest,
D₃ und D₄, gleich oder verschieden, ein Stickstoffatom, einen Rest Methin oder einen Rest Methylen bedeuten, gegebenenfalls substituiert durch einen unter R₂ und R₃ ausgewählten Rest,
mindestens eines von D₃ und D₄ einen Rest Methin oder Methylen darstellt, gegebenenfalls substituiert durch einen unter R₂ und R₃ ausgewählten Rest,
R₁ einen Rest Methyl, Ethyl, n-Propyl, n-Propenyl, n-Butyl bedeutet,
R₂ und R₃, gleich oder verschieden, aus der Gruppe gewählt werden, die gebildet wird durch:
- das Wasserstoffatom,
- die Reste Mercapto und Alkylthio,
- die linearen oder verzweigten Reste Alkyl mit höchstens 6 Kohlenstoffatomen und gegebenenfalls substituiert durch einen oder mehrere, gleiche oder verschiedene Substituenten, ausgewählt unter den Halogenatomen, dem Rest Hydroxyl, dem Rest Amino, Mono- und Dialkylamino, Pyrrolidinyl, Morpholinyl, Piperidinyl,
- den Rest Carboxy, frei, in Salz überführt oder verestert durch einen linearen oder verzweigten Rest Alkyl mit höchstens 4 Kohlenstoffatomen,
- den Rest Pyrrolidinyl-carbonyl, Morpholinyl-carbonyl, Carbamoyl, Dialkylcarbamoyl, Piperidinyl-carbonyl,
A ein Stickstoffatom oder einen Rest CH darstellt,
X ein Sauerstoffatom oder ein Schwefelatom bedeutet,
Y ein Rest Phenyl oder Biphenyl ist, gegebenenfalls substituiert durch einen oder mehrere Reste, ausgewählt unter den Resten Cyano, Carboxy, frei, in Salz überführt und verestert, Tetrazol und Isoxazol,
mit Ausnahme der Produkte der Formel (I), in der 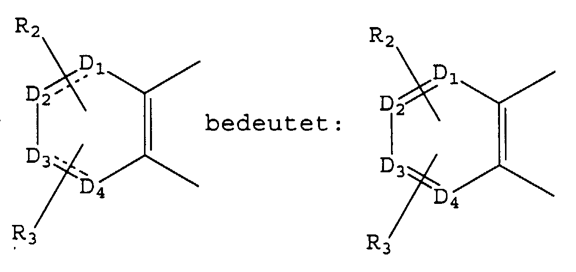
mit D₁, D₂, D₃ und D₄, die jeweils einen Rest Methin darstellen, X ein Sauerstoffatom ist und A einen Rest CH bedeutet, R₁, R₂ und R₃ gleich sind und alle drei einen Rest Methyl darstellen, und -Y ein Rest Phenyl ist,
die genannten Produkte der Formel (I) in allen ihren möglichen isomeren racemischen Formen, Enantiomeren und Diastereoisomeren, sowie die Additionssalze der genannten Produkte der Formel (I) mit Mineralsäuren und organischen Säuren oder mit Mineralbasen und organischen Basen, dadurch gekennzeichnet,
daß man entweder ein Produkt der Formel (II_{E}) 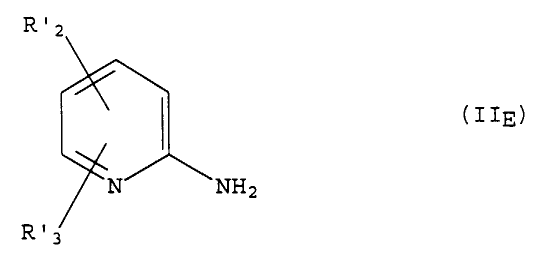 in der R'₂ und R'₃ die jeweils oben bei R₂ und R₃ angegebenen Bedeutungen besitzen, bei denen die eventuellen reaktiven Funktionen gegebenenfalls durch Schutzgruppen geschützt sind, mit einem Produkt der Formel (XI) 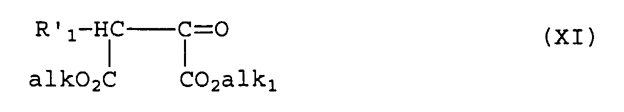 zur Reaktion bringt, in der R'₁ die oben bei R₁ angegebene Bedeutung besitzt, worin die eventuellen reaktiven Funktionen gegebenenfalls durch Schutzgruppen geschützt sind und alk und alk₁, gleich oder verschieden, einen Rest Alkyl mit höchstens 6 Kohlenstoffatomen darstellen, um Produkte der Formel (XII) 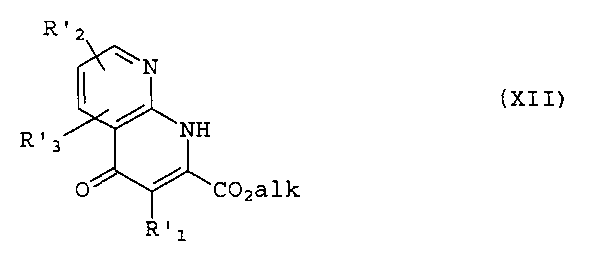 zu erhalten, in der R'₁, R'₂, R'₃ und alk die oben angegebenen Bedeutungen besitzen,
oder ein Produkt der Formel (II_{F}) 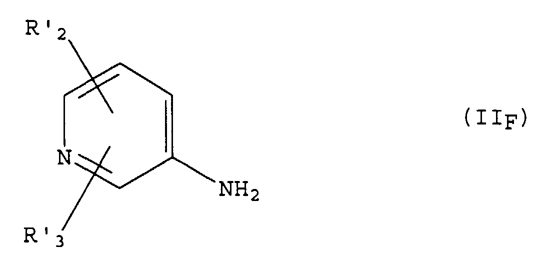 in der R'₂ und R'₃ die oben angegebenen Bedeutungen besitzen, mit dem wie oben definierten Produkt der Formel (XI) zur Reaktion bringt, um Produkte der Formel (XIII) 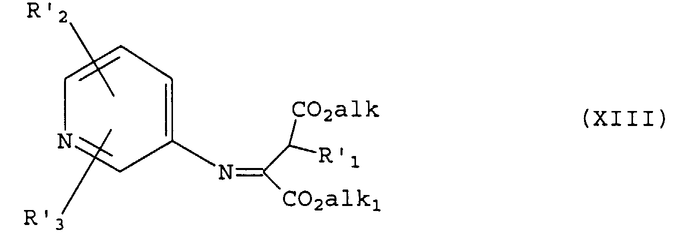 zu erhalten, in der R'₁, R'₂, R'₃, alk und alk₁ die oben angegebenen Bedeutungen besitzen, und man die Produkte der Formeln (XII) und (XIII) in ein Produkt der Formel (XIV) 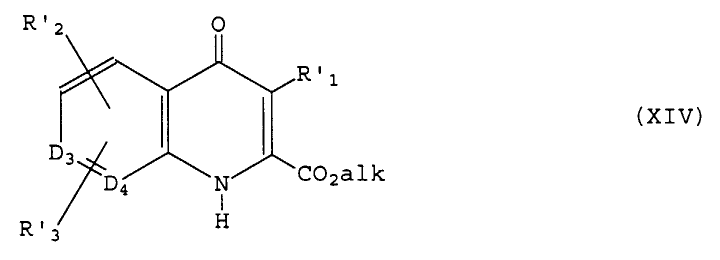 umwandelt, in der R'₁, R'₂, R'₃ und alk₁ die oben angegebenen Bedeutungen besitzen und D₃ und D₄ die in Anspruch 1 angegebenen Bedeutungen besitzen, und man die Produkte der Formel (XIV), wenn erwünscht, einer Reaktion zur Hydrierung unterzieht, um die Produkte der Formel (XV) 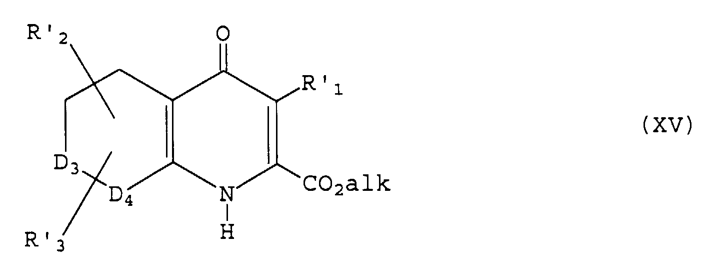 zu erhalten, in der D₃, D₄ , R'₁, R'₂, R'₃ und alk₁ die oben angegebenen Bedeutungen besitzen, und man die Produkte der Formel (XIV) und (XV) einer Reaktion der Verseifung und anschließend einer Decarboxylierung unterzieht, um die entsprechenden Produkte der Formeln (XIV') 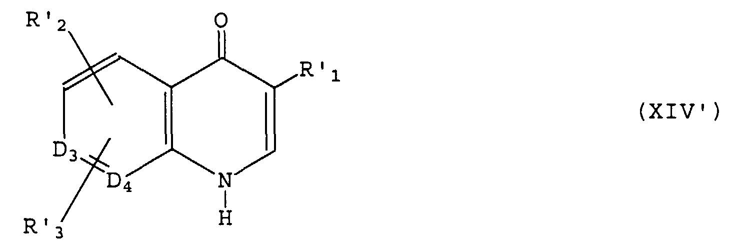 und der Formel (XV') 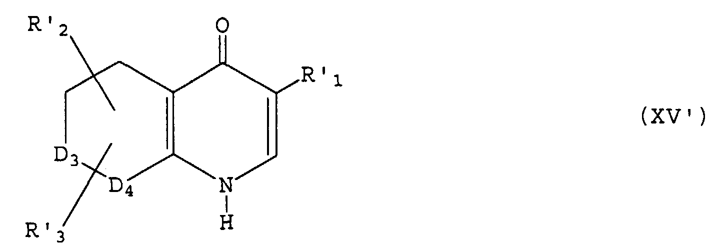 zu erhalten, und man die Produkte der Formeln (XIV') und (XV'), wenn erwünscht, einer Reaktion zu Umwandlung der Funktion Oxo in die Funktion Thioxo unterzieht, und daß man sie mit einer Verbindung der Formel (X)
Hal-CH₂-Y' (X)
zur Reaktion bringt, in der Y' die in Anspruch 1 bei Y angegebene Bedeutung besitzt, worin die eventuellen reaktiven Funktionen gegebenenfalls durch Schutzgruppen geschützt sind, um ein Produkt der Formel (I'_{EB}) 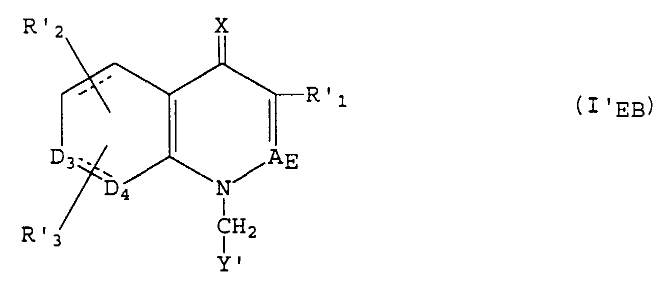 zu erhalten, in der 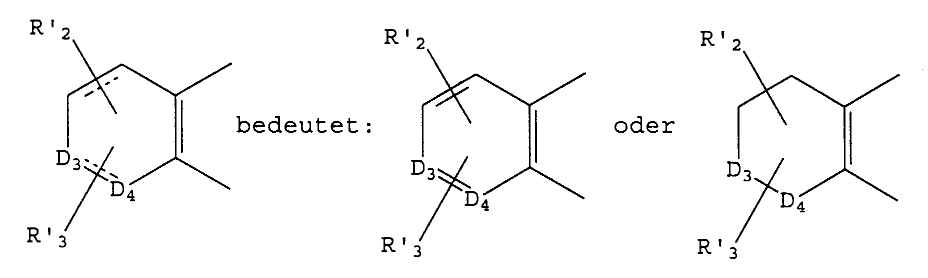 D₃, D₄, R'₁, R'₂, R'₃ und Y' die oben angegebenen Bedeutungen besitzen, A_{E} den Rest CH darstellt und X die oben angegebene Bedeutung besitzt, und man die Produkte der Formel (I'_{EB}), in der 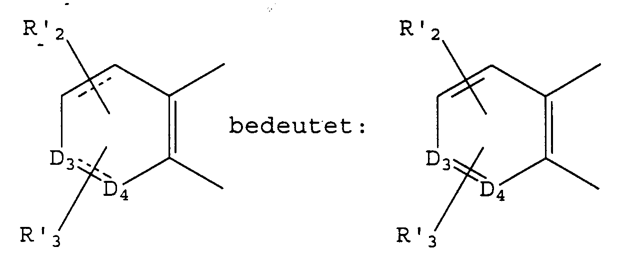 wenn erwünscht, einer Reaktion der Hydrierung unterziehen kann, um Produkte der Formel (I'_{EB}) zu erhalten, in der 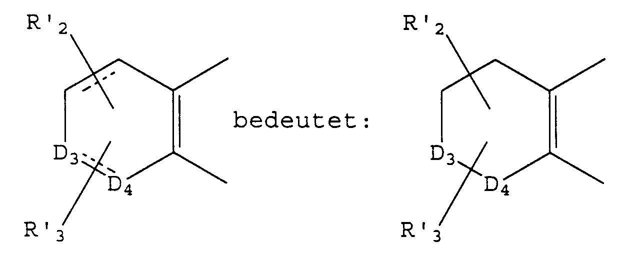 und man das Produkt der Formel (I'_{EB}), wenn erwünscht und wenn erforderlich, einer oder mehreren der folgenden Reaktionen in irgendeiner Reihenfolge unterzieht:
- einer Reaktion zur Umwandlung der Funktion Oxo C=O in die Funktion Thioxo C=S,
- einer Reaktion zur Entfernung der Schutzgruppen, die von den geschützten, reaktiven Funktionen getragen werden können,
- einer Reaktion zur Salzbildung durch eine Mineralsäure oder organische Säure oder durch eine Mineralbase oder organische Base, um das entsprechende Salz zu erhalten,
- einer Reaktion zur Veresterung der Säurefunktion,
- einer Reaktion zur Verseifung der Esterfunktion in die Säurefunktion,
- einer Reaktion zur Umwandlung der Funktion Alkyloxy in die Funktion Hydroxyl,
- einer Reaktion zur Umwandlung der Funktion Cyano in die Säurefunktion,
- einer Reaktion zur Reduzierung der Funktion Carboxy in die Alkoholfunktion,
- einer Reaktion zur Spaltung der racemischen Formen,
und die auf diese Weise erhaltenen Produkte in allen ihren möglichen isomeren racemischen Formen, Enantiomeren und Diastereoisomeren.

4. Verfahren wie in Anspruch 3 definiert zur Herstellung der folgenden Produkte:
. 4'-[(3-Butyl-1,4-dihydro-7-formyl-4-oxo-1-chinolinyl)-methyl]-(1,1'-biphenyl)-2-carbonsäure;
. 3-Butyl-1-{[2'-carboxy-(1,1'-biphenyl)-4-yl]-methyl}-1,4-dihydro-4-oxo-7-chinolin-propansäure;
. 4'-{[3-Butyl-6-(2-carboxyethenyl)-1,4-dihydro-4-oxo-1-chinolinyl]-methyl}-(1,1'-biphenyl)-2-carbonsäure;
. 4'-[(3-Butyl-1,4-dihydro-6-formyl-4-oxo-1-chinolinyl)-methyl]-(1,1'-biphenyl)-2-carbonsäure;
. 4'-{[3-Butyl-1,4-dihydro-7-(3-hydroxy-3-oxo-1-propenyl)-4-oxo-1-chinolinyl]-methyl}-(1,1'-biphenyl)-2-carbonsäure;
. 3-Butyl-1-{[2'-carboxy-(1,1'-biphenyl)-4-yl]-methyl}-1,4-dihydro-4-oxo-7-chinolin-essigsäure;
. 4'-{{3-Butyl-1,4-dihydro-4-oxo-7-[2-(1-pyrrolidinyl)-2-oxo-ethyl]-1-chinolinyl}-methyl}-(1,1'-biphenyl)-2-carbonsäure;
. 1,4-Dihydro-4-oxo-1-{[2'-(1H-tetrazol-5-yl)-(1,1'-biphenyl)-4-yl]-methyl}-chinolin-3,8-dicarbonsäure;
. 3-Butyl-1-{[2'-(1H-tetrazol-5-yl)-(1,1'-biphenyl)-4-yl]-methyl}-6,7,8-trifluor-4-(1H)-chinolinon;
. 3-Butyl-6,8-difluor-7-(1-pyrrolidinyl)-1-{[2'-(1H-tetrazol-5-yl)-(1,1'-biphenyl)-4-yl]-methyl}-4-(1H)-chinolinon;
diese Produkte in allen ihren möglichen isomeren racemischen Formen, Enantiomeren und Diastereoisomeren, sowie die Additionssalze dieser Produkte, in denen A CH darstellt, mit Mineralsäuren und organischen Säuren oder mit Mineralbasen und organischen Basen.

5. Verfahren nach irgendeinem der Ansprüche 1 bis 4, in denen die Produkte den folgenden Definitionen entsprechen:
. 4'-[(3-Butyl-1,4-dihydro-4-oxo-1-chinolinyl)-methyl]-(1,1'-biphenyl)-2-carbonsäure;
. 4'-[(3-Butyl-1,4-dihydro-4-thioxo-1-chinolinyl)-methyl]-(1,1'-biphenyl)-2-carbonsäure;
. 3-Butyl-1-{[2'-carboxy-(1,1'-biphenyl)-4-yl]-methyl}-1,4-dihydro-4-oxo-7-chinolincarbonsäure;
. 4'-{[3-Butyl-1,4-dihydro-7-(hydroxymethyl)-4-oxo-1-chinolinyl]-methyl}-(1,1'-biphenyl)-2-carbonsäure;
. 4'-{{3-Butyl-1,4-dihydro-4-oxo-7-[(1-pyrrolidinyl)-carbonyl]-1-chinolinyl}-methyl}-(1,1'-biphenyl)-2-carbonsäure;
. 4'-[3-Butyl-1,4,5,6,7,8-hexahydro-4-oxo-1-(1-chinolinyl)-methyl]-(1,1'-biphenyl)-2-carbonsäure;
. 4'-[3-Butyl-1,4,5,6,7,8-hexahydro-6-methyl-4-oxo-(1-chinolinyl)-methyl]-(1,1'-biphenyl)-2-carbonsäure;
. 3-Butyl-1,4-dihydro-1-{[2'-(1H-tetrazol-5-yl)-(1,1'-biphenyl)-4-yl]-methyl}-4-oxo-8-chinolincarbonsäure;
. 1,4-Dihydro-3-methyl-4-oxo-1-{[2'-(1H-tetrazol-5-yl)-(1,1'-biphenyl)-4-yl]-methyl}-8-chinolincarbonsäure;
. 1,4-Dihydro-3-ethyl-4-oxo-1-{[2'-(1H-tetrazol-5-yl)-(1,1'-biphenyl)-4-yl]-methyl}-8-chinolincarbonsäure.

6. Verfahren nach irgendeinem der Ansprüche 1 bis 5, worin die Produkte für die Herstellung von Arzneimitteln verwendet werden, die vorgesehen sind für:
entweder die Behandlung von arteriellem Überdruck, Herzinsuffizienzen, Glaukom, Niereninsuffizienzen und die Vorbeugung von post-angioplastischen Restenosen,
oder die Behandlung von einigen gastro-intestinalen oder gynäkologischen Störungen,
oder die Behandlung von Störungen des Gedächtnisses oder der kognitiven Funktionen, von Angst, Depression, seniler Demenz oder der Alzheimer-Erkrankung.
